# EUROPEAN PATENT APPLICATION

(11) **EP 0 915 086 A1**
(43) Date of publication of application: **12.05.1999**
(21) Application number: 97922148.8
(22) Date of filing: 23.05.1997
(51) Int. Cl.: C07C 311/00, C07D 209/20, C07D 403/12, C07D 207/16, C07D 233/56, C07D 213/52, C07D 213/40, C07D 295/13, A61K 31/405, A61K 31/415, A61K 31/40

(54) **PHENYLSULFONAMIDE DERIVATIVES**

(30) Priority: 24.05.1996 JP 151864/96; 21.01.1997 JP 20879/97
(71) Applicant: ONO PHARMACEUTICAL CO., LTD., Osaka-shi, Osaka 541-8526 (JP)
(72) Inventor: TAKAHASHI, K.-Minase Res.Inst.-ONO Pharma. Co. Ltd, Osaka 618-8585 (JP); SUGIURA, T.-Minase Res. Inst.-ONO Pharm. Co., Ltd, Osaka 618-8585 (JP)
(74) Representative: Bentham, Stephen
(86) International application number: JP9701735
(87) International publication number: WO9745402

(57) **Abstract**

A phenylsulfonamide derivative of formula (I) 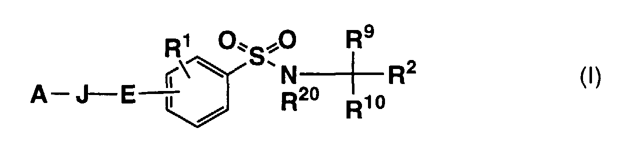
(wherein R¹ is H, alkyl; R² is COOR³, CONHOR⁴; E is vinylene, ethynylene; A is H, alkyl, a carbocyclic ring, a heterocyclic ring; J is a single bond, aklylene; R⁹, R¹⁰ is H, (substituted) alkyl, COR¹¹, a carbocyclic ring, a heterocyclic ring etc.; R²⁰ is H, (substituted) alkyl etc.) and a non-toxic salt thereof, and a process for the preparation thereof, a matrix metalloproteinase inhibitor containing a compound above, and an agent for prevention and/or treatment of rheumatoid diseases, arthrosteitis, unusual bone resorption, osteoporosis, periodontitis, intestitial nephritis, arteriosclerosis, pulmonary emphysema, cirrhosis, cornea injury, metastasis or invasion of tumor cells, a disease induced by proliferation of tumor cells, an autoimmune disease (Crohn's disease or Sjogren's syndrome), diseases caused by vascular emigration and infiltration of leukocytes, or arterialization.

## Description

The present invention relates to phenylsulfonamide derivatives, processes for preparation thereof and pharmaceutical compositions containing the derivatives as active ingredients.

More particularly, this invention relates to phenylsulfonamide derivatives of formula (I) 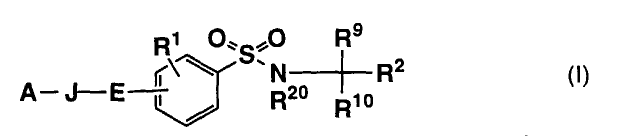
(wherein all symbols are as defined below), non-toxic salts thereof, processes for preparation thereof and matrix metalloproteinase inhibitors containing them.

### Background of the invention

The matrix metalloproteinases (MMPs) are neutral metalloproteinases and zinc (Zn²⁺) is essential in the active site for their activation. They degrade collagen, laminin, proteoglycans, fibronectin, elastin, gelatin etc. under physiological conditions and, therefore, are effective on growth and tissue remodeling of articulation tissue, bone tissue and connective tissue. At least 10 classes of MMPs which differ in primary structures have been identified till now. Concretely, interstitial collagenase (MMP-1), leukocyte collagenase (MMP-8), gelatinase A (MMP-2), gelatinase B (MMP-9), stromlycin 1 (MMP-3), stromlycin 2 (MMP-10), matrilycin (MMP-7) etc. are known.

As common characteristics to these enzymes, MMPs
(1) have Zn²⁺ in the active site and the activity depends on calcium (Ca²⁺),
(2) are secreted as an inactive proenzyme and activated outside of cells,
(3) have high homology on amino acid sequence,
(4) have an ability to degrade various extracellular matrix components *in vivo,*
(5) are regulated by tissue inhibitors of metalloproteinase (TIMP) which are specific to MMPs.

MMP inhibitors are assumed to be useful for prevention and/or treatment of various diseases, which are caused by unusual sthenia of MMP secretion and activity; such diseases are, for example, a rheumatoid disease, arthrosteitis, unusual bone resorption, osteoporosis, periodontitis, interstitial nephritis, arteriosclerosis, pulmonary emphysema, cirrhosis, cornea injury, metastasis or invasion of tumor cells, a disease induced by proliferation of tumor cells, an autoimmune disease (e.g. Crohn's disease, Sjogren's syndrome), a disease caused by vascular emigration or infiltration of leukocytes or arterialization.

Some compounds are known to have activity against matrix metalloproteinases. In particular, a substrate near the cleaving site of collagen (Gly-Ile-Ala-Gly or Gly-Leu-Ala-Gly) is known to have high affinity with collagenase. A lot of chemically modified matrix metalloproteinase inhibitors analogous to the substrate are under research [Inhibitors of matrix metalloproteinases (MMP's), Nigel RA Beeley, Philip RJ Ansell, Andrew JP Docherty, et al. Curr. Opin. Ther. Patents., 4, 7-16(1994), Current Drugs Ltd. ISSN 0962-2594].

But these inhibitors are peptide analogues and are analogous to substrates, and so various problems are expected to arise. It is, therefore, desired to convert them to non-peptide types. Some compounds have been reported.

For example, in the specification of EP606046, it is disclosed that arylsulfonamide derivatives of formula (X) 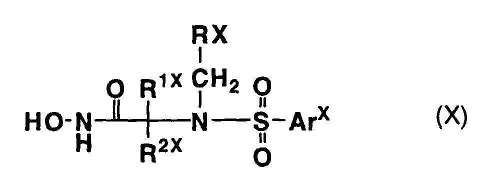
(wherein (a) Ar^{X} is carbocyclic or heterocyclic aryl; R^{X} is hydrogen, lower alkyl, carbocyclic aryl-lower alkyl etc.; R^{1X} is hydrogen, lower alkyl, carbocyclic aryl-lower alkyl etc.; R^{2X} is hydrogen or lower alkyl; or (b) R^{X} and R^{1X} are taken together with the chain to which they are connected to form 1,2,3,4-tetrahydro-isoquinoline, piperidine ring etc.; Ar^{X} and R^{2X} are the same meaning as defined in (a); or (c) R^{1X} and R^{2X} are taken together with carbon atom to which they are attached to form C₃₋₇cycloalkane, oxa-cyclohexane, thia-cyclohexane etc., which is unsubstituted or substituted by lower alkyl (Ar^{X} and R^{2X} represent the same meaning as defined in (a)) have an activity against matrix metalloproteinase.

In the specification of WO9535276, it is disclosed that compounds of formula 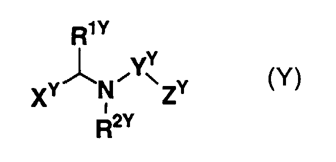
(wherein X^{Y} is COOH, CONHOH; R^{1Y} is a side chain of natural or synthetic α-amino acid; R^{2Y} is Z^{1Y}Q^{Y}W^{Y}; Z^{1Y} is hydrogen, aryl etc.; (i) Q^{Y}W^{Y} are taken together to form a single bond, (ii) Q^{Y} is O, S, W^{Y} is C₁₋₂₀alkyl etc., (iii) Q^{Y} is a single bond, W^{Y} is C₉₋₂₀ alkyl etc., (iv) Q^{Y} is a single bond, W^{Y} is C₁₋₈alkyl; Y^{Y} is SO₂; Z^{Y} is aryl, heteroaryl) have an activity against matrix metalloproteinase.

On the other hand, in the specification of WO9315047, it is disclosed that compounds of formula (Z) 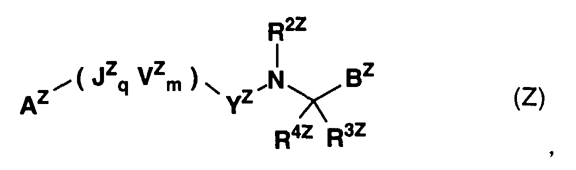
(wherein A^{Z} is a)-Q^{Z}-X^{Z} (wherein Q^{Z} is -O-, -S-, -NR^{Z}-, a single bond; X^{Z} is a five- or six- membered aromatic or heterocyclic ring) b) -CN, -NO₂, -N₃, -NR^{Z}R^{1Z}, -OR^{Z}, -COR^{Z}, -CO₂R^{Z} (R^{Z} and R^{1Z} are each independently, hydrogen, C₁₋₁₈alkyl, C₂₋₁₈alkenyl etc.) etc. J^{Z} is bivalent C₁₋₈alkandiyl, alkendiyl or alkyndiyl; V^{Z} is phenylene, furandiyl, thiophendiyl, thiazoldiyl etc.; q and m are 0 or 1; Y^{Z} is a single bond, -CH₂-, -C(=O)-, -C(=S)-, -S(=O)₂-, -P(=O)(OC₁₋₆alkyl)-, with the proviso that Q^{Z} is not a single bond when Y^{Z} is -S(=O)₂-); R^{2Z} is hydrogen, C₁₋₆alkyl, C₂₋₆alkenyl, C₂₋₆alkynyl, -CO(C₁₋₆alkyl), -CO₂(C₁₋₆alkyl) etc.; R^{3Z} and R^{4Z} are each independently, hydrogen, halogen, C₁₋₆alkyl, a side chain of natural amino acid etc.; B^{Z} is a) Z^{Z}R^{8Z} (Z^{Z} is a single bond, -C(=O)-, -C(=O)O-, -CH₂O- etc., R^{8Z} is hydrogen, C₁₋₁₈alkyl, C₂₋₁₈alkenyl, C₂₋₁₈alkynyl etc.). b) -CH₂NR^{9Z}R^{10Z} or -CONR^{9Z}R^{10Z} (R^{9Z} and R^{10Z} are each independently, hydrogen, C₁₋₁₈ alkyl, C₂₋₁₈alkenyl, C₂₋₁₈alkynyl etc.). c) E^{Z} (E^{Z} is a five- or six- membered heterocyclic ring which contains one or more hetero atom selected from nitrogen, oxygen, sulfur) or d) -CH₂E^{Z}, -C(=O)NHE^{Z} or -C(=O)NHCH₂E^{Z})) are effective as a PAF competitive inhibitor.

In the specification of WO9412181, it is disclosed that compounds of formula (W)

X^{W}-Y^{W}-Z^{W}-Ar^{W}-A^{W}-B^{W} (W)

(wherein Ar^{W} is a monocyclic aromatic six-membered ring which contains 0-4 nitrogen, and is unsubstituted or substituted by R^{5W};
X^{W} is -NR^{1W}R^{2W}, -NR^{1W}-C(=NR^{2W})-R^{1W}, or monocyclic or multicyclic, aromatic or non-aromatic, 4-10 membered ring system unsubstituted or substituted by R^{1W}, R^{2W}, R^{3W} or R^{4W}; R^{1W}, R^{2W}, R^{3W} and R^{4W} are independently, selected from a group consisting of hydrogen, C₁₋₁₀alkyl, C₃₋₈cycloalkyl, aryl C₀₋₈alkyl and amino C₀₋₈alkyl etc.;
Y^{W} is C₀₋₈alkyl, C₄₋₁₀cycloalkyl, C₀₋₈alkyl-NR^{3W}-CO-C₀₋₈alkyl, C₀₋₈alkyl-CONR^{3W}-C₀₋₈ alkyl etc.;
Z^{W} and A^{W} are independently selected from (CH₂)ₘ, (CH₂)ₘO(CH₂)ₙ, (CH₂)ₘNR^{3W}(CH₂)ₙ, (CH₂)ₘCONR^{3W}(CH₂)ₙ, (CH2)ₘNR^{3W}CO(CH₂)ₙ, (CH₂)ₘSO₂NR^{3w}(CH₂)ₙ, (CH₂)ₘC≡ C(CH₂)ₙ etc., m and n are independently, an interger selected from 0-6; R^{5W} is hydrogen, C₁₋₆alkyl, C₀₋₆alkylcarboxy C₀₋₆alkyl etc.;
B^{W} is 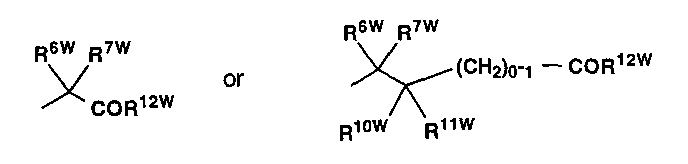
(wherein R^{6W}, R^{7W}, R^{8W}, R^{9W}, R^{10W}, and R^{11W} is hydrogen, fluorine, hydroxy C₁₋₆alkyl, carboxy C₀₋₆alkyl etc.; R^{12W} is hydroxy, C₁₋₈alkyloxy, aryl C₀₋₆alkyloxy etc.) are active against fibrinogen receptor).

### Disclosure of this invention

Energetic investigation has been carried out in order to discover compounds having an inhibitory effect on matrix metalloproteinase (e.g. gelatinase, stromlysin, and/or collagenase) and the present inventors have found that the phenylsulfonamide derivatives of formula (I) achieve the above purpose.

The present invention relates to
1) a phenylsulfonamide derivative of formula (I): 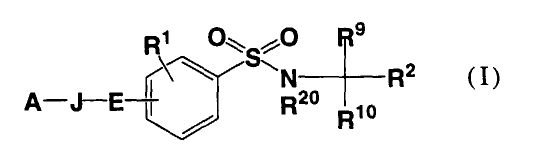
   (wherein R¹ is hydrogen, or C₁₋₄alkyl;
   R² is -COOR³, or -CONHOR⁴;
   R³ is
      1) hydrogen,
      2) C₁₋₈alkyl,
      3) phenyl, or
      4) C₁₋₄alkyl substituted by a group selected from phenyl, -OCOR¹⁵ (wherein R¹⁵ is C₁₋₄ alkyl) and -CONR¹⁶R¹⁷ (wherein R¹⁶ and R¹⁷ are each independently, hydrogen or C₁₋₄ alkyl);
   R⁴ is hydrogen, C₁₋₈alkyl, phenyl, or C₁₋₄alkyl substituted by phenyl;
   E is
      ―C≡C― or ―CH=CH―;
   A is hydrogen, C₁₋₈alkyl, a carbocyclic ring or a heterocyclic ring, in which the said rings may be substituted by 1-3 groups selected from:
   C₁₋₁₅alkyl, C₁₋₁₅alkoxy, halogen, nitro, cyano, guanidino, amidino, hydroxy, benzyloxy, trifluoromethyl, NR⁵R⁶ (wherein R⁵ and R⁶ are each independently, hydrogen, C₁₋₄alkyl, or -COOR⁷ (wherein R⁷ is C₁₋₄alkyl or benzyl)), -COOR⁸ (wherein R⁸ is hydrogen, C₁₋₄ alkyl, phenyl, or C₁₋₄alkyl substituted by phenyl), a carbocyclic ring and a heterocyclic ring or C₁₋₄alkyl substituted by hydroxy, C₁₋₄alkoxy, NR⁵R⁶, a carbocyclic ring or a heterocyclic ring;
   J is a single bond or C₁₋₈alkylene;
   R⁹ and R¹⁰ are each independently,
      (1) hydrogen,
      (2) C₁₋₈alkyl (wherein one carbon atom may be replaced by a sulfur atom),
      (3) -COR¹¹ (wherein R¹¹ is hydroxy, C₁₋₈alkyl, C₁₋₈alkoxy, phenoxy, C₁₋₄alkoxy substituted by phenyl, or NR¹⁸R¹⁹ (wherein R¹⁸ and R¹⁹ are each independently, hydrogen, C₁₋₄alkyl, phenyl, or C₁₋₄alkyl substituted by 1-2 phenyl, in which the said phenyl may be substituted by 1-3 groups selected from C₁₋₄alkyl, C₁₋₄alkoxy, halogen, hydroxy, and trifluoromethyl)),
      (4) a carbocyclic ring,
      (5) a heterocyclic ring, or
      (6) C₁₋₈alkyl substituted by a group selected from (i)-(viii) below:
         (i) -COR¹¹, wherein R¹¹ is as defined above,
         (ii) C₁₋₄alkoxy,
         (iii) hydroxy,
         (iv) benzyloxy,
         (v) guanidino,
         (vi) -NR¹²R¹³ (wherein R¹² and R¹³ are each independently, hydrogen, C₁₋₄alkyl or COOR¹⁴ (wherein R¹⁴ is C₁₋₄alkyl or benzyl)),
         (vii) a carbocyclic ring,
         (viii) a heterocyclic ring,
            (in which the carbocyclic ring or the heterocyclic ring defined above may be substituted by 1-3 groups selected from C₁₋₄alkyl, C₁₋₄alkoxy, halogen, hydroxy and trifluoromethyl);
   R²⁰ is hydrogen, C₁₋₄alkyl, C₁₋₈alkoxycarbonyl, C₁₋₄alkoxycarbonyl substituted by phenyl, or C₁₋₈alkyl substituted by a group selected from hydroxy, C₁₋₄alkoxy, benzoyloxy, -COOR²¹ (wherein R²¹ is hydrogen, C₁₋₈alkyl or benzyl), -NR²²R²³ (wherein R²² and R²³ are each independently, hydrogen or C₁₋₄alkyl), a carbocyclic ring and a heterocyclic ring;
   or R⁹ and R²⁰ together with the carbon atom and nitrogen atom to which they are attached represent a 5-7 membered heterocyclic ring containing one nitrogen);
   or a non-toxic salt thereof.
2) a process for the preparation of phenylsulfonamide derivatives of formula (I) or a non-toxic salt thereof; and
3) a drug containing a phenylsulfonamide derivative of formula (I) or a non-toxic salt thereof as an active ingredient.

### Description of the invention

Otherwise specified, all isomers are included in the present invention. For example, alkyl, alkoxy, and alkylene include straight and branched isomers. Double bonds in alkenylene contain E, Z, and EZ mixed isomers. Isomers resulting from the presence of asymmetric carbon(s) (e.g. branched alkyl, alkoxy and alkylene) are also included in the present invention.

In the formula (I), C₁₋₄alkyl represented by R¹, R⁵, R⁶, R⁷, R⁸, R¹², R¹³, R¹⁴, R¹⁵, R¹⁶, R¹⁷, R¹⁸, R¹⁹, R²⁰, R²², R²³, a substituent of a carbocyclic ring or a heterocyclic ring in R⁹ and R¹⁰, and C₁₋₄alkyl as a substituent of phenyl in R¹⁸ and R¹⁹ is methyl, ethyl, propyl, butyl and isomeric groups thereof.

In the formula (I), C₁₋₈alkyl represented by R³, R⁴, R⁹, R¹⁰, R¹¹, R²¹, A is methyl, ethyl, propyl, butyl, pentyl, hexyl, heptyl, octyl and isomeric groups thereof.

In the formula (I), C₁₋₁₅alkyl as a substituent of a carbocyclic ring or a heterocyclic ring represented by A is methyl, ethyl, propyl, butyl, pentyl, hexyl, heptyl, octyl, nonyl, decyl, undecyl, dodecyl, tridecyl, tetradecyl, pentadecyl and isomeric groups thereof.

In the formula (I), C₁₋₄alkyl substituted by a group selected from phenyl, -OCOR¹⁵ and -CONR¹⁶R¹⁷ represented by R³ is methyl, ethyl, propyl and butyl and isomeric groups thereof substituted by one group selected from phenyl, -OCOR¹⁵, -CONR¹⁶R¹⁷.

In the formula (I), C₁₋₄alkyl substituted by phenyl represented by R⁴, R⁸ is methyl, ethyl, propyl, butyl and isomeric groups thereof which are substituted by one phenyl.

In the formula (I), C₁₋₄alkyl substituted by 1 or 2 phenyl represented by R¹⁸, R¹⁹ is methyl, ethyl, propyl, butyl and isomeric groups thereof which are substituted by 1 or 2 phenyl.

In the formula (I), C₁₋₄alkyl substituted by a group selected from hydroxy, C₁₋₄ alkoxy, -NR⁵R⁶, -COOR⁸, a carbocyclic ring and a heterocyclic ring represented by A is methyl, ethyl, propyl, butyl and isomeric groups thereof which are substituted by a group selected from hydroxy, C₁₋₄alkoxy, NR⁵R⁶, -COOR⁸, a carbocyclic ring or a heterocyclic ring.

In the formula (I), C₁₋₈alkyl substituted by a group selected from (i)-(viii) below represented by R⁹, R¹⁰; (i) -COR¹¹, (ii) C₁₋₄alkoxy, (iii) hydroxy (iv) benzyloxy (v) guanidino (vi) -NR¹²R¹³, (vii) a carbocyclic ring and (viii) a heterocyclic ring is methyl, ethyl, propyl, butyl, pentyl, hexyl, heptyl, octyl and isomeric groups thereof which are substituted by (i)-(viii) above.

In the formula (I), C₁₋₈alkyl substituted by a group selected from hydroxy, C₁₋₄ alkoxy, benzyloxy, -COOR²¹, -NR²²R²³, a carbocyclic ring and a heterocyclic ring represented by R²⁰ is methyl, ethyl, propyl, butyl, pentyl, hexyl, heptyl, octyl and isomeric groups thereof which are substituted by a group selected from hydroxy, C₁₋₄ alkoxy, benzyloxy, -COOR²¹, -NR²²R²³, a carbocyclic ring and a heterocyclic ring.

In the formula (I), C₁₋₈alkyl wherein one carbon atom is replaced by one sulfur atom represented by R⁹, R¹⁰ is a group wherein one -CH₂- in methyl, ethyl, propyl, butyl, pentyl, hexyl, heptyl, octyl, and isomeric groups thereof, is replaced by a sulfur atom. For example, such groups are -SH, -CH₂-SH, -CH₂CH₂-S-CH₃ etc.

In the formula (I), C₁₋₄alkoxy as a substituent on a carbocyclic ring or a heterocyclic ring in R⁹ and R¹⁰, C₁₋₄alkoxy as a substituent of phenyl in R¹⁸ and R¹⁹ is methoxy, ethoxy, propoxy, butoxy and isomeric groups thereof.

In the formula (I), C₁₋₁₅alkoxy as a substituent on a carbocyclic ring or a heterocyclic ring represented by A is methoxy, ethoxy, propoxy, butoxy, pentyloxy, hexyloxy, heptyloxy, octyloxy, nonyloxy, decyloxy, undecyloxy, dodecyloxy, tridexyloxy, tetradecyloxy, pentadecyloxy and isomeric groups thereof.

In the formula (I), C₁₋₈ alkoxy represented by R¹¹ is methoxy, ethoxy, propoxy, butoxy, pentyloxy, hexyloxy, heptyloxy, octyloxy and isomeric groups thereof.

In the formula (I), C₁₋₄alkoxy substituted by phenyl represented by R¹¹ is methoxy, ethoxy, propoxy, butoxy substituted by phenyl and isomeric groups thereof.

In the formula (I), C₁₋₈alkoxycarbonyl represented by R²⁰ is methoxycarbonyl, ethoxycarbonyl, propoxycarbonyl, butoxycarbonyl, pentyloxycarbonyl, hexyloxycarbonyl, heptyloxycarbonyl, octyloxycarbonyl and isomeric groups thereof.

In the formula (I), C₁₋₄alkoxycarbonyl substituted by phenyl represented by R²⁰ is methoxycarbonyl, ethoxycarbonyl, propoxycarbonyl, butoxycarbonyl and isomeric groups thereof substituted by a phenyl.

In the formula (I), halogen as a substituent on a carbocyclic ring or a heterocyclic ring represented by A, halogen as a substituent on a carbocyclic ring or a heterocyclic ring in R⁹ and R¹⁰, and halogen as a substituent of phenyl in R¹⁸ and R¹⁹ is fluorine, chlorine, bromine and iodine.

In the formula (I), C₁₋₈alkylene represented by J is ethylene, trimethylene, tetramethylene, pentamethylene, hexamethylene, heptamethylene, octamethylene and isomeric groups thereof.

In the formula (I), a carbocyclic ring represented by A, R⁹ and R¹⁰, or a carbocyclic ring included in A, R⁹, R¹⁰ and R²⁰ is a C₃₋₁₀carbocyclic ring. For example, such rings are cyclopropane, cyclobutane, cyclopentane, cyclopentene, cyclohexane, cyclohexene, cyclohexadiene, benzene, pentalene, indan, indene, dihydronaphthalene, tetrahydronaphthalene, naphthalene etc.

In the formula (I), a heterocyclic ring represented by A, R⁹ and R¹⁰, or a heterocyclic ring included in A, R⁹, R¹⁰ and R²⁰ is a 5-15 membered mono- or bi-heterocyclic ring containing one or two nitrogen and/or one oxygen and/or one sulfur. For example, a 5-15 membered mono- or bi- heterocyclic ring containing one or two nitrogen and/or one oxygen and/or one sulfur is heterocyclic aryl (e.g. pyrrole, imidazole, pyrazole, pyridine, pyrazine, pyrimidine, pyridazine, azepine, diazepine, furan, pyran, oxepin, oxazepine, thiophene, thiain (thiopyran), thiepin, oxazole, isoxazole, thiazole, isothiazole, oxadiazole, oxazine, oxadiazine, oxazepine, oxadiazepine, thiadiazole, thiazine, thiadiazine, thiazepine, thiadiazepine, indole, isoindole, benzofuran, isobenzofuran, benzothiophene, isobenzothiophene, indazole, quinoline, isoquinoline, phthalazine, naphthyridine, quinoxaline, quinazoline, cinnoline, benzoxazole, benzothiazel, or benzoimidazole), and a partially or fully saturated heterocyclic ring thereof (e.g. pyrroline, pyrrolidine, imidazoline, imidazolidine, pyrazoline, pyrazolidine, piperidine, piperazine, tetrahydropyrimidine, tetrahydropyridazine, dihydrofuran, tetrahydrofuran, dihydropyran, tetrahydropyran, dihydrothiophene, tetrahydrothiophene, dihydrothiain (dihydrothiopyran), tetrahydrothiain (tetrahydrothiopyran), dihydroxazole, tetrahydroxazole, dihydroisoxazole, tetrahydroisoxazole, dihydrothiazole, tetrahydrothiazole, dihydroisothiazole, tetrahydroisothiazole, morpholine, thiomorpholine, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, dihydrobenzoxazole, perhydrobenzoxazole, dihydrobenzothiazole, perhydrobenzothiazole, dihydrobenzimidazole or perhydrobenzimidazole).

In the formula (I), a 5-7 membered heterocyclic ring containing one nitrogen represented by R⁹ and R²⁰ together with carbon and nitrogen to which they are attached means 5-7 membered monocyclic hetero aryl containing one nitrogen or the heterocyclic ring partially or fully saturated, for example, pyrrole, pyrroline, pyrrolidine, pyridine, dihydropyridine, tetrahydropyridine, piperidine, azepine, dihydroazepine, perhydroazepine.

In the formula (I), R⁹ and R¹⁰ may represent α-amino acid side chain(s), for example, glycine, alanine, serine, threonine, cystein, valine, methionine, leucine, isoleucine, phenylalanine, tyrosine, aspartic acid, glutamic acid, asparagine, arginine, lysine, histidine, tryptophan, glutamine, proline etc. In the α-amino acid side chain(s), side chains of α-amino acid derivatives, D, L, and DL mixed isomers or alloisomers thereof are included.

### [Salt]

Non-toxic salts include all the salts in the present invention; for example, a general salt, an acid addition salt, a salt of hydrate etc.

The compounds of the present invention of formula (I) may be converted to the corresponding salts by a conventional means. Non-toxic, and water-soluble salts are preferable. Appropriate salts are described below; salts of alkali metals (e.g. potassium, sodium), salts of alkaline-earth metals (e.g. calcium, magnesium), ammonium salts, salts of pharmaceutically acceptable organic amines (tetramethylammonium, triethylamine, methylamine, dimethylamine, cyclopentylamine, benzylamine, phenethylamine, piperidine, monoethanolamine, diethanolamine, tris(hydroxymethyl)amine, lysine, arginine, N-methyl-D-glucamine)

The compounds of the present invention of the formula (I) can be converted to the corresponding acid addition salts by a conventional means. Water-soluble, non-toxic acid addition salts are preferable. Suitable salts, for example, include: salts of inorganic acid (e.g. hydrochloride, hydrobromide, hydroiodide, sulfate, phosphate, nitrate); salts of organic acids (e.g. acetate, trifluoroacetate, lactate, tartarate, oxalate, fumarate, maleate, citrate, benzoate, methanesulphonate, ethanesulphonate, benzenesulphonate, toluenesulphonate, isethionate, glucronate, gluconate.)

The compounds of the present invention of formula (I) and the salts thereof may be converted into the corresponding hydrates by a conventional means.

Among the compounds of the present invention of formula (I), preferable compounds are:
phenylsulfonamide derivatives of formula (I-A) 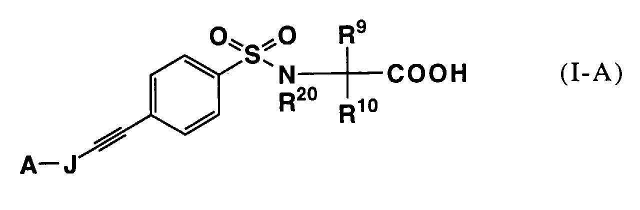 (wherein all symbols are as defined above),
(I-B) 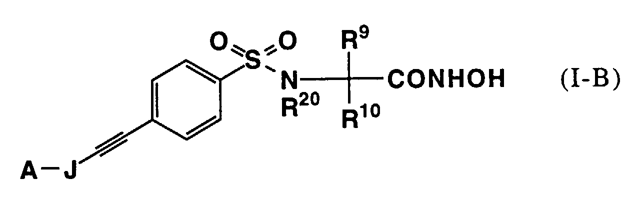 (wherein all symbols are as defined above),
(I-C) 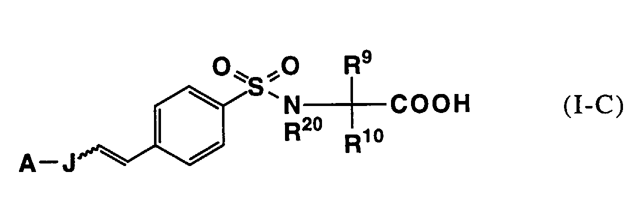 (wherein all symbols are as defined above),
(I-D) 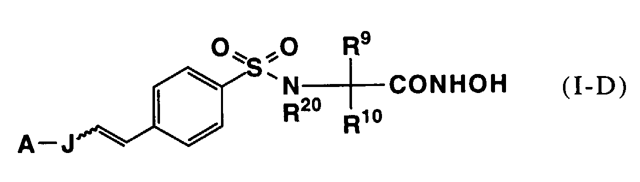 (wherein all symbols are as defined above)
and non-toxic salts thereof.

Among the compounds of the present invention of formula (I), the compounds described in tables 1-22, and non-toxic salts thereof are mentioned.

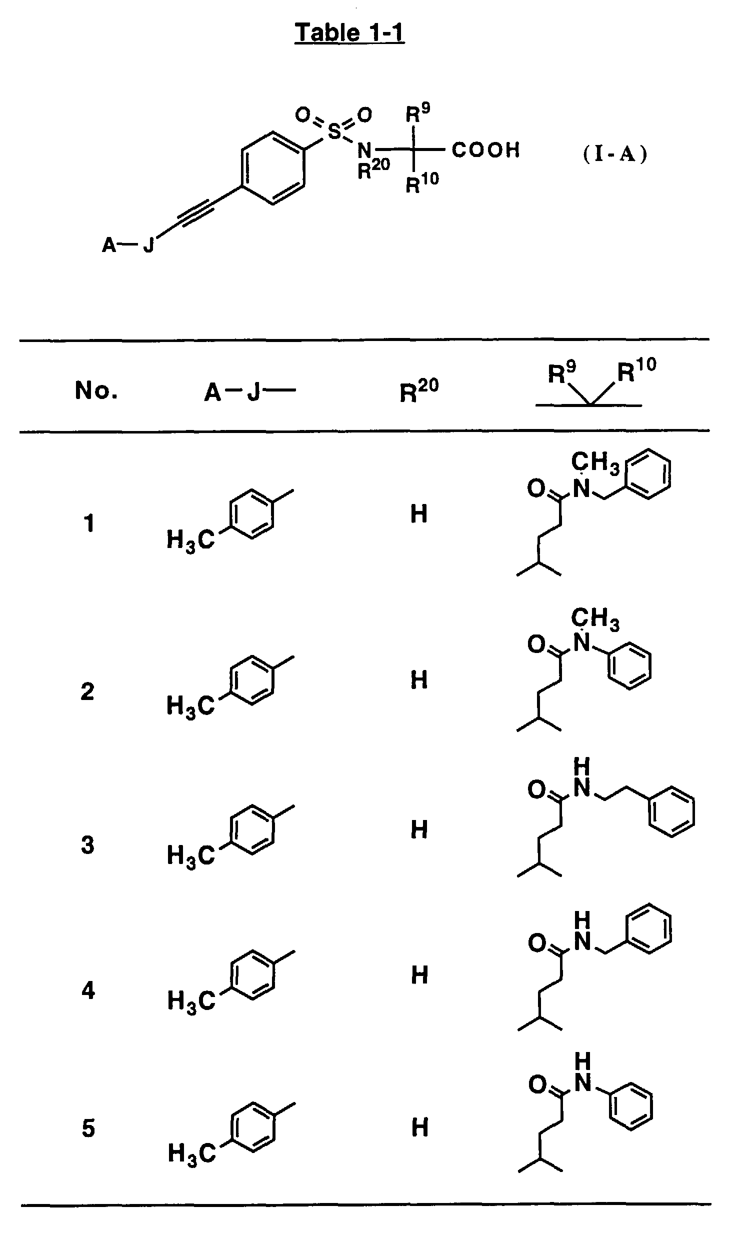

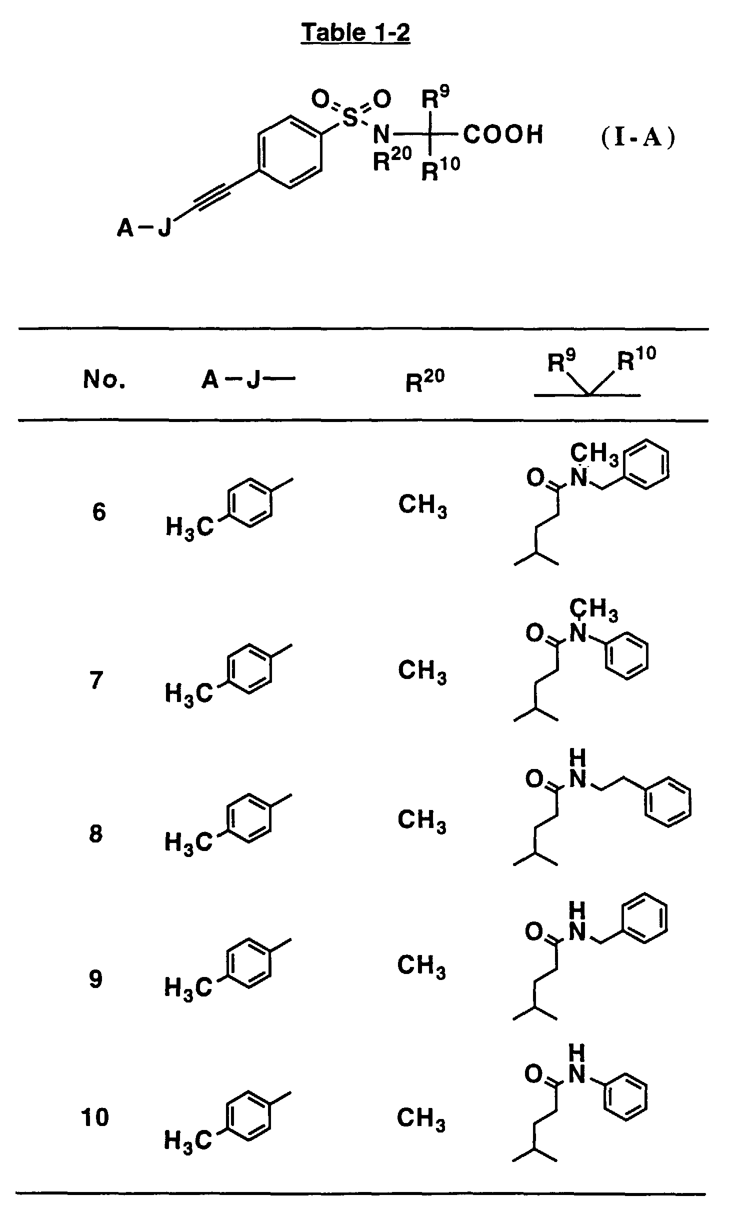

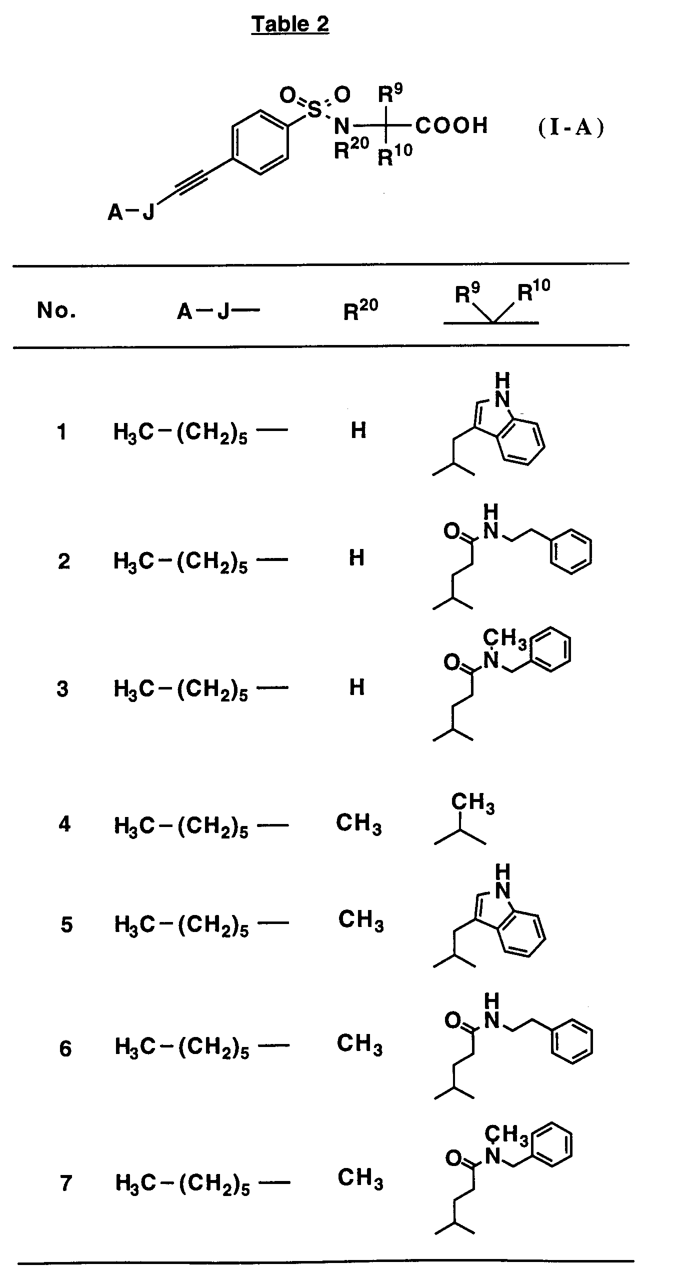

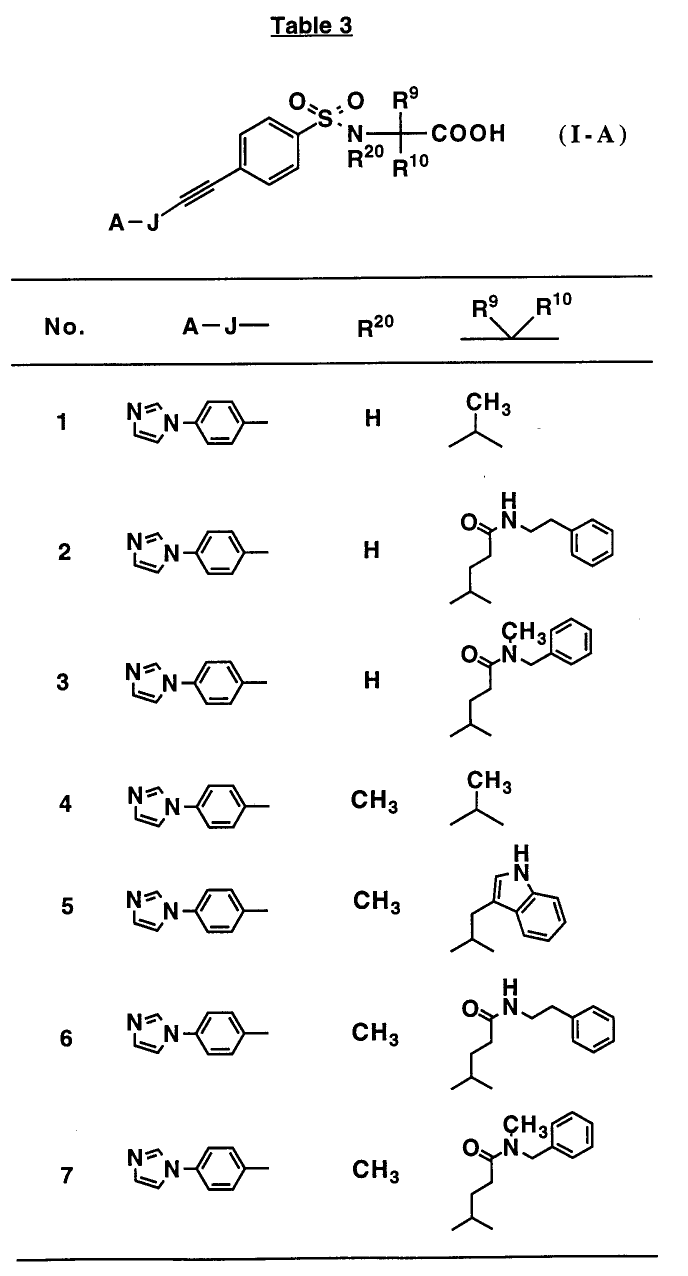

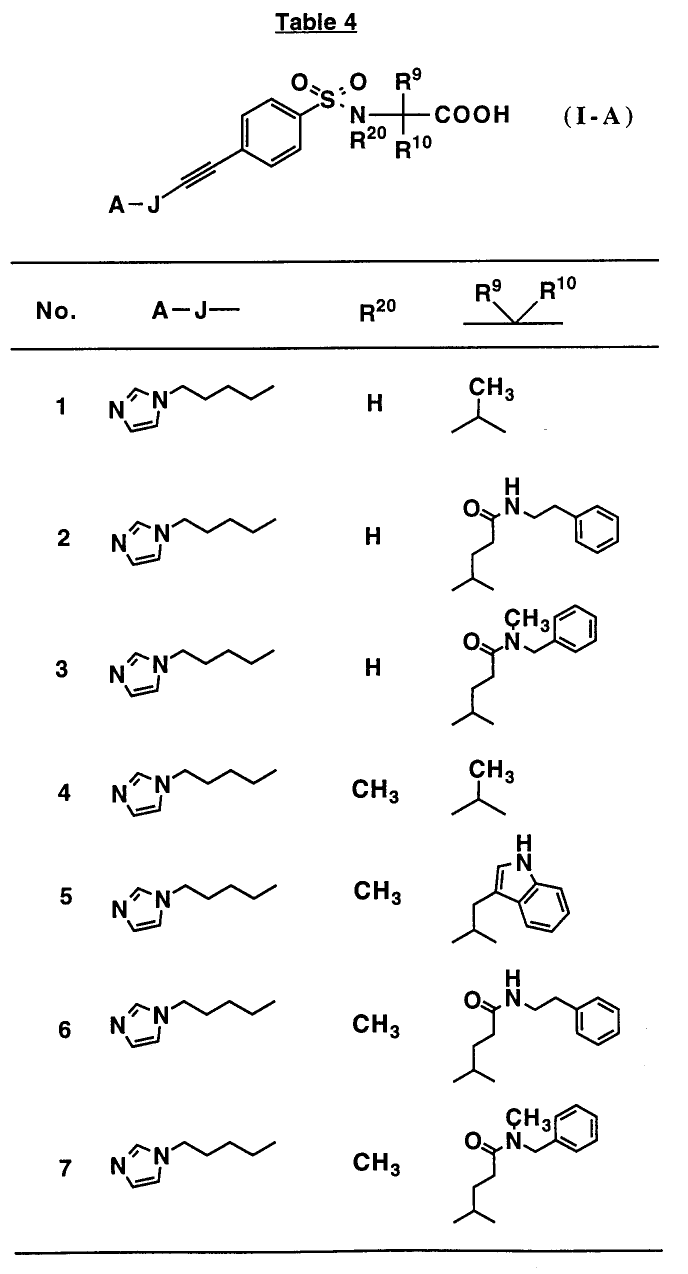

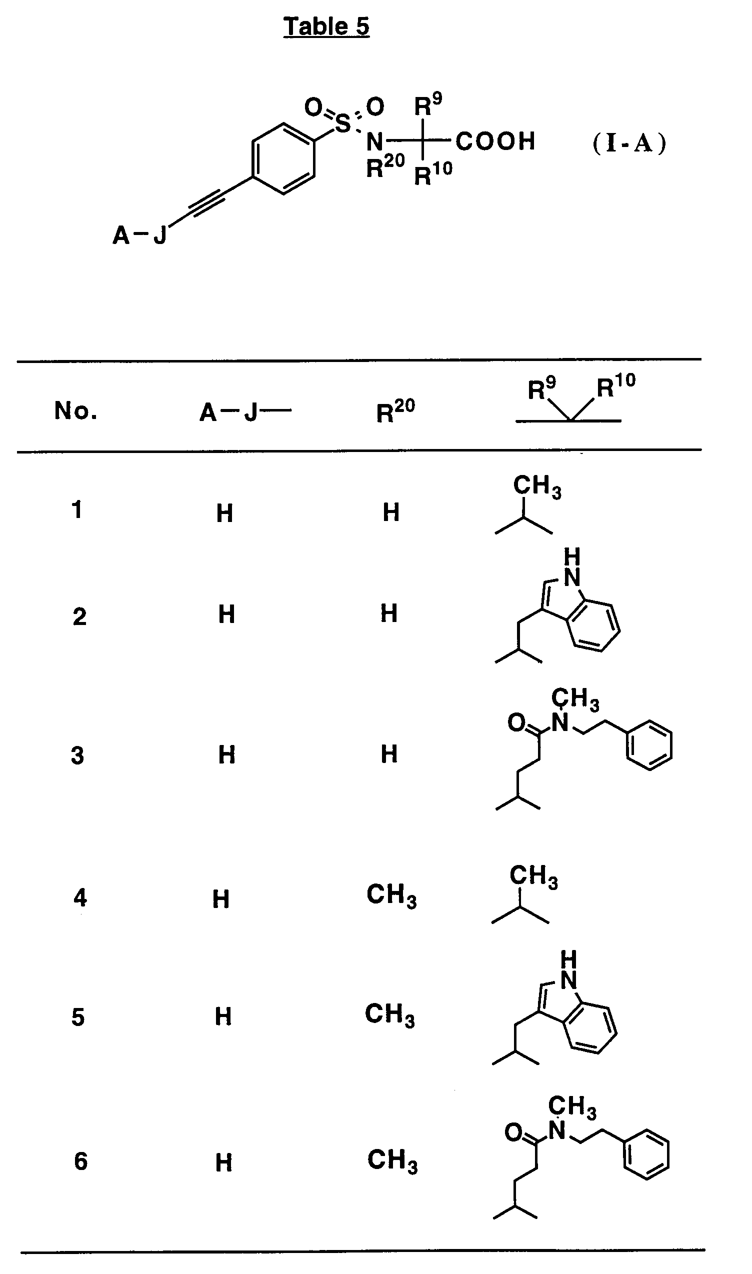

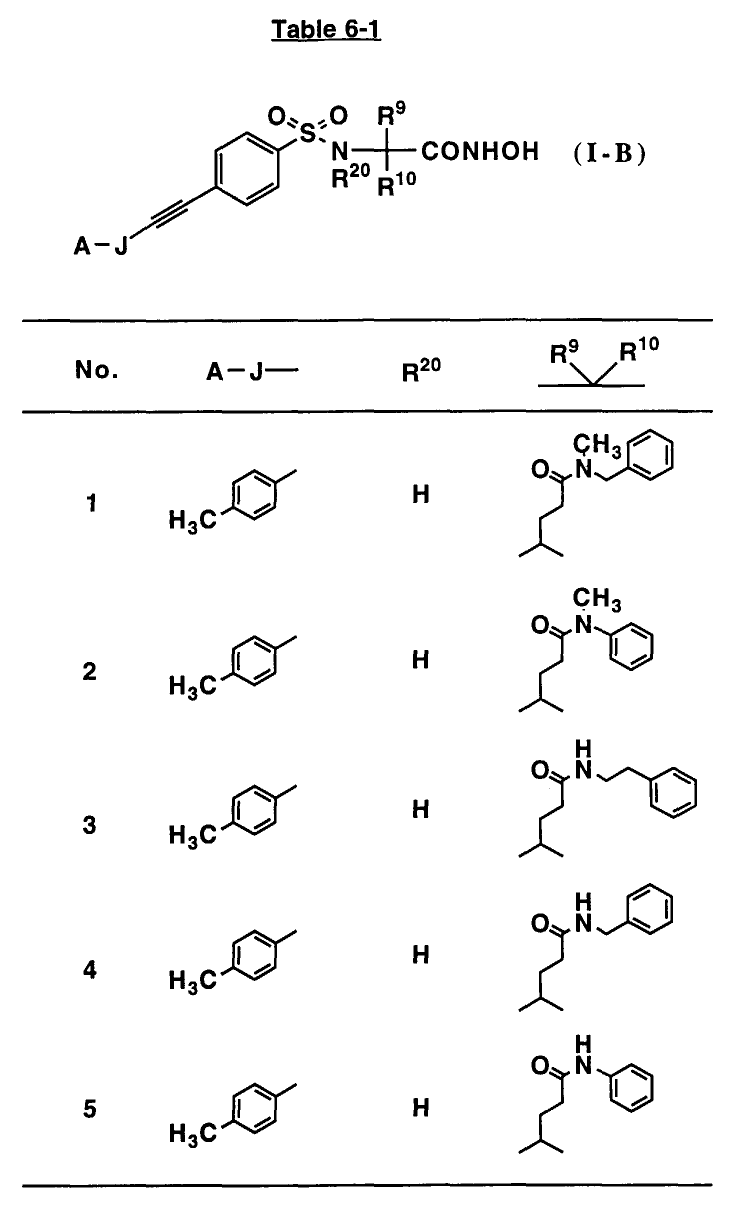

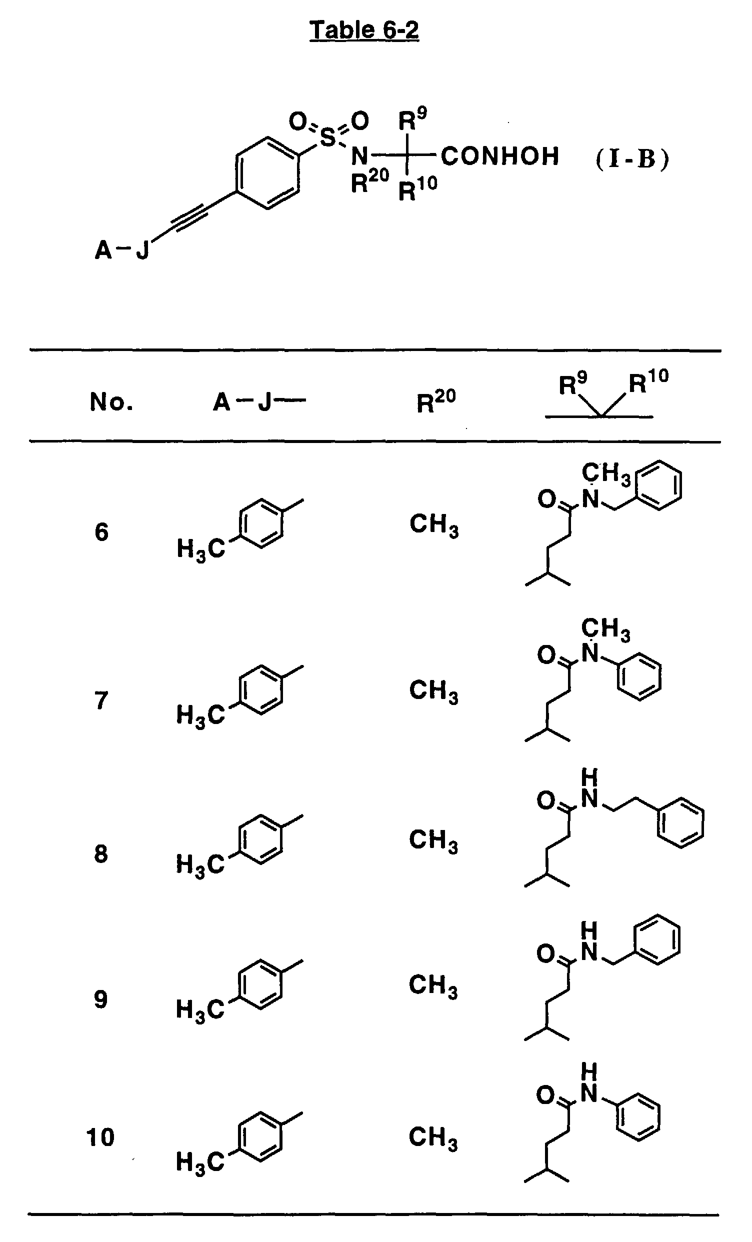

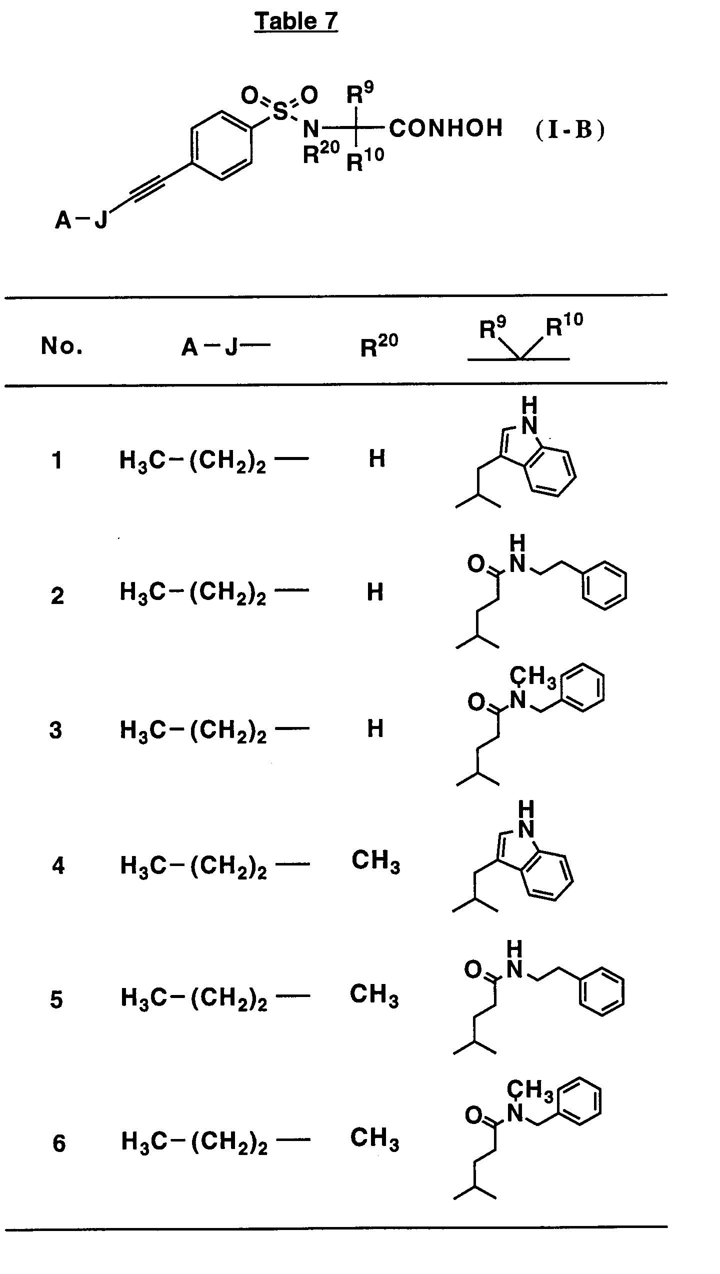

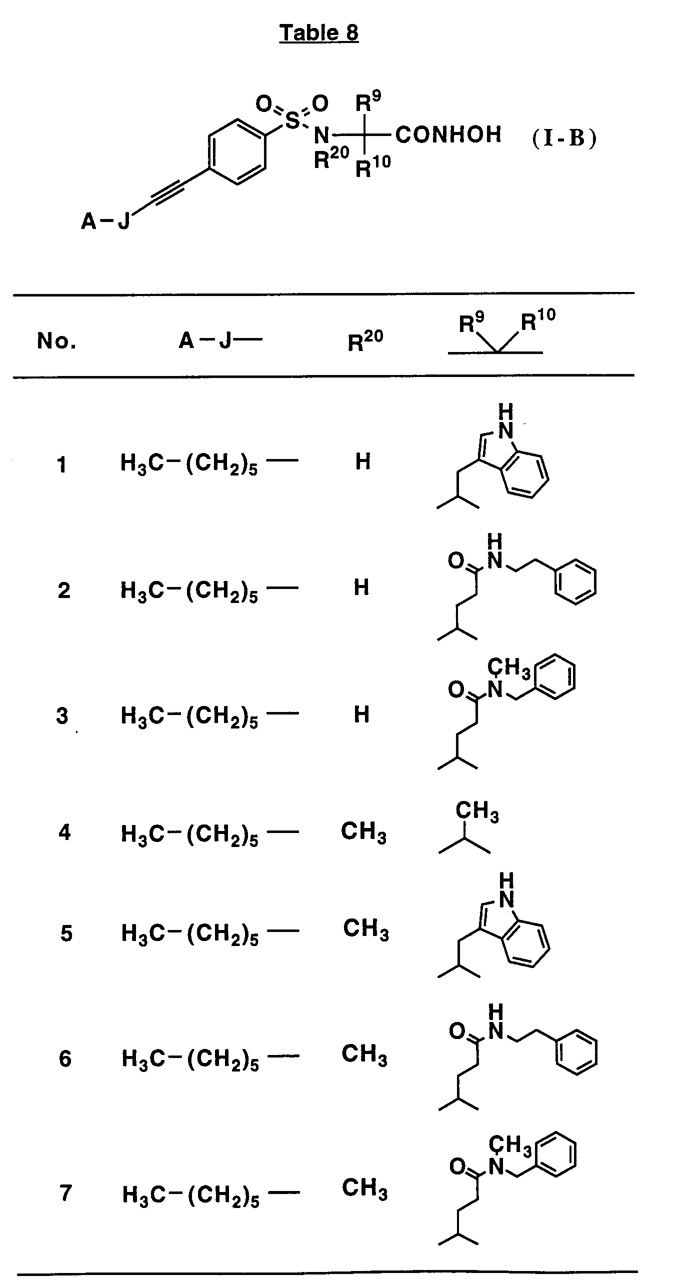

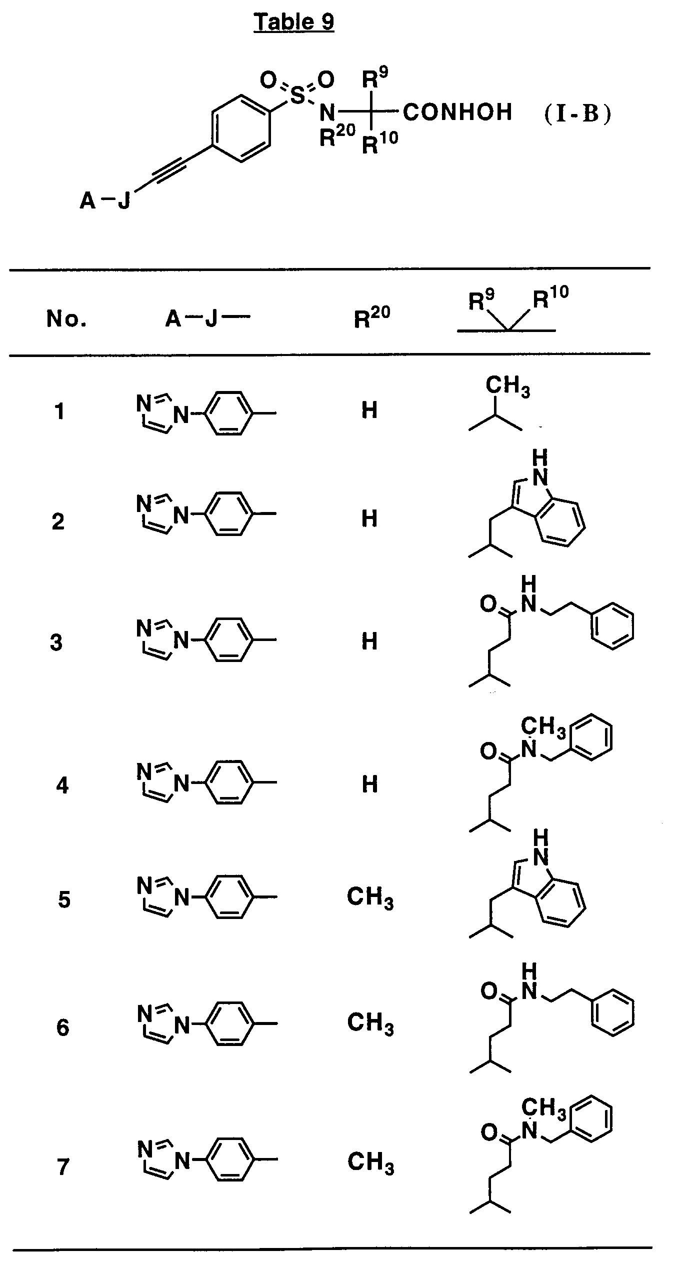

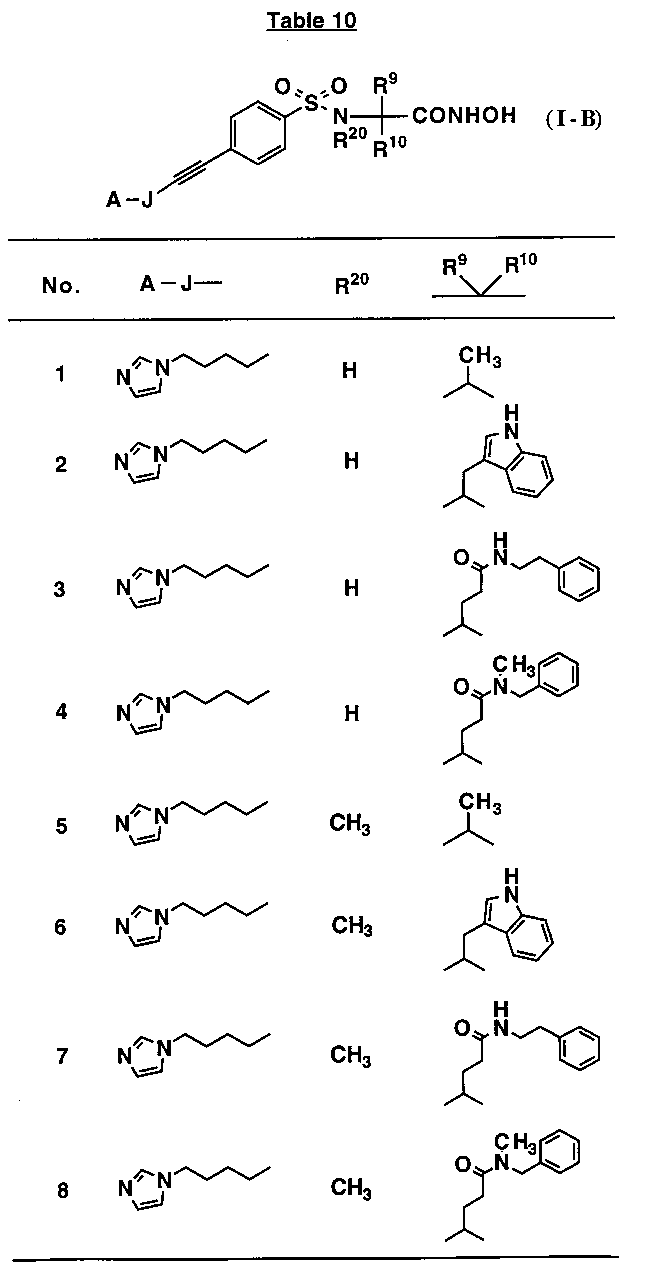

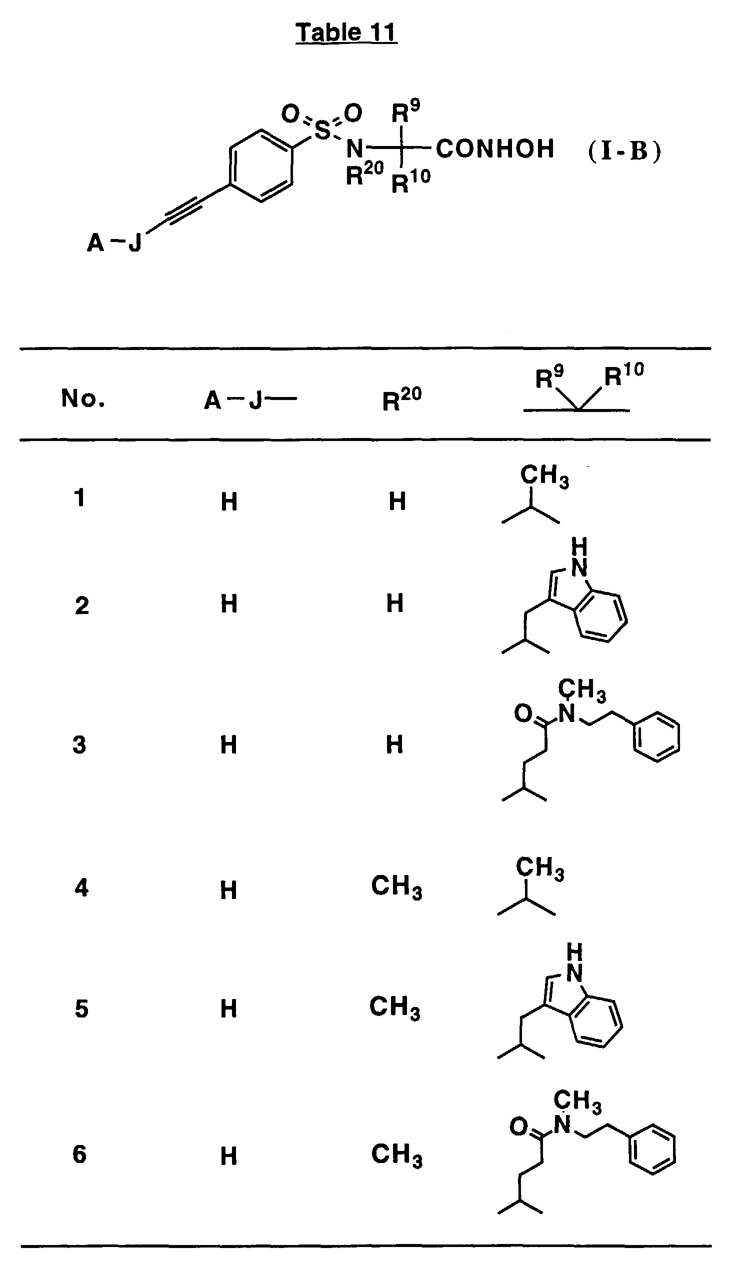

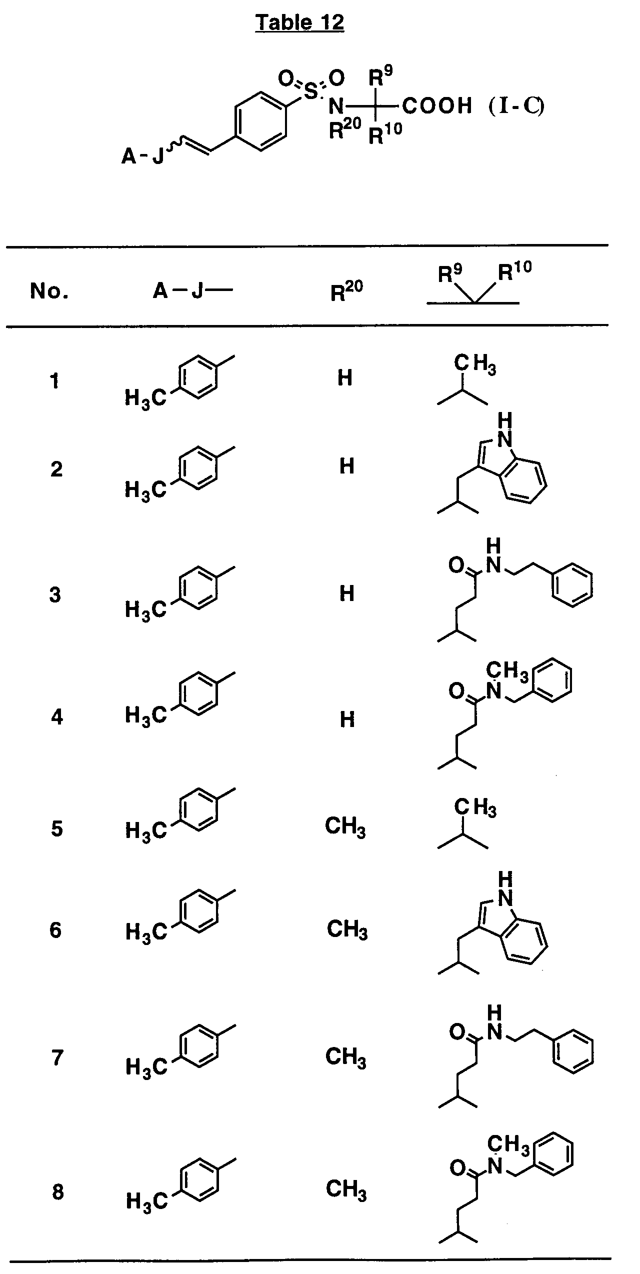

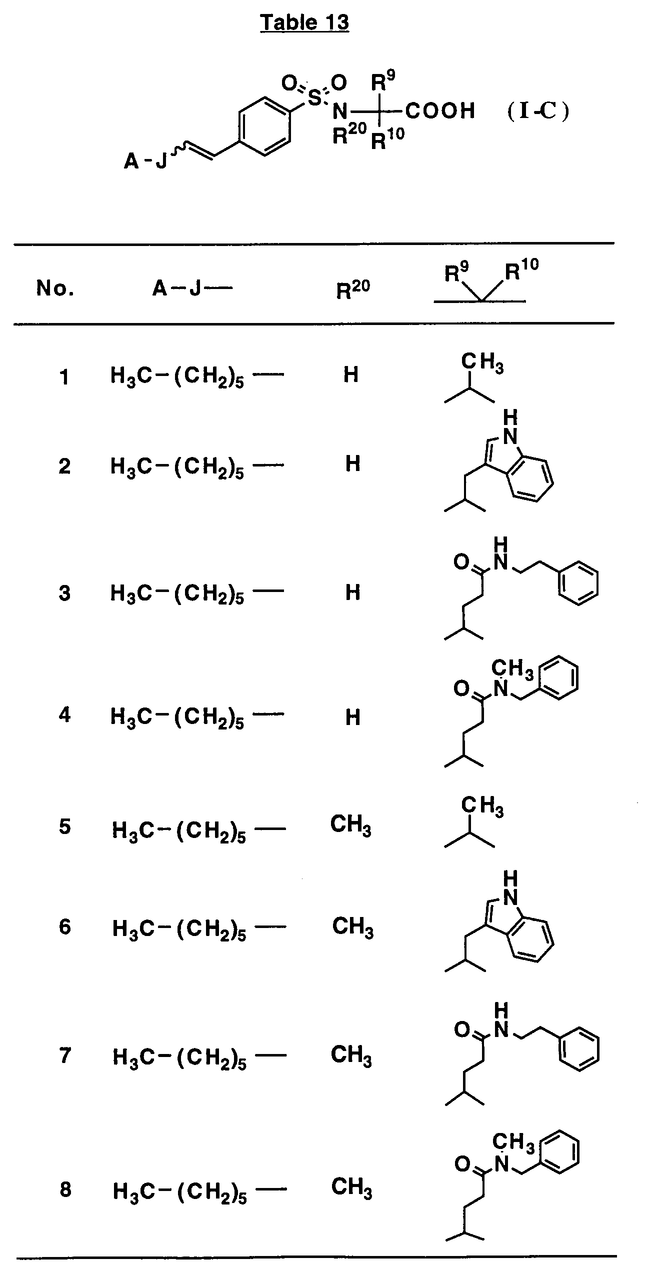

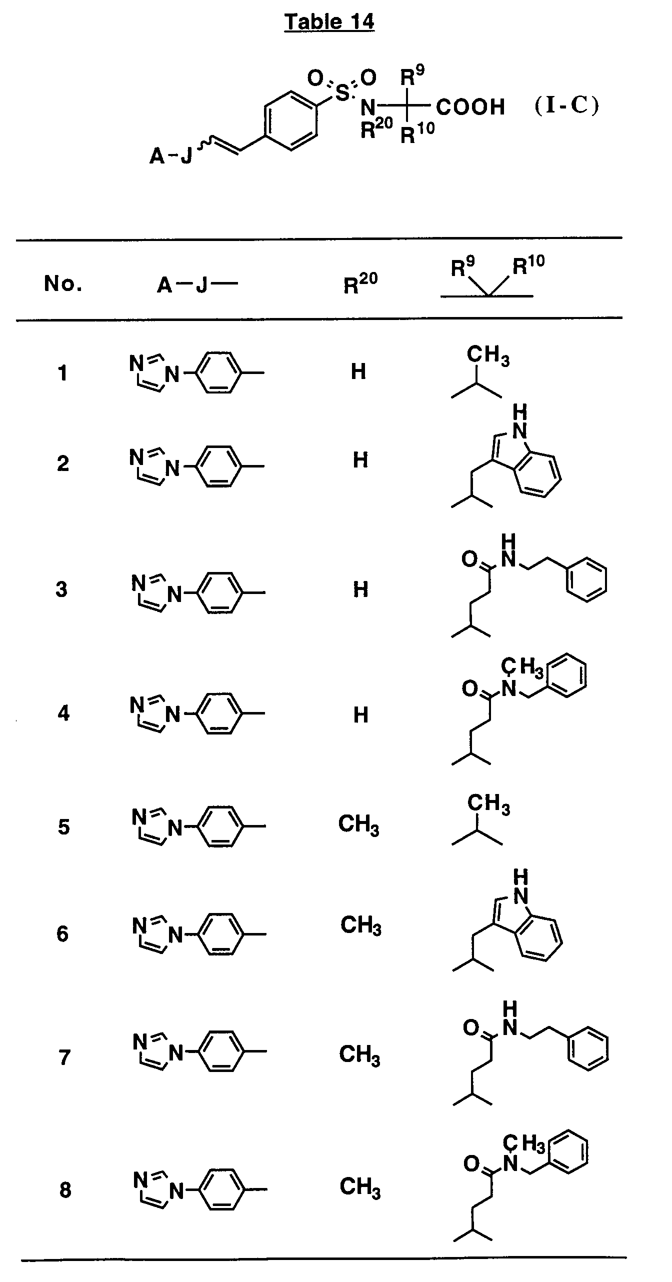

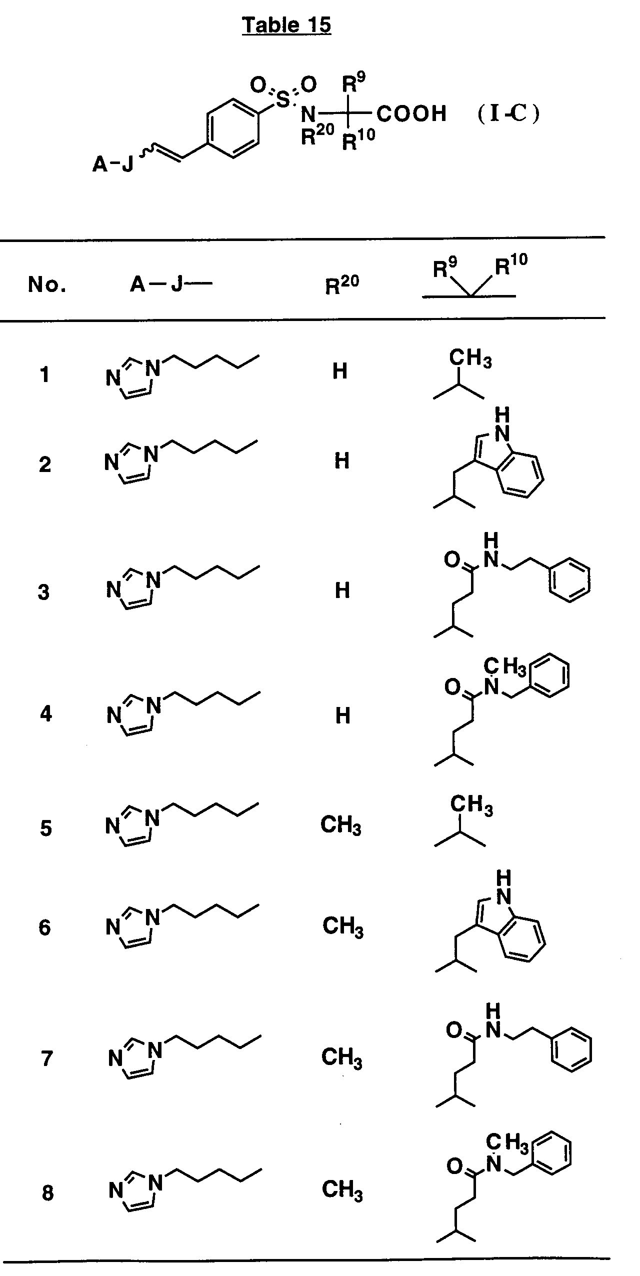

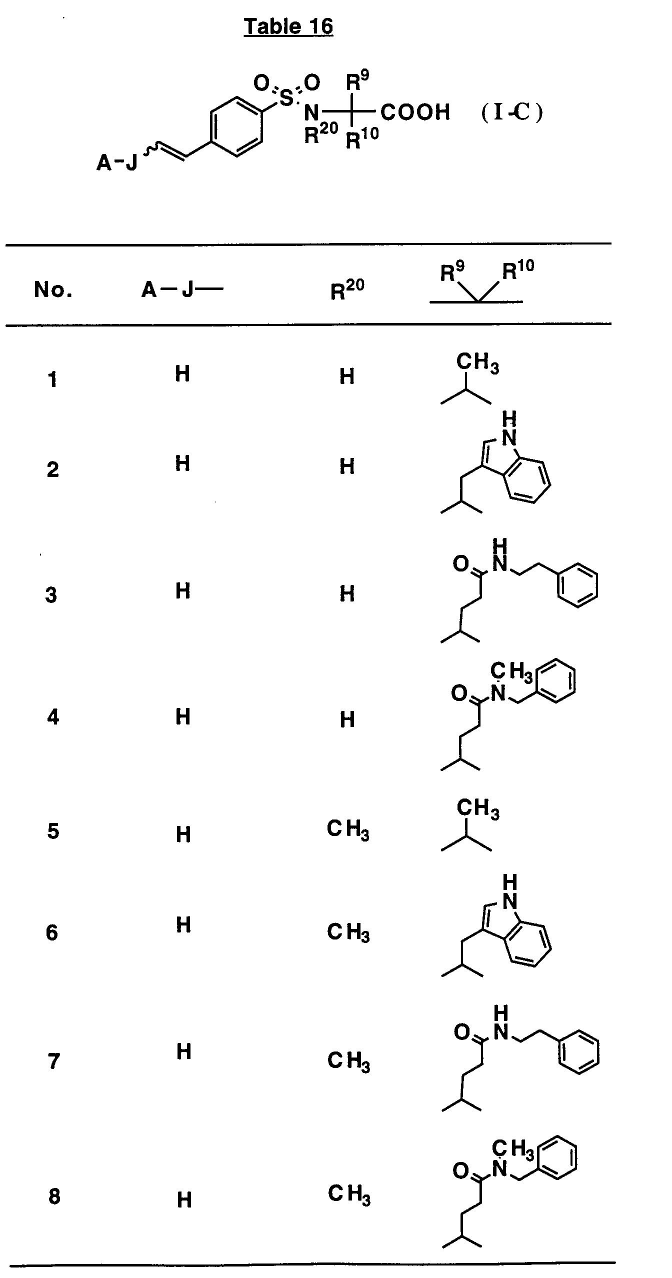

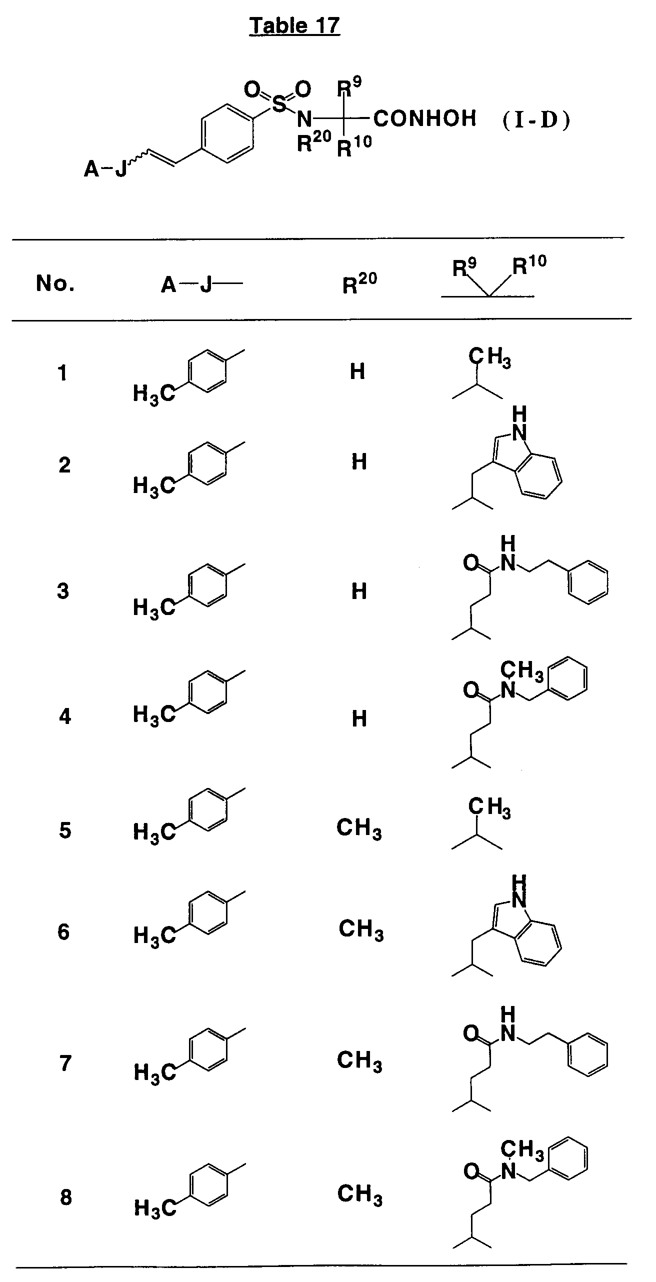

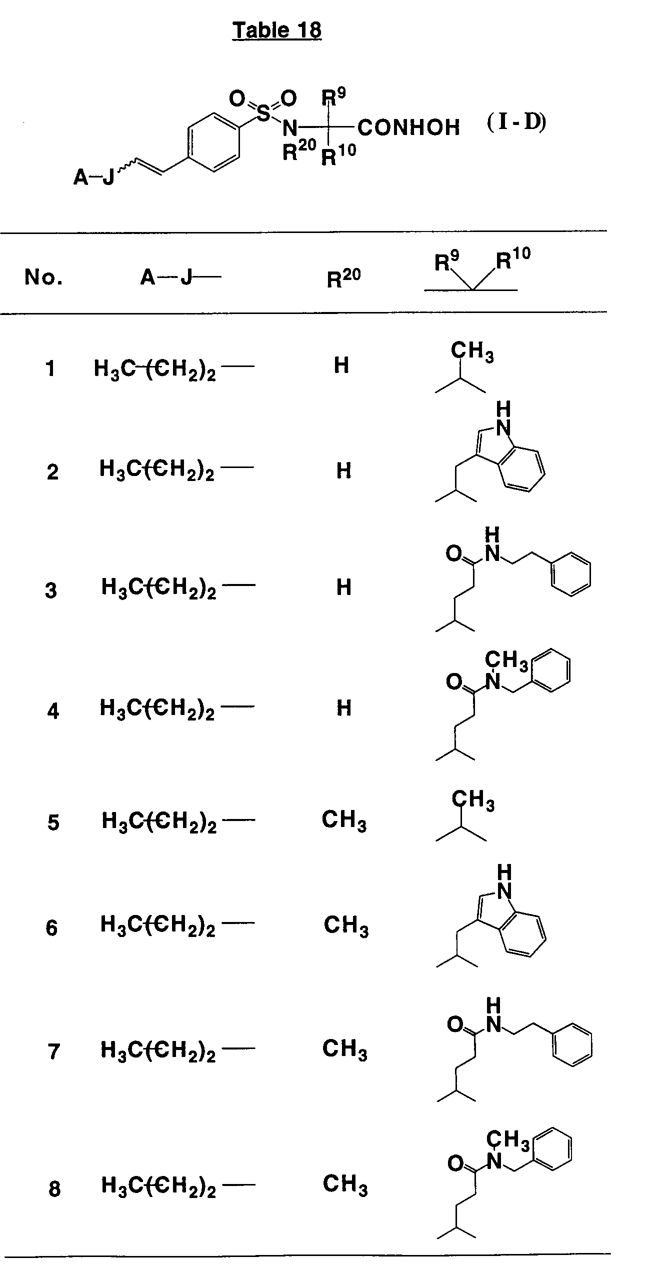

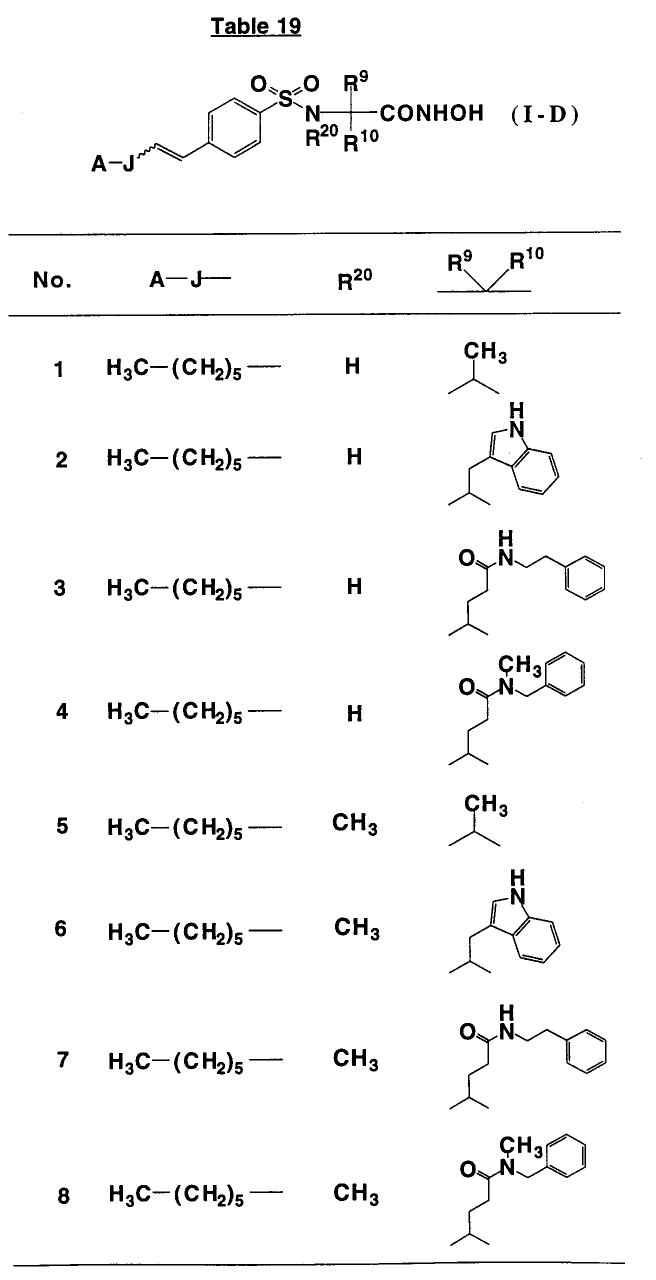

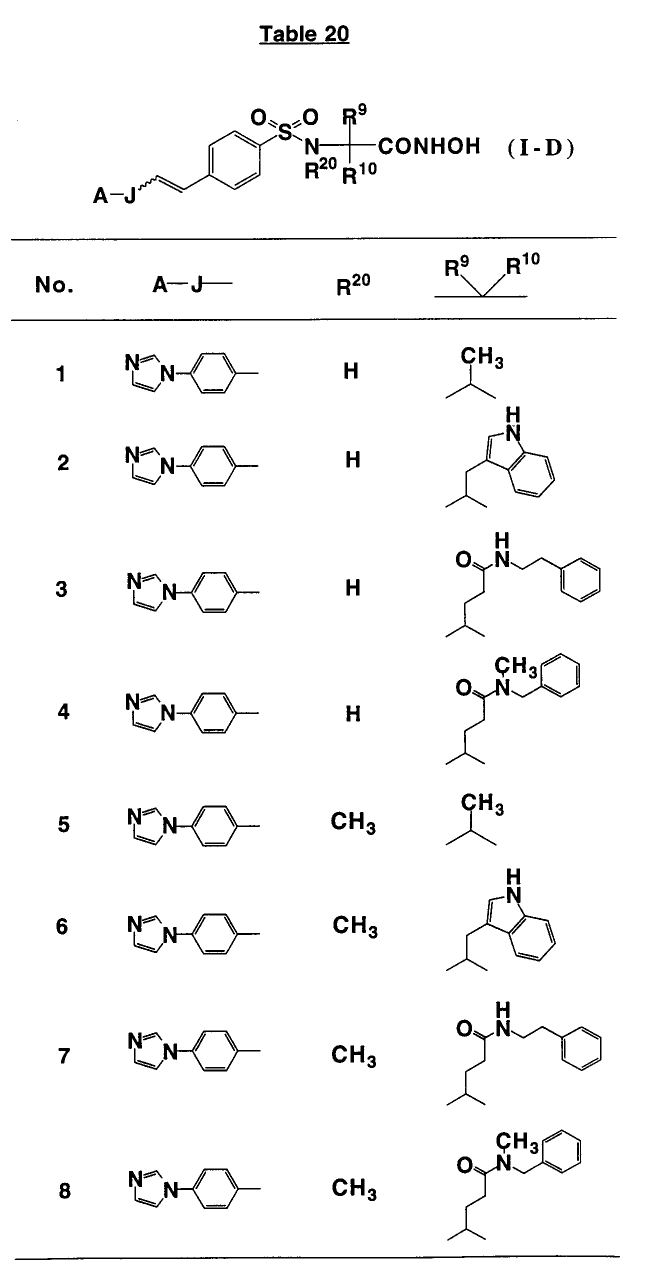

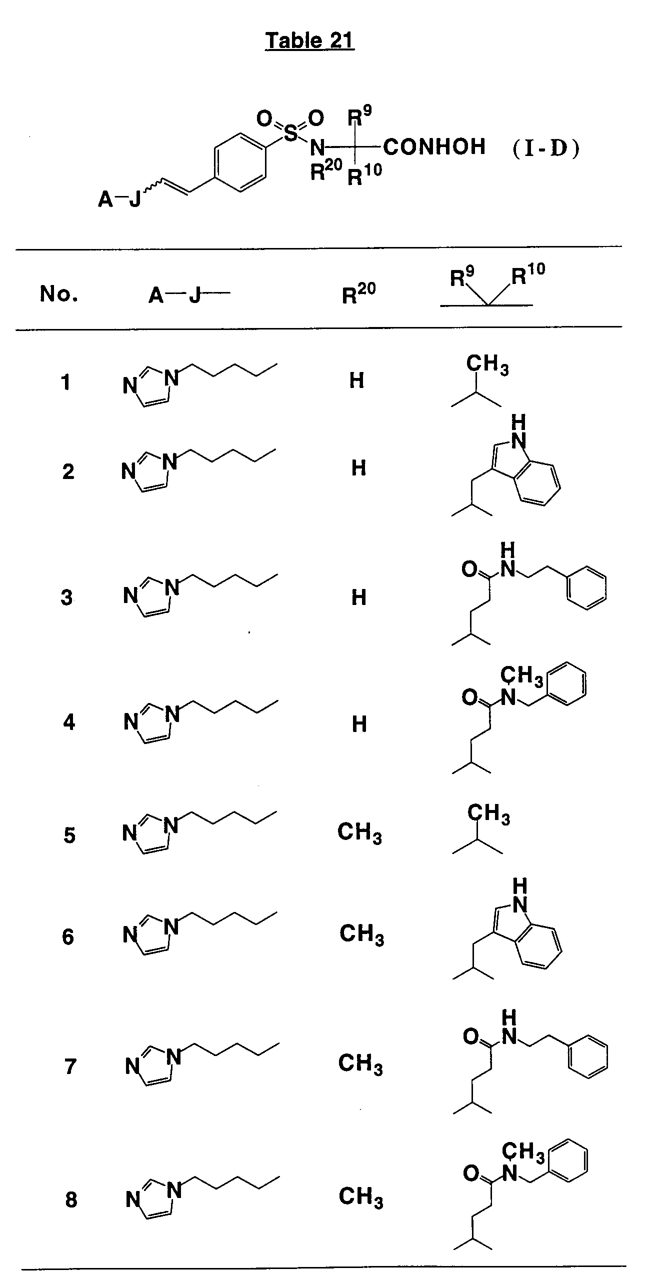

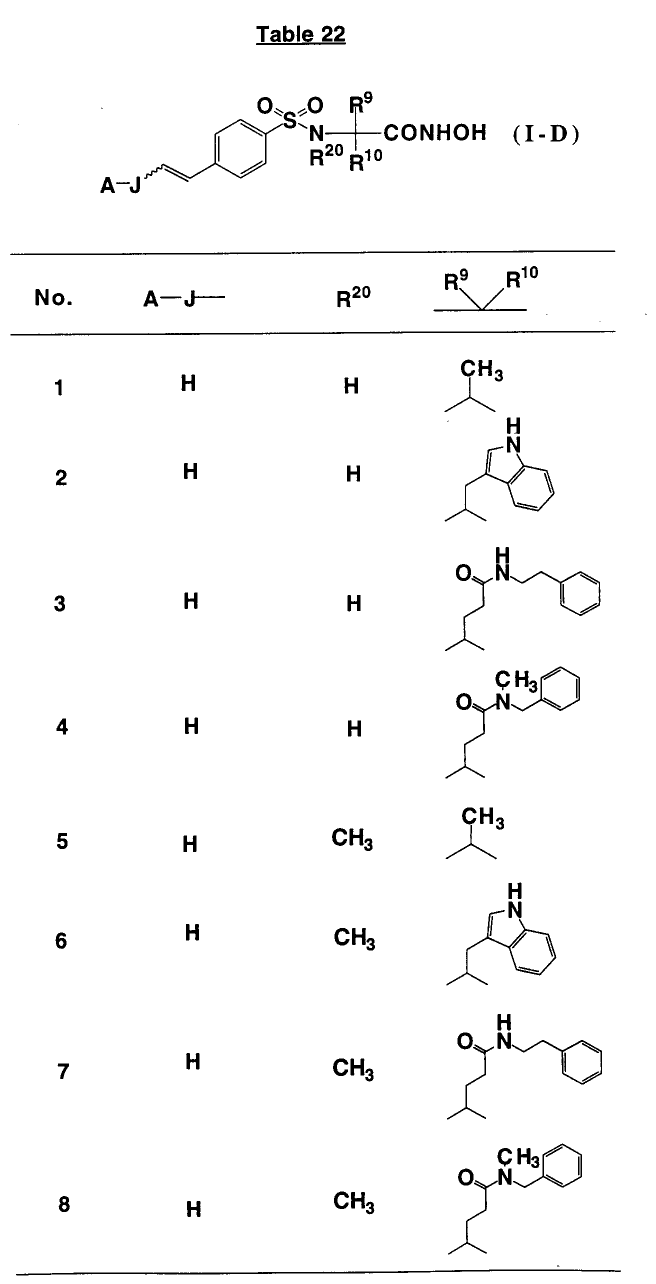

### Processes for the preparation of the compounds of the present invention

(1) Among the compounds of the present invention of formula (I), a compound wherein E is
   -C≡C-,
   and R² is COOR³, of formula (IA) 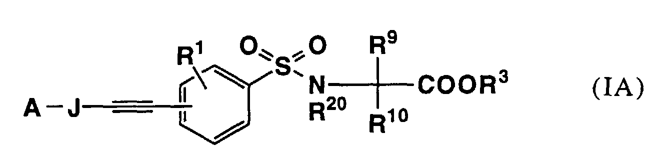
   (wherein all symbols are as defined above)
   may be prepared according to method (a) or (b) described below.
   (a) A compound wherein none of a substituent on a carbocyclic ring or a heterocyclic ring in A or R⁹, R¹⁰, R²⁰ or -COOR³ represents or includes -COOH, hydroxy or amino; i.e. a compound of formula (IA-1) 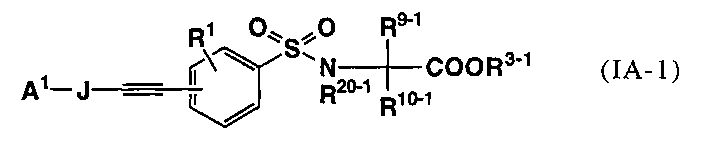
      (wherein R³⁻¹ is C₁₋₈alkyl, phenyl, or C₁₋₄alkyl substituted by phenyl, -OCOR¹⁵ (wherein R¹⁵ is as defined above), or -CONR¹⁶R¹⁷ (wherein R¹⁶ and R¹⁷ are as defined above); A¹ has the same meaning as A, with the proviso that -COOH, hydroxy or amino contained in A represents a protected form thereof; R⁹⁻¹ or R¹⁰⁻¹ is R⁹ or R¹⁰ respectively, with the proviso that -COOH, hydroxy, amino or a group containing them is a protected form thereof; R²⁰⁻¹ has the same meaning as R²⁰, with the proviso that -COOH, hydroxy, amino or a group containing them represents a protected form thereof, and the other symbols are as defined above, with the proviso that, the group of formula: 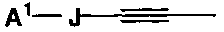 represents an ethynyl group protected by silyl, when J is a single bond and A¹ is hydrogen) may be prepared by reacting a compound of formula (III-1-1) 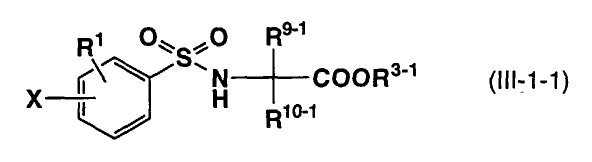
      (wherein X is halogen or trifluoromethanesulfonyloxy, and the other symbols represent the same meaning as described above) or a compound of formula (III-1-2) 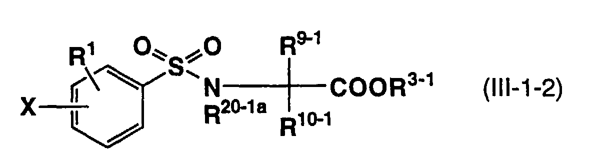
      (wherein R^{20-1a} is C₁₋₄alkyl, C₁₋₈alkoxycarbonyl, C₁₋₄alkoxycarbonyl substituted by phenyl or C₁₋₈alkyl substituted by a group selected from hydroxy, C₁₋₄alkoxy, benzoyloxy, -COOR²¹, -NR²²R²³, a carbocyclic ring and a heterocyclic ring, with the proviso that -COOH, hydroxy, amino in each group are protected, respectively), with an acetylene derivative, or acetylene protected by silyl(trimethylsilyl, t-butyl-dimethylsilyl etc.) of formula (IV-1) 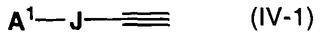
      (wherein all symbols are as defined above), or by reacting a compound of formula (III-1-1) with a compound of formula (IV-1) or acetylene protected by silyl(trimethylsilyl, or t-butyl-dimethylsilyl etc.), followed by introducing R^{20-1a}.

      The reaction described above is known, for example, it is carried out in an organic solvent (e.g. acetonitrile, tetrahydrofuran, dimethylformamide) in the presence or in the absence of a base (e.g. triethylamine) and cuprous iodide, using a catalyst (e.g. tetrakis (triphenylphosphine)palladium, dichlorobis(triphenylphosphine)palladium) at 0-100°C.
      Reaction for introduction of R^{20-1a} is known; for example, it is carried out by reacting alkyl halide (methyl iodide etc.) in an organic solvent (N,N-dimethylformamide etc.) in the presence of a base (potassium carbonate, cesium carbonate, sodium hydride etc.) at 0-50 °C, or by reacting an alcohol compound (1-(2-hydroxyethyl)imidazole etc.) in an organic solvent (tetrahydrofuran (THF) etc.) in the presence of triphenylphosphine and diethylazodicarboxylate, at 0-50 °C.
   (b) A compound (wherein any one of a substituent on a carbocyclic ring or a heterocyclic ring of A, or R⁹, R¹⁰, R²⁰, or -COOR₃ represents -COOH or a group containing it, or any one of a substituent on a carbocyclic ring or a heterocyclic ring of A, or R⁹, R¹⁰, or R²⁰ represents hydroxy or a group containing it, or any of a substituent of a carbocyclic ring or a heterocyclic ring of A, R⁹, R¹⁰, R²⁰ represents amino or a group containing it); a compound of formula (IA-2) 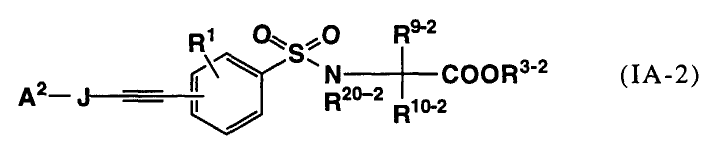
      (wherein A², R⁹⁻², R¹⁰⁻² and R³⁻² each have the same meaning as A, R⁹, R¹⁰, R²⁰, with the proviso that at least one of them represents -COOH, hydroxy, or amino, or a group containing them, and the other symbols are as defined above) may be prepared by alkali hydrolysis or deprotection of a compound of formula (IA-1) under acidic conditions or may be prepared by decomposition with a base of a compound of formula (II) 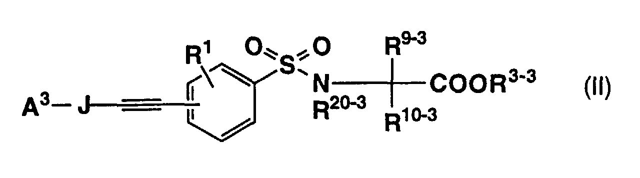
      (wherein R³⁻³ represents C₁₋₈alkyl, phenyl, or C₁₋₄alkyl substituted by phenyl, -OCOR¹⁵ (wherein R¹⁵ is as defined above), or -CONR¹⁶R¹⁷ (wherein R¹⁶ and R¹⁷ are as defined), or a group removable by decomposition with a base (e.g. 9-fluorenylmethyl), A³ has the same meaning as A, with the proviso that -COOR⁸, which is a substituent on a carbocyclic ring or a heterocyclic ring in A represents -COOR⁸⁻³ (R⁸⁻³ represents C₁₋₄alkyl, phenyl, C₁₋₄alkyl substituted by phenyl, a group removable with a base), hydroxy or amino represents a protected form thereof removable by decomposition with a base, R⁹⁻³ and R¹⁰⁻³ have the same meaning as R⁹ and R¹⁰, with the proviso that -COOH, hydroxy, amino or a group containing them represents a protected form thereof removable with a base, R²⁰⁻³ has the same meaning as R²⁰, with the proviso that a group containing -COOH, hydroxy, amino represents a protected form thereof, and the other symbols are as defined).

   Alkali hydrolysis is known, for example, it is carried out in an organic solvent (e.g. methanol, THF, dioxane) using hydroxide of alkali metal (e.g. sodium hydroxide, potassium hydroxide, lithium hydroxide), hydroxide of alkaline-earth metal (e.g. calcium hydroxide) or carbonate (e.g. sodium carbonate, potassium carbonate) or an aqueous solution thereof or the mixture thereof at 0-40 °C.
   Deprotection under acidic condition is known, for example, it is carried out in an organic solvent (e.g. methylenechloride, chloroform, dioxane, ethyl acetate, anisole), in an organic acid (e.g. trifluoroacetic acid (TFA), methanesulfonic acid, trimethylsilyl iodide), or in an inorganic acid (e.g. hydrochloric acid) or in the mixture thereof (e.g. hydrobromide-acetic acid), at 0-90 °C.
   Decomposition with a base is known, for example, it is carried out in an organic solvent (e.g. methylene chloride, N,N-dimethylformamide) using a base (e.g. piperidine, morpholine, diethylamine) at 0-50 °C.
(2) Among the compounds of the present invention of formula (I), a compound wherein E is
   ―CH=CH―,
   and R² is COOR³, of formula (IB) 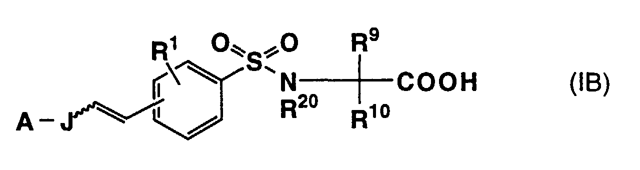
   (wherein all symbols are as defined above) may be prepared according to the method (a), (b), or (c) described below.

   (a) A compound wherein A is a carbocyclic ring or a heterocyclic ring, i.e. a compound of formula (IB-1) 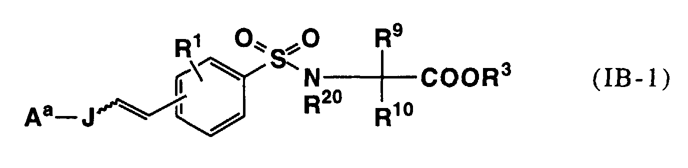
      (wherein A^{a} is a carbocyclic ring or a heterocyclic ring, and the other symbols are as defined above) may be prepared by dehydration of a compound of formula (VII) 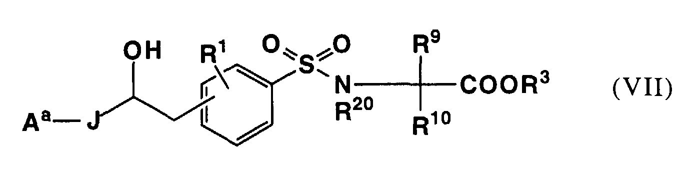
      (wherein all symbols are as defined above), optionally followed by deprotection.

      Dehydration reaction is known, for example, it is carried out in an organic solvent (e.g. toluene, benzene) in the presence of catalytic amount of p-toluenesulfonic acid, with refluxing.
   (b) A compound wherein J is a single bond, or C₁₋₈alkylene, and A is C₁₋₈alkyl, or a compound wherein J is C₁₋₈alkylene, and A is hydrogen, i.e. a compound of formula (IB-2) 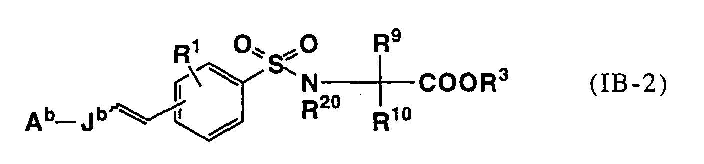
      (wherein in A^{b}-J^{b}-, A^{b} is C₁₋₈alkyl when J^{b} is a single bond or C₁₋₈alkylene, A is hydrogen when J^{b} is C₁₋₈alkylene and the other symbols are as defined above) can be prepared by reacting a compound of formula (III-1-1) or a compound of formula (III-1-2) with a vinylzirconium compound of formula (XII) 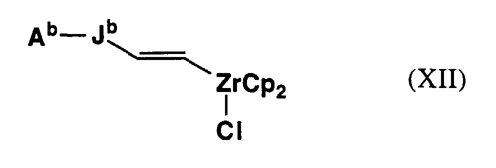
      (wherein Cp₂ is bis(cyclopentadienyl), and the other symbols are as defined above), optionally followed by deprotection.

      The reaction described above is known, for example, it is carried out in an organic solvent (e.g. tetrahydrofuran), by using a catalyst (e.g. tetrakis(triphenylphosphine) palladium) at 0-100 °C.
   (c) A compound wherein J is a single bond, and A is hydrogen; i.e. a compound of formula (IB-3) 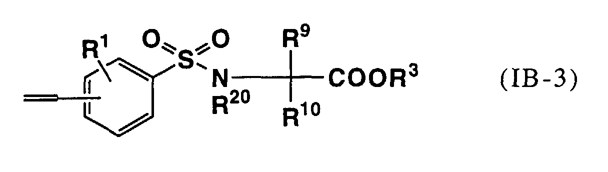
      (wherein all symbols are as defined above) can be prepared by reducing a compound of formula (IA-1) wherein the group of formula: 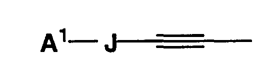 is ethynyl protected by silyl, followed by deprotection,

   Reduction reaction is known, for example, it is carried out in an organic solvent (e.g. THF, dioxane, diethyl ether, ethyl acetate, methanol, ethanol etc.) in the presence of Lindlar's catalyst (palladium-barium sulfate, Raney-nickel etc.), under atmosphere of hydrogen under atmospheric pressure or high pressure, at 0-200 °C.
(3) Among the compounds of formula (I), a compound wherein R² is CONHOR⁴; i.e. a compound of formula (IC) 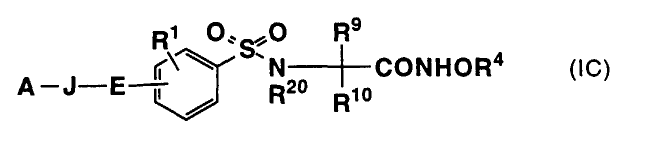
   (wherein all symbols are as defined above)
      can be prepared by amidation of a compound of formula (IA-1a) 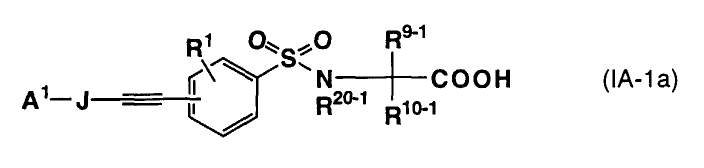
   (wherein all symbols are as defined above), or a compound of formula (IB-4) 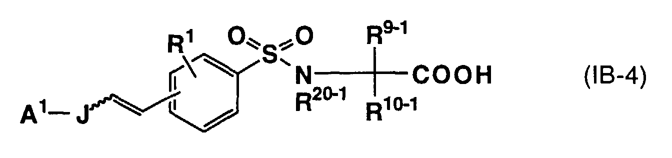
   (wherein all symbols are as defined above) and a compound of formula (XIII)

      H₂N―OR⁴ (XIII)
   (wherein R⁴ is as defined above) or protected hydroxylamine (e.g. H₂N-O-C(CH₃)₂-OCH₃, HN(Boc)-O-Boc (cf. Boc represents t-butoxycarbonyl), optionally followed by alkali hydrolysis and/or deprotection under acidic conditions.

The method of amidation is known. It includes the method
(1) via an acyl halide,
(2) via a mixed acid anhydride,
(3) using a condensing agent.

These methods are explained as follows.
(1) The method via an acyl halide, for example, may be carried out in an organic solvent (e.g. chloroform, methylene chloride, diethyl ether or tetrahydrofuran) or without a solvent, by reacting a carboxylic acid with an acid halide (e.g. oxalyl chloride or thionyl chloride) at -20 °C to reflux temperature, and the obtained acyl halide derivative may be reacted with an amine in an organic solvent (e.g. chloroform, methylene chloride, diethyl ether or tetrahydrofuran) in the presence of a tertiary amine (e.g. pyridine, triethyl amine, dimethyl aniline or dimethylaminopyridine) at 0-40 °C.
(2) The method via a mixed acid anhydride may be carried out, for example, by reacting a carboxylic acid with an acyl halide (e.g. pivaloyl chloride, tosyl chloride, mesyl chloride, ethyl chloroformate or isobutyl chloroformate) in an organic solvent (e.g. chloroform, methylene chloride, diethyl ether or tetrahydrofuran) or without a solvent, in the presence of a tertiary amine (e.g. pyridine, triethyl amine, dimethyl aniline or dimethylaminopyridine) at -20-40 °C, and the obtained mixed acid anhydride derivative may be reacted with a corresponding amine in an organic solvent (e.g. chloroform, methylene chloride, diethyl ether or tetrahydrofuran) at 0-40°C.
(3) The method using a condensing agent (e.g. 1,3-dicyclohexylcarbodiimide (DCC), 1-ethyl-3-[3-(dimethylamino)propyl]carbodiimide (EDC), 1,1'-carbonyldiimidazole (CDI), or 2-chloro-1-methylpyridinium iodide) may be carried out, for example, by reacting a carboxylic acid with an amine in an organic solvent (e.g. chloroform, methylene chloride, diethyl ether or tetrahydrofuran) or without a solvent at 0-40 °C, optionally in the presence of a tertiary amine (e.g. pyridine, triethylamine, dimethylaniline or dimethylaminopyridine) using a condensing agent, and optionally in the presence of 1 - hydroxybenzotriazole (HOBt).

The reactions described in (1), (2), and (3) may preferably be carried out under an inert gas (e.g. argon or nitrogen) to avoid water.

In the present invention, deprotection reaction means a general deprotection reaction which will be apparent to those skilled in the art (e.g. alkali hydrolysis, deprotection under acidic condition, or decomposition reaction with a base.). The desired compound of the present invention may be prepared with ease using these reactions. Alkali hydrolysis, deprotection under acidic condition or decomposition reaction with a base may be carried out as described above.

As will be apparent to those skilled in the art, t-butyl or benzyl may be used as protecting proups for carboxy or hydroxy. Other groups which may be removed with ease and selectively are also preferred. For example, the groups described in T. W. Greene, Protective Groups in Organic Synthesis, Wiley, New York, 1991, may be used. Benzyloxycarbonyl or t-butoxycarbonyl may be used as protecting groups for amino, but other groups which may be removed easily and selectively are also preferred. For example, -C(CH₃)₂-OCH₃ may be used. The desired compound of the present invention may be prepared with ease using these protecting groups.

Other groups than t-butyl or benzyl which may be removed easily and selectively are also preferred as protecting groups for hydroxylamine. For example, -C(CH₃)₂-OCH₃, t-butoxycarbonyl or benzyloxycarbonyl may be used. The desired compound of the present invention may be prepared using these protecting groups.

The compounds of the formulae (II), (III-1-1), (III-1-2), (VII), (IA-1a) and (IB-4) may be prepared by known methods, methods described in the following schemes 1-5, or methods described in the Examples.

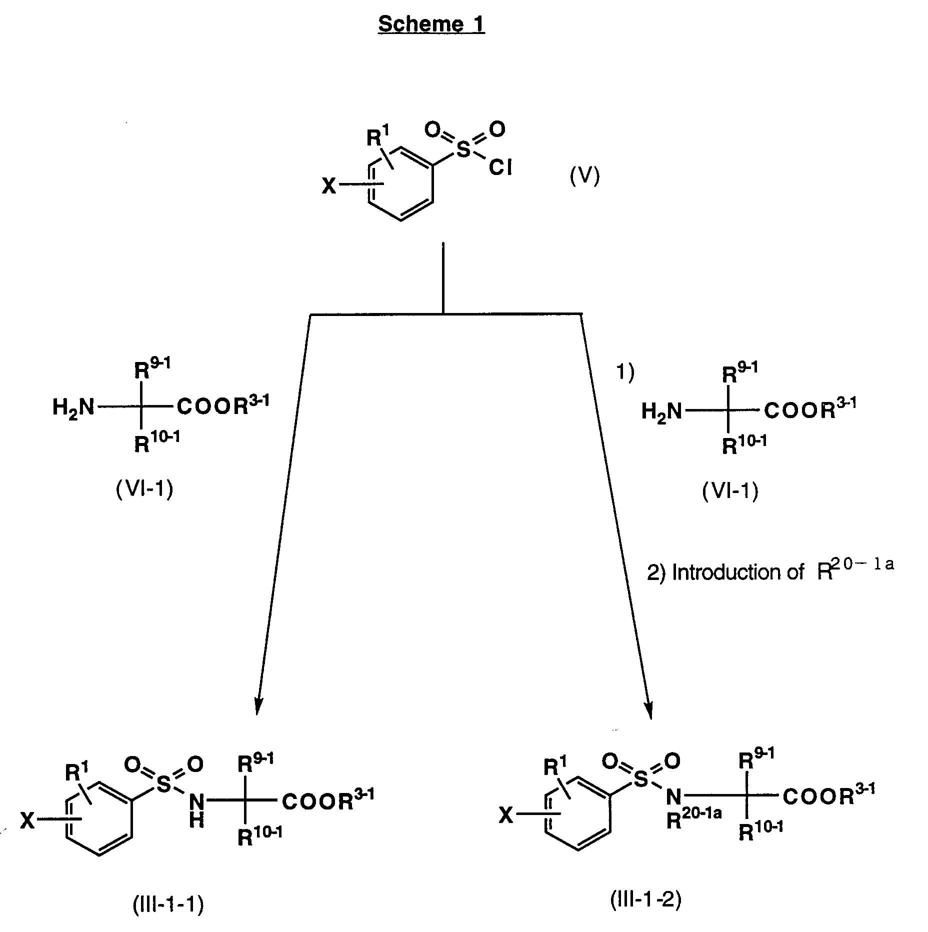

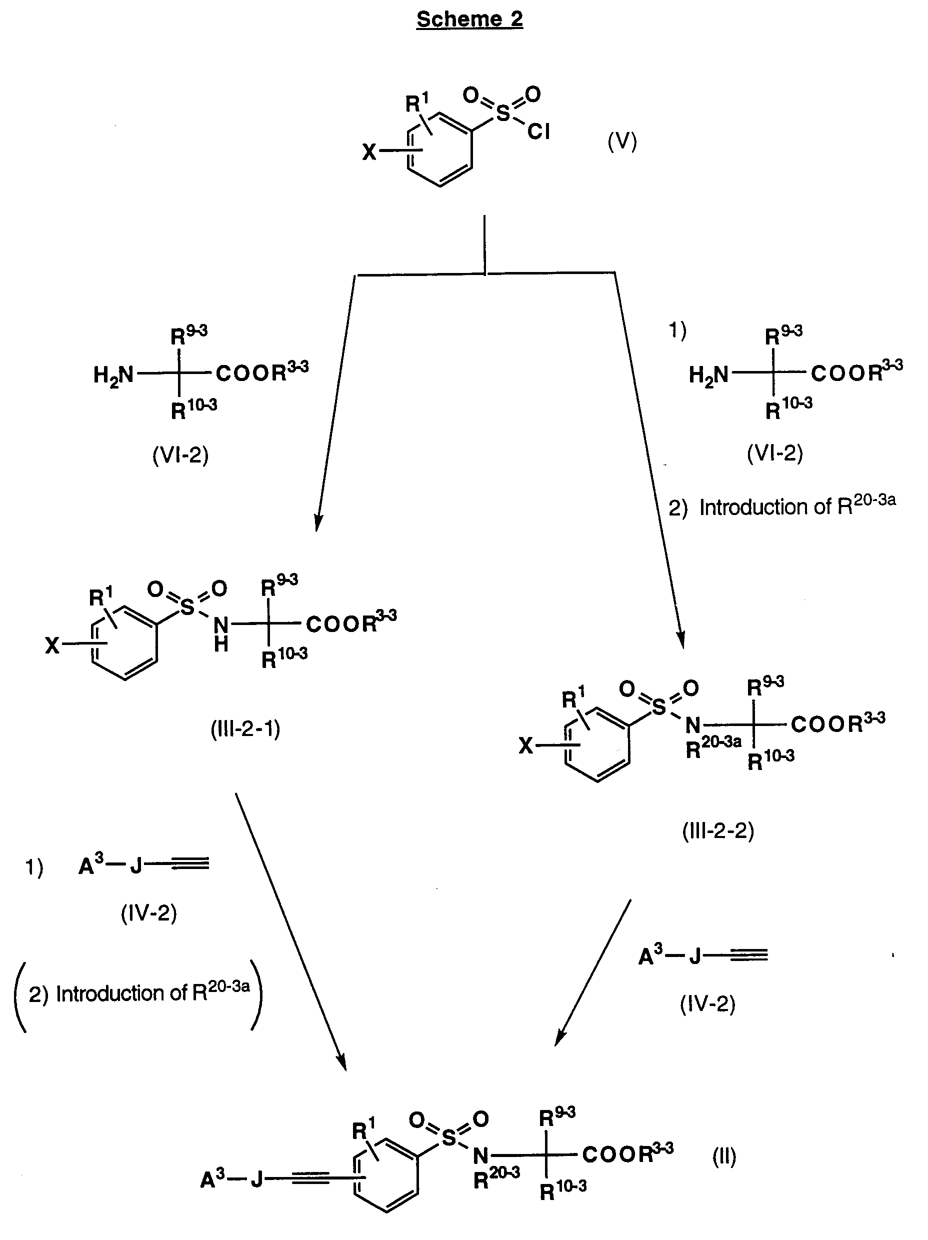

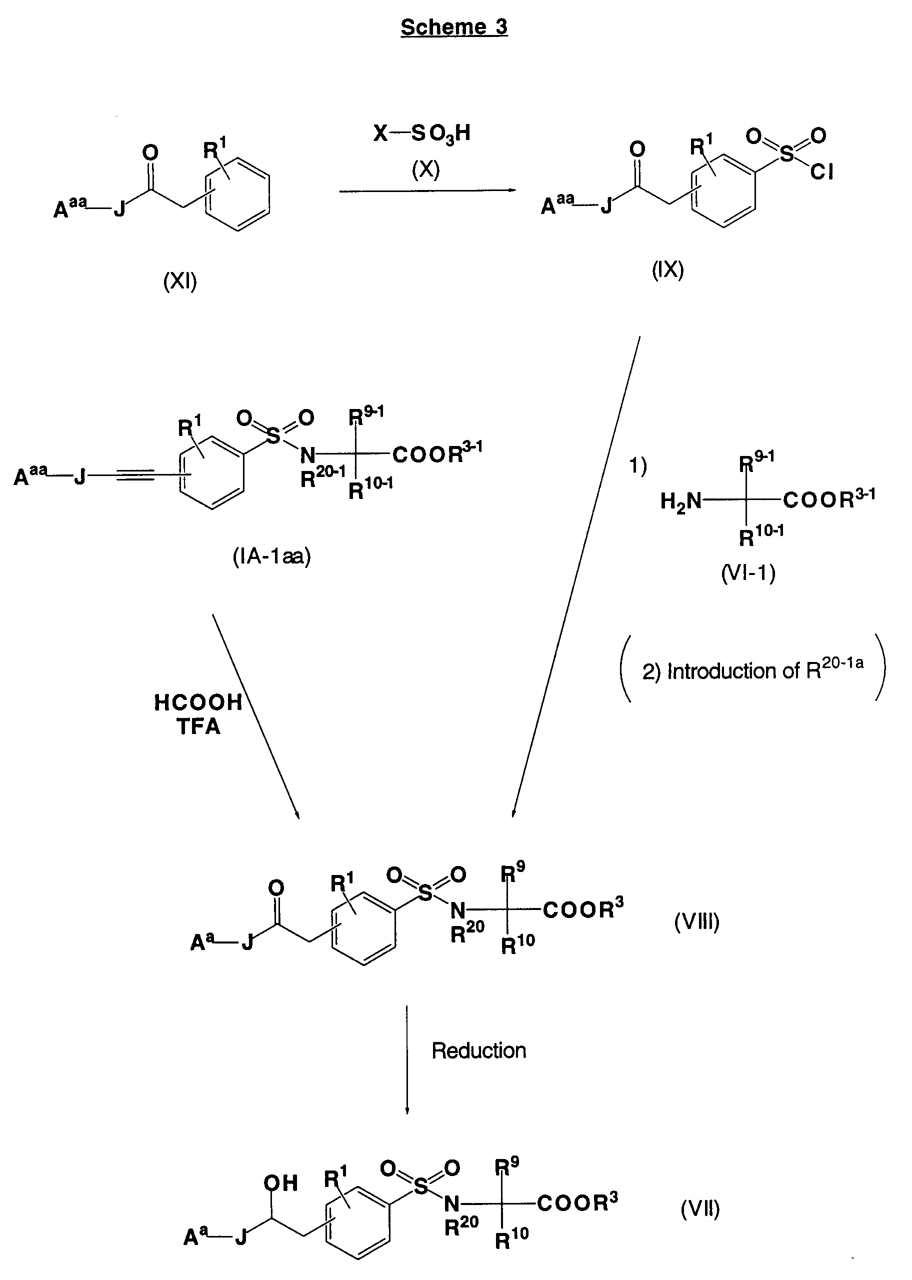

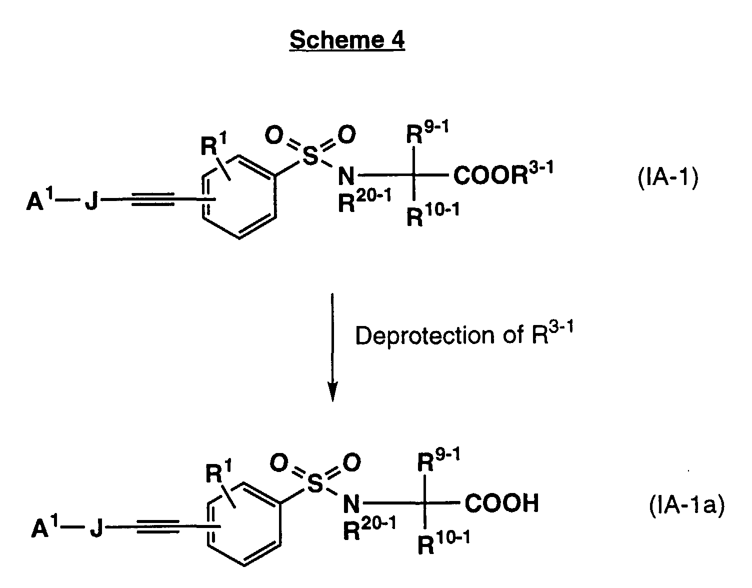

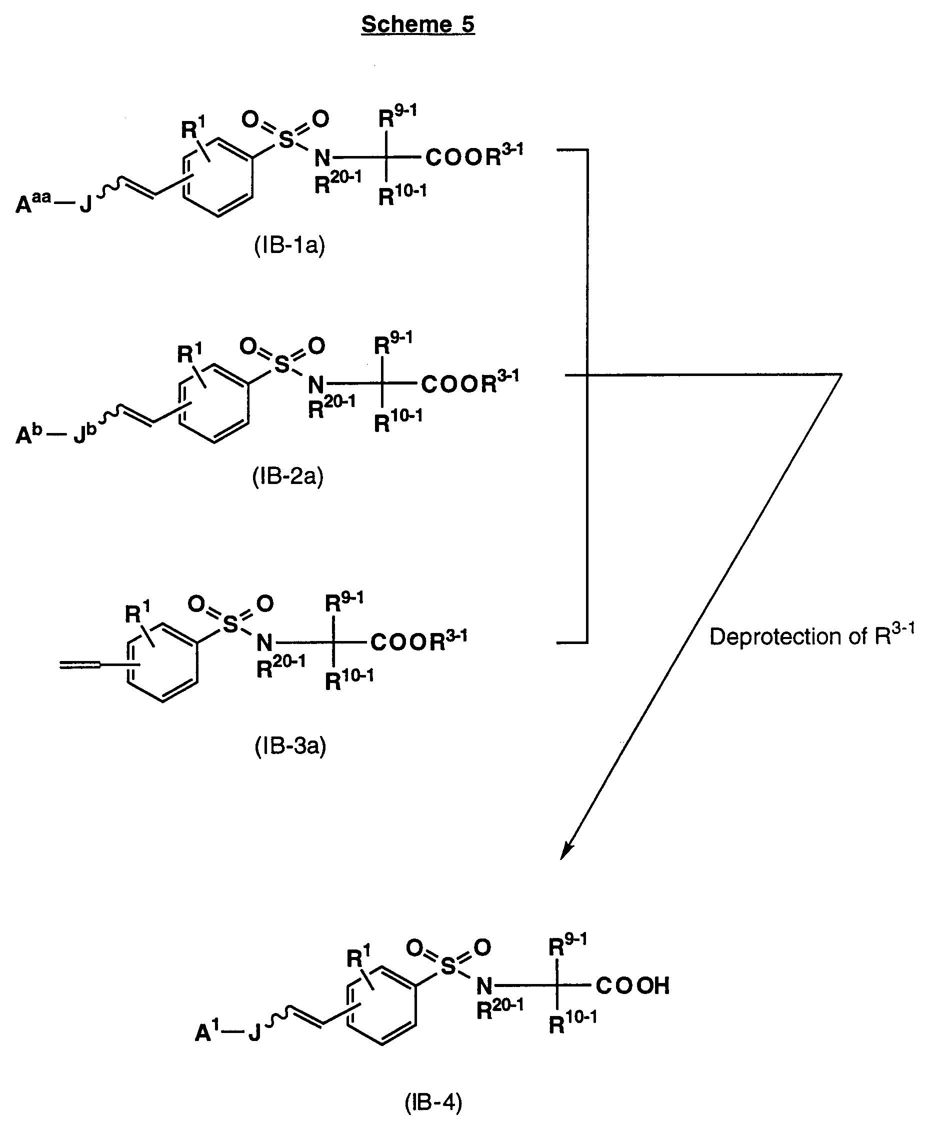

In the reaction scheme, R^{20-3a} is C₁₋₄alkyl, C₁₋₈alkoxycarbonyl, C₁₋₄alkoxycarbonyl substituted by phenyl, or C₁₋₈alkyl substituted by a group selected from hydroxy, C₁₋₄alkoxy, benzoyloxy, -COOR²¹, -NR²²R²³, a carbocyclic ring and a heterocyclic ring, with the proviso that, when -COOH, hydroxy or amino is included in R^{20-3a}, the group is protected by a group which may be removed with a base. A^{aa} is the same meaning as A^{a}, with the proviso that -COOH, hydroxy or amino which is a substituent on a carbocyclic ring or a heterocyclic ring in A^{a} each represents a protected form thereof. The other symbols represent the same meaning as described above.

Each reaction in the above schemes may be carried out by a known method. In the above schemes, the compounds of formulae (V) and (XI) used as starting materials may be known *per se*, or may be prepared by known methods. The other starting materials and reagents may be known *per se* or may be prepared by known methods.

In each reaction in the present specification, products may be purified by conventional techniques. For example, purification may be carried out by distillation at atmospheric or reduced pressure, by high performance liquid chromatography, by thin layer chromatography or by column chromatography using silica gel or magnesium silicate, by washing or by recrystallization. Purification may be carried out after each reaction, or after a series of reactions.

### [Pharmacological Activity]

The potency of inhibitory activity of the compound of the present invention of formula (I) against matrix metalloproteinase is confirmed as below.

### (1) Inhibitory activity against gelatinase A

### [Method]

Progelatinase A (7 µl; in assay buffer (90 µl)) was purified from human normal skin dermal fibroblasts (HNDF). It was activated by addition of 10 mM p-aminophenyl mercuric acetate (APMA) (10 µl) and preincubation at 37 °C for 1 hour. The solution (890 µl; the final concentration was 13.5 µl) of the synthetic substrates (MOCAc-Pro-Leu-Gly-A₂pr(Dnp)-Ala-Arg-NH₂) was mixed with various concentrations of the test compound or a solution without it (10 µl) and the mixture was preincubated at 37 °C for 5 minutes. The activated gelaninase A prepared above (7 µl/tube, 100 µl) was added to the reaction mixture and further incubated at 37 °C for 20 minutes. The enzyme reaction was terminated by addition of 0.1 M sodium acetate buffer (2ml; pH 4.0).

The IC₅₀ value was determined by measuring the fluorescent intensity (Ex = 328 nm, and Em = 393 nm) in this solution. The results are shown in Table 23.

**Table 23**

| No. | IC₅₀(uM) |
|---|---|
| 2(1) | 0.013 |
| 2(4) | 0.0065 |
| 2(18) | 0.011 |
| 2(27) | 0.0033 |
| 3(1) | 0.0062 |
| 6(1) | 0.0012 |
| 6(2) | 0.0002 |
| 6(4) | 0.0007 |
| 6(11) | 0.0024 |
| 6(17) | 0.0008 |
| 10 | 0.0075 |
| 10(3) | 0.0004 |
| 10(4) | 0.0007 |

### (2) Inhibitory activity against collagenase

### [Method]

Trypsin (45 µl; 1 mg/ml) was added to a solution of procollagenase (5 µM) purified from human normal dermal fibloblasts (HNDF) in assay buffer (105 µl). The solution was preincubated in order to activate the enzyme at 37°C for 1 minute.

Trypsin was inactivated by addition of soybean trypsin inhibitor (SBTI; 50 µl). Solutions (20 µl) of synthetic substrate (Ac-Pro-Leu-Gly-[2-mercapto-4-methyl-pentanoyl]-Leu-Gly-OEt) (105 µl; the final concentration 1.33 M) with or without various concentrations of the test compound were preincubated for 5 minutes at 26 °C.

To the solutions, activated enzyme (75 µl/tube, 50 µl) prepared above was added. The mixtures were incubated at 26 °C for 10 minutes.

Absorption at 324 nm was measured at 40 points during the 10 minutes.

Vₘₐₓ value was determined as measured value in 30 points therein.
The results are shown in Table 24.

**Table 24**

| No. | IC₅₀(uM) |
|---|---|
| 6(17) | 0.063 |
| 6(19) | 0.065 |
| 10(13) | 0.29 |

### [Toxicity]

The toxicity of the compounds of the present invention is very low and therefore the compounds may be considered safe for pharmaceutical use.

### [Application to pharmaceutical drugs]

Inhibition of matrix metalloproteinases, for example, gelatinases, stromlycins, or collagenases, is useful for prevention an/or treatment of diseases, for example, rheumatoid diseases, arthrosteitis, abnormal bone resorption, osteoporosis, periodontitis, interstitial nephritis, arteriosclerosis, pulmonary emphysema, cirrhosis, cornea injury, metastasis or invasion of tumor cells, a disease induced by proliferation of tumor cells, an autoimmune disease (e.g. Crohn's disease, Sjogren's syndrome), a disease caused by vascular emigration or infiltration of leukocytes, arterialization in animals including human beings, especially human beings.

For the purpose described above, the compounds of formula (I), of the present invention, non-toxic salts thereof, acid addition salts thereof or hydrates thereof may normally be administered systemically or locally, usually by oral or parenteral administration.

The doses to be administered are determined depending upon, for example, age, body weight, symptom, the desired therapeutic effect, the route of administration, and the duration of the treatment. In the human adult, the doses per person are generally from 1 mg to 1000 mg, by oral administration, up to several times per day, and from 1 mg to 100 mg, by parenteral administration (preferably intravenous administration), up to several times per day, or continuous administration for from 1 to 24 hours per day from vein.

As mentioned above, the doses to be used depend upon various conditions. Therefore, there are cases wherein doses lower than or greater than the ranges specified above may be used.

The compounds of the present invention may be administered in the form of, for example, solid compositions, liquid compositions or other compositions for oral administration, injections, liniments or suppositories for parenteral administration.

Solid compositions for oral administration include compressed tablets, pills, capsules, dispersible powders and granules.

Capsules include hard capsules and soft capsules.

In such solid compositions, one or more of the active compound(s) may be admixed with at least one inert diluent (e.g. lactose, mannitol, glucose, hydroxypropyl cellulose, microcrystalline cellulose, starch, polyvinyl pyrrolidone or magnesium metasilicate aluminate). The compositions may also comprise, as is normal practice, additional substances other than inert diluents: e.g. lubricating agents (e.g. magnesium stearate), disintegrating agents (e.g. cellulose calcium glycolate), stabilizing agents (e.g. lactose), and agents to assist dissolution (e.g. glutamic acid or asparatic acid). The tablets or pills may, if desired, be coated with a film of gastric or enteric material (e.g. sugar, gelatin, hydroxypropyl cellulose or hydroxypropylmethyl cellulose phthalate), or be coated with two or more films. Further, coating may include containment within capsules of absorbable materials such as gelatin.

Liquid compositions for oral administration include pharmaceutically acceptable emulsions, solutions, syrups and elixirs. In such compositions, one or more of the active compound(s) may be contained in inert diluent(s) commonly used in the art (e.g. purified water or ethanol). Besides inert diluents, such compositions may also comprise adjuvants (e.g. wetting agents or suspending agents), sweetening agents, flavouring agents, perfuming agents, and preserving agents.

Other compositions for oral administration include spray compositions which may be prepared by known methods and which comprise one or more of the active compound(s). Spray compositions may comprise additional substances other than inert diluents: e.g. stabilizing agents (e.g. sodium sulfite hydride), isotonic buffers (e.g. sodium chloride, sodium citrate or citric acid). For preparation of such spray compositions, for example, the method described in the United States Patent No.2,868,691 or No.3,095,355 may be used.

Injections for parenteral administration include sterile aqueous or non-aqueous solutions, suspensions and emulsions. Aqueous solutions and suspensions include distilled water for injection and physiological salt solution. Non-aqueous solutions and suspensions may include propylene glycol, polyethylene glycol, vegetable oil such as olive oil, alcohol such as ethanol or POLYSORBATE80 (registered trade mark).

Injections may comprise additional ingredients other than inert diluents: e.g. preserving agents, wetting agents, emulsifying agents, dispersing agents, stabilizing agents, assisting agents such as agents to assist dissolution (e.g. glutamic acid or aspartic acid).

They may be sterilized for example, by filtration through a bacteria-retaining filter, by incorporation of sterilizing agents in the compositions or by irradiation. They may also be manufactured in the form of sterile solid compositions which may be dissolved in sterile water or some other sterile diluent(s) for injection immediately before use.

Other compositions for parenteral administration include liquids for external use, and ointment, endermic liniments, suppositories for rectal administration and pessaries for vaginal administration which comprise one or more of the active compound(s) and may be prepared by methods known *per se.*

### Reference Example and Examples

The following Reference Examples and Examples illustrate the present invention, but do not limit the present invention.

The solvents in parentheses show the eluting or developing solvents and the ratios of the solvents used are by volume in chromatographic separations or TLC.

The solvents in parentheses in NMR show the solvents used in measurement.

### Reference Example 1

### N-(4-Bromophenylsulfonyl)-D-phenylalanine t-butyl ester

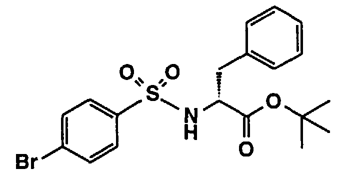

To a solution of D-phenylalanine t-butyl ester (1.29 g) in pyridine (10 ml) 4-bromobenzenesulfonyl chloride (1.28 g) was added slowly under cooling with ice. The mixture was removed from ice bath, and stirred at room temperature for 1 hour. The reaction mixture was concentrated. The residue was extracted with ethyl acetate. The extract was washed with 1N hydrochloric acid, water, a saturated aqueous solution of sodium chloride, dried over anhydrous magnesium sulfate, and concentrated. The residue was purified by column chromatography on silica gel (hexane : ethyl acetate = 5 : 1) to give the title compound (1.83 g) having the following physical data.
TLC: Rf 0.37 (hexane : ethyl acetate = 3 : 1);
NMR (CDCl₃): δ 7.60 (2H, d, J=8.8Hz), 7.56 (2H, d, J=8.8Hz), 7.25 (3H, m), 7.13 (2H, m), 5.09 (1H, d, J=9.4Hz), 4.08 (1H, dt, J=9.4Hz, 6.4Hz), 3.01 (2H, d, J=6.4Hz), 1.24 (9H, s).

### Reference Example 2

### N-(4-Bromophenylsulfonyl)-N-t-butoxycarbonyl-D-alanine t-butyl ester

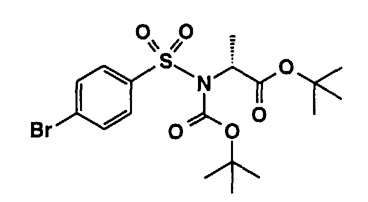

N-(4-Bromophenylsulfonyl)-N-t-butoxycarbonyl-D-alanine t-butyl ester (8.0 g) obtained by the same procedure as described in Reference Example 1 (using 4-bromobenzenesulfonyl chloride and D-alanine t-butyl ester), di-t-butyl carbonate (5.6 ml), 4-(dimethylamino)pyridine (269 mg) and acetonitrile (50 ml) were mixed at 0 °C, and the mixture was stirred at room temperature for 3 hours. The reaction mixture was concentrated. The residue was extracted with ethyl acetate. The extract was washed with 1N hydrochloric acid, a saturated aqueous solution of sodium bicarbonate, a saturated aqueous solution of sodium chloride, dried over anhydrous magnesium sulfate, and concentrated to give the title compound (10.8 g) having the following physical data.
TLC : Rf 0.57 (hexane : ethyl acetate = 4 : 1).

### Example 1

### N-[4-[2-(4-Methylphenyl)ethynyl]phenylsulfonyl]-D-phenylalanine t-butyl ester

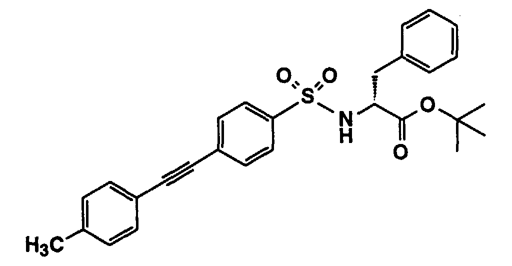

A mixture of the compound prepared in Reference Example 1 (440 mg), 4-methyl phenyl acetylene (133 µl), 10% palladium on carbon (42 mg), triphenyl phosphine (42 mg), cuprous iodide (7.6 mg), acetonitrile (1.25 ml) and triethylamine (2.5 ml) was refluxed for 3 hours under atmosphere of argon. The reaction mixture was cooled down, unsolved precipitate was filtered off over celite, and the filtrate was concentrated. The residue was purified by column chromatography on silica gel (chloroform) to give the title compound (456 mg) having the following physical data.
TLC : Rf 0.35 (hexane : ethyl acetate = 3 : 1) ;
NMR (CDCl₃): δ 7.73 (2H, d, J=8.8Hz), 7.55 (2H, d, J=8.8Hz), 7.43 (2H, d, J=8.3Hz), 7.25-7.10 (7H, m), 5.10 (1H, d, J=9.0Hz), 4.09 (1H, dt, J=9.0Hz, 6.1Hz), 3.03 (2H, d, J=6.1Hz), 2.39 (3H, s), 1.24 (9H, s).

### Examples 1(1) - 1(34)

By the same procedure as Reference Example 1 or Reference Example 2, and Example 1, by using 4-bromobenzenesulfonyl chloride, a corresponding amino acid derivative, and a corresponding acetylene derivative, following compounds were obtained.

### Example 1(1)

### N-[4-[2-(4-Methylphenyl)ethynyl]phenylsulfonyl]-N-t-butoxycarbonyl-D-alanine t-butyl ester

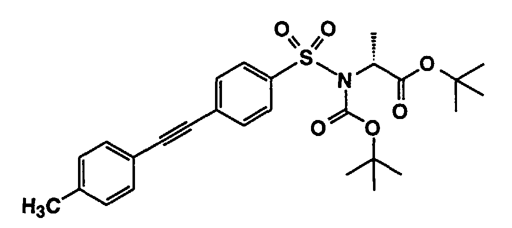

TLC : Rf 0.18 (hexane : ethyl acetate = 19 : 1) ;
NMR (CDCl₃): δ 7.97 (2H, d, J=8.6Hz), 7.62 (2H, d, J=8.6Hz), 7.44 (2H, d, J=8.0Hz), 7.18 (2H, d, J=8.0Hz), 5.06 (1H, q, J=6.8Hz), 2.39 (3H, s), 1.64 (3H, d, J=6.8Hz), 1.45 (9H, s), 1.33 (9H, s).

### Example 1(2)

### N-[4-(1-Pentynyl)phenylsulfonyl]-N-t-butoxycarbonyl-D-alanine t-butyl ester

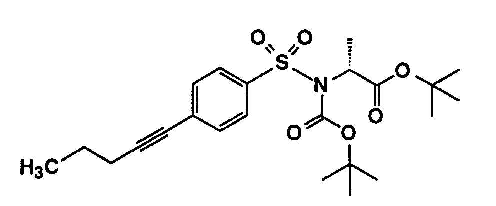

TLC : Rf 0.60 (hexane : ethyl acetate = 4 : 1) ;
NMR (CDCl₃): δ 7.93 (2H, d, J=8.6Hz), 7.50 (2H, d, J=8.6Hz), 5.05 (1H, q, J=6.0Hz), 2.43 (2H, t, J=6.0Hz), 1.65 (5H, m), 1.43 (9H, s), 1.33 (9H, s), 1.06 (3H, t, J=6.0Hz).

### Example 1(3)

### N-[4-(2-Phenylethynyl)phenylsulfonyl]-N-t-butoxycarbonylglycine t-butyl ester

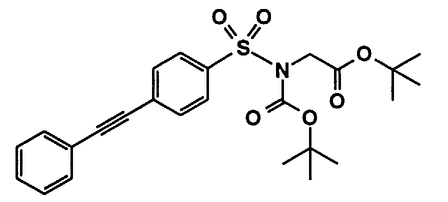

TLC : Rf 0.27 (hexane : ethyl acetate = 9 : 1) ;
NMR (CDCl₃): δ 8.05 (2H, d, J=8.2Hz), 7.65 (2H, d, J=8.2Hz), 7.61-7.50 (2H, m), 7.44-7.34 (3H, m), 4.47 (2H, s), 1.49 (9H, s), 1.34 (9H, s).

### Example 1(4)

### N-[4-[2-(4-Methylphenyl)ethynyl]phenylsulfonyl]-D-tryptophan methyl ester

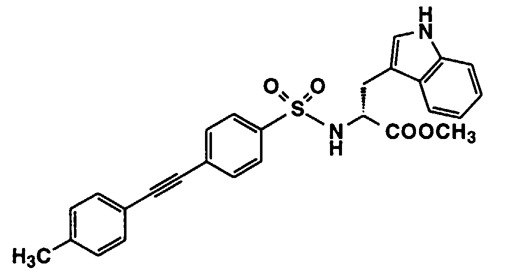

TLC : Rf 0.34 (chloroform : ethyl acetate = 9 : 1) ;
NMR (DMSO-d₆): δ 10.83 (1H, s), 8.60 (1H, d, J=8.8Hz), 7.59 (2H, d, J=8.8Hz), 7.53 (2H, d, J=8.8Hz), 7.49 (2H, d, J=8.2Hz), 7.36-7.22 (4H, m), 7.10-6.90 (3H, m), 4.08-3.94 (1H, m), 3.36 (3H, s), 3.07 (1H, dd, J=6.8Hz, 14.4Hz), 2.90 (1H, dd, J=8.0Hz, 14.4Hz), 2.35 (3H, s).

### Example 1(5)

### N-[4-[2-(Pyridine-2-yl)ethynyl]phenylsulfonyl]glycine t-butyl ester

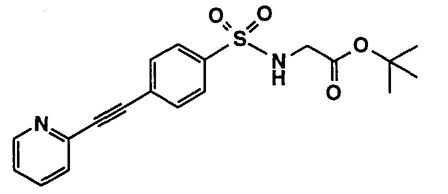

TLC : Rf 0.23 (hexane : ethyl acetate = 1 : 1) ;
NMR (CDCl₃): δ 8.64 (1H, m), 7.85 (2H, d, J=8.6Hz), 7.75-7.70 (3H, m), 7.56 (1H, m), 7.30 (1H, m), 5.06 (1H, t, J=5.5Hz), 3.65 (2H, d, J=5.5Hz), 1.36 (9H, s).

### Example 1(6)

### N-[4-[2-(4-Methoxyphenyl)ethynyl]phenylsulfonyl]glycine t-butyl ester

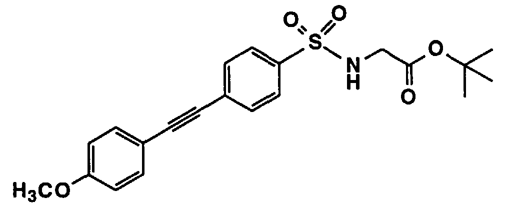

TLC : Rf 0.51 (hexane : ethyl acetate = 1 : 1) ;
NMR (CDCl₃): δ 7.81 (2H, d, J=8.6Hz), 7.61 (2H, d, J=8.6Hz), 7.48 (2H, d, J=8.8Hz), 6.90 (2H, d, J=8.8Hz), 5.05 (1H, t, J=5.4Hz), 3.84 (3H, s), 3.69 (2H, d, J=5.4Hz), 1.36 (9H, s).

### Example 1(7)

### N-[4-[2-(4-Methylphenyl)ethynyl]phenylsulfonyl]-D-valine t-butyl ester

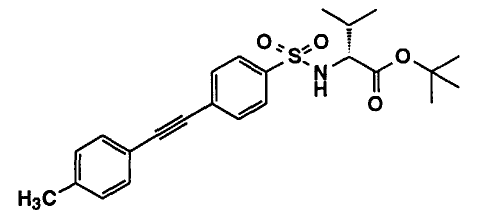

NMR (CDCl₃): δ 7.80 (2H, d, J = 8.2 Hz), 7.59 (2H, d, J = 8.2 Hz), 7.43 (2 H, d, J=8.5 Hz), 7.17 (2H, d, J=8.5 Hz), 5.13 (1 H, d, J = 9.5 Hz), 3.63 (1H, dd, J = 9.5, 5.1 Hz), 2.38 (3 H, s), 2.04 (1 H, m), 1.24 (9H, s), 1.00 (3H, d, J=6.4 Hz), 0.84 (3H, d, J=6.4Hz).

### Example 1(8)

### N-[4-[2-(2-Methylphenyl)ethynyl]phenylsulfonyl]-D-alanine t-butyl ester

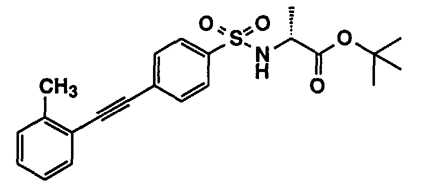

TLC : Rf 0.47 (hexane : ethyl acetate = 2 : 1) ;
NMR (CDCl₃): δ 7.83 (2H, d, J=8.5Hz), 7.62 (2H, d, J=8.5Hz), 7.55-7.15 (4H, m), 5.30 (1H, d, J=8.4Hz), 3.88 (1H, dt, J=7.2, 8.4Hz), 2.51 (3H, s), 1.36 (3H, d, J=7.2Hz), 1.32 (9H, s).

### Example 1(9)

### N-[4-(1-Heptenyl)phenylsulfonyl]-D-alanine t-butyl ester

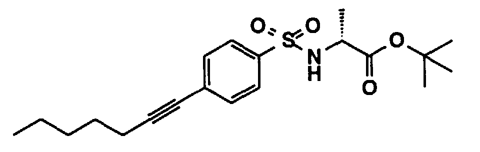

TLC : Rf 0.36 (hexane : ethyl acetate = 4 : 1) ;
NMR (CDCl₃): δ 7.69 (2H, d, J=8.0Hz), 7.40 (2H, d, J=8.0Hz), 5.11-5.23 (1H, br.), 3.61-3.87 (1H, m), 2.36 (2H, t, J=7.0Hz), 1.14-1.63 (6H, m), 1.33 (3H, d, J=7.0Hz), 1.28 (9H, s), 0.89 (2H, t, J=7.0Hz).

### Example 1(10)

### N-[4-(1-Hexynyl)phenylsulfonyl]-D-alanine t-butyl ester

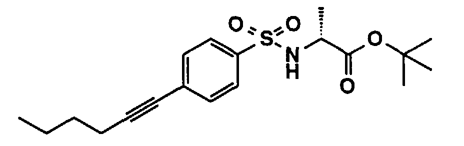

TLC : Rf 0.40 (hexane : ethyl acetate = 3 : 1) ;
NMR (CDCl₃): δ 7.69 (2H, d, J=9.0Hz), 7.41 (2H, d, J=9.0Hz), 5.11-5.23 (1H, br.), 3.70-3.87 (1H, m), 2.37 (2H, t, J=7.0Hz), 1.33-1.62 (4H, m), 1.29 (3H, d, J=7.0Hz), 1.24 (9H, s), 0.89 (2H, t, J=7.0Hz).

### Example 1(11)

### N-[4-(1-Octynyl)phenylsulfonyl]-D-alanine t-butyl ester

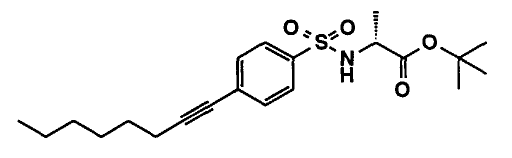

TLC : Rf 0.50 (hexane : ethyl acetate = 3 : 1) ;
NMR (CDCl₃): δ 7.74 (2H, d, J=8.0Hz), 7.46 (2H, d, J=8.0Hz), 5.22 (1H, d, J=8.0Hz), 3.72-3.92 (1H, m), 2.40 (2H, t, J=7.0Hz), 1.13-1.68 (8H, m), 1.33 (3H, d, J=7.0Hz), 1.28 (9H, s), 0.89 (2H, t, J=7.0Hz).

### Example 1(12)

### N-[4-[2-3-Methylphenyl)ethynyl]phenylsulfonyl]-D-alanine t-butyl ester

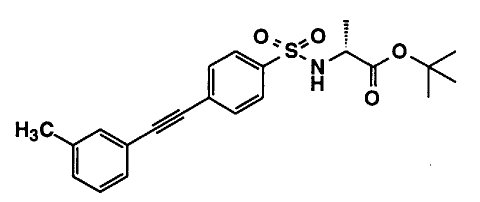

TLC : Rf 0.47 (hexane : ethyl acetate = 2 : 1) ;
NMR (CDCl₃): δ 7.82 (2H, d, J=8.6Hz), 7.62 (2H, d, J=8.6Hz), 7.40-7.15 (4H, m), 5.26 (1H, d, J=8.6Hz), 3.88 (1H, dt, J=7.2, 8.6Hz), 2.36 (3H, s), 1.36 (3H, d, J=7.2Hz), 1.31 (9H, s).

### Example 1(13)

### N-[4-[2-(2-Naphthyl)ethynyl]phenylsulfonyl]-D-tryptophan methyl ester

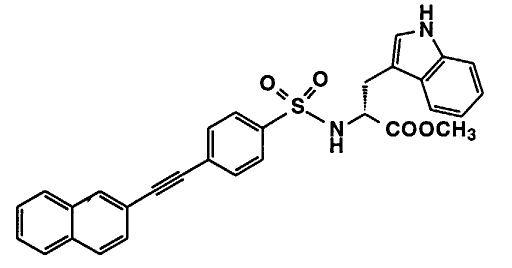

TLC : Rf 0.41 (chloroform : ethyl acetate = 9 : 1) ;
NMR (CDCl₃): δ 8.32-7.98 (2H, m), 7.95-7.75 (3H, m), 7.68 (1H, d, J=7.7Hz), 7.62-7.40 (6H, m), 7.38-6.90 (5H, m), 5.18 (1H, d, J=8.8Hz), 4.36-4.20 (1H, m), 3.49 (3H, s), 3.36-3.15 (2H, m).

### Example 1(14)

### N-[4-[2-(4-Isobutylphenyl)ethynyl]phenylsulfonyl]-D-tryptophan methyl ester

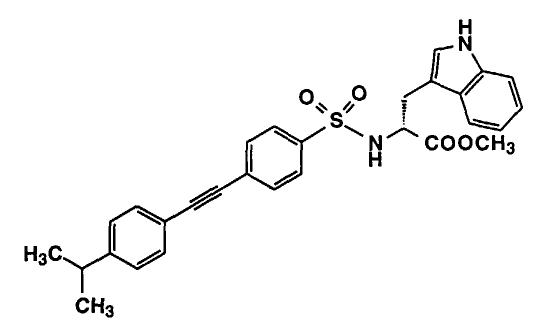

TLC : Rf 0.35 (chloroform : ethyl acetate = 9 : 1) ;
NMR (CDCl₃): δ 8.05 (1H, s), 7.65 (2H, d, J=8.0Hz), 7.55-7.38 (5H, m), 7.36-6.95 (6H, m), 5.17 (1H, d, J=9.0Hz), 4.35-4.20 (1H, m), 3.48 (3H, s), 3.38-3.10 (2H, m), 3.02-2.82 (1H, m), 1.26 (6H, d, J=6.7Hz).

### Example 1(15)

### N-[4-[2-(1,1'-Biphenyl-4-yl)ethynyl]phenylsulfonyl]-D-tryptophan methyl ester

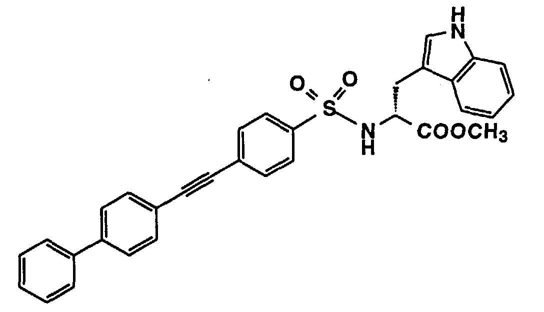

TLC : Rf 0.35 (chloroform : ethyl acetate = 9 : 1) ;
NMR (DMSO-d₆): δ 10.82 (1H, s), 8.60 (1H, d, J=8.4Hz), 7.78 (2H, d, J=8.6Hz), 7.68 (2H, d, J=8.6Hz), 7.63-7.25 (14H, m), 7.14-6.90 (3H, m), 4.12-3.95 (1H, m), 3.38 (3H, s), 3.09 (1H, dd, J=6.8, 14.2Hz), 2.92 (1H, dd, J=8.0, 14.2Hz).

### Example 1(16)

### N-[4-[2-(4-Benzyloxyphenyl)ethynyl]phenylsulfonyl]-D-tryptophan methyl ester

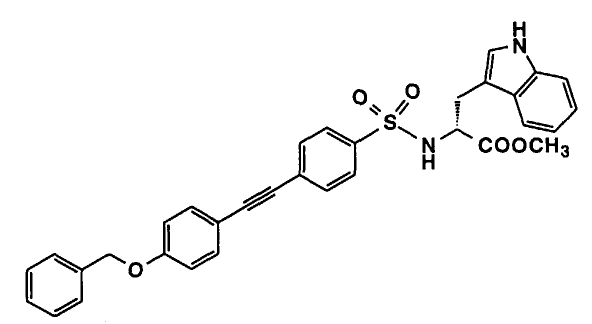

TLC : Rf 0.35 (chloroform : ethyl acetate = 9 : 1) ;
NMR (DMSO-d₆): δ 10.82 (1H, s), 8.58 (1H, d, J=6.4Hz), 7.62-7.22 (13H, m), 7.16-6.82 (5H, m), 5.16 (2H, s), 4.12-3.92 (1H, m), 3.36 (3H, s), 3.08 (1H, dd, J=6.4, 14.6Hz), 2.91 (1H, dd, J=7.4, 14.6Hz).

### Example 1(17)

### N-[4-[2-(4-Hydroxymethylphenyl)ethynyl]phenylsulfonyl]-D-alanine t-butyl ester

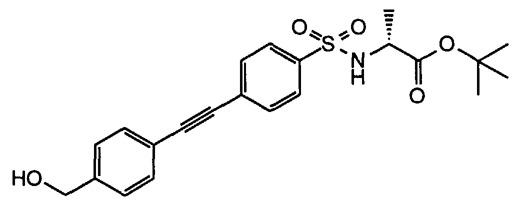

TLC : Rf 0.41 (chloroform : ethyl acetate = 9 : 1) ;
NMR (CDCl₃): δ 7.82 (2H, d, J=8.5Hz), 7.62 (2H, d, J=8.5Hz), 7.54 (2H, d, J=8.1Hz), 7.38 (2H, d, J=8.1Hz), 5.29 (1H, d, J=8.4Hz), 4.74 (2H, d, J=5.0Hz), 3.96-3.80 (1H, m), 1.90-1.74 (1H, m), 1.36 (3H, d, J=7.1Hz), 1.31 (9H, s).

### Example 1(18)

### N-[4-[2-(4-Imidazolylphenyl)ethynyl]phenylsulfonyl]-D-tryptophan methyl ester

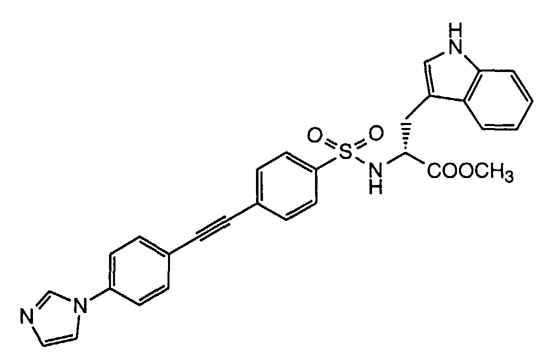

TLC : Rf 0.46 (chloroform : methanol = 95 : 5) ;
NMR (CDCl₃): δ 8.18 (1H, s), 7.90 (1H, s), 7.80-7.60 (4H, m), 7.58-7.00 (11H, m), 5.29 (1H, d, J=8.4Hz), 4.37-4.20 (1H, m), 3.49 (3H, s), 3.40-3.10 (2H, m).

### Example 1(19)

### N-[4-[2-(4-Methylphenyl)ethynyl]phenylsulfonyl]-L-tryptophan methyl ester

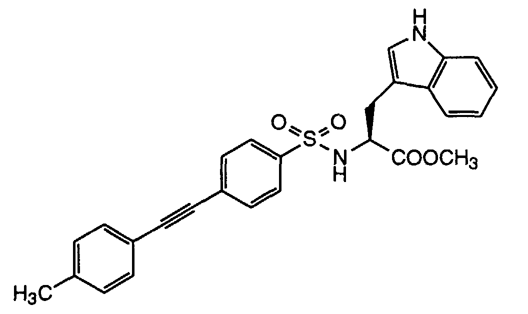

TLC : Rf 0.34 (chloroform : ethyl acetate = 9 : 1) ;
NMR (DMSO-d₆): δ 10.83 (1H, s), 8.60 (1H, d, J=8.8Hz), 7.59 (2H, d, J=8.8Hz), 7.53 (2H, d, J=8.2Hz), 7.49 (2H, d, J=8.2Hz), 7.36-7.22 (4H, m), 7.10-6.90 (3H, m), 4.08-3.94 (1H, m), 3.36 (3H, s), 3.07 (1H, dd, J=6.8, 14.4Hz), 2.90 (1H, dd, J=8.0Hz, 14.4Hz), 2.35 (3H, s).

### Example 1(20)

### N-[4-[2-(4-Methylphenyl)ethynyl]phenylsulfonyl]-3-t-butoxy-D-propionic acid t-butyl ester

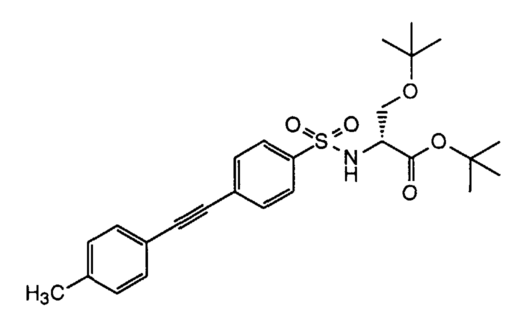

NMR (CDCl₃): δ 7.82 (2H, d, J = 8.2 Hz), 7.60 (2H, d, J = 8.2 Hz), 7.43 (2H, d, J=8.5 Hz), 7.17 (2H, d, J=8.5 Hz), 5.47 (1H, d, J = 9.5 Hz), 4.00 (1H, dt, J=9.5, 3.2 Hz), 3.69 (1H, dd, J=3.2, 8.0 Hz), 3.52 (1H, dd, J=3.2, 8.0 Hz), 2.38 (3H, s), 1.32 (9H, s), 1.10 (9H, s).

### Example 1(21)

### N-[4-[2-(4-Methylphenyl)ethynyl]phenylsulfonyl]-D-glutamic acid di-t-butyl ester

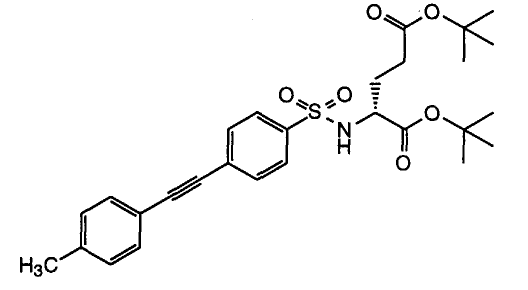

NMR (CDCl₃): δ 7.79 (2H, d, J = 8.2 Hz), 7.58 (2H, d, J = 8.2 Hz), 7.43 (2 H, d, J=8.5 Hz), 7.17 (2 H, d, J=8.5 Hz), 5.24 (1 H, d, J = 9.5 Hz), 3.83 (1 H, m), 3.08 (2 H, m), 2.5-2.3 (5 H, m), 2.04(1H, m), 1.77 (1 H, m), 1.45 (9H, s), 1.27 (9H, s).

### Example 1(22)

### N-[4-[2-(4-Methylphenyl)ethynyl]phenylsulfonyl]-t-butoxycarbonyl-D-lysine t-butyl ester

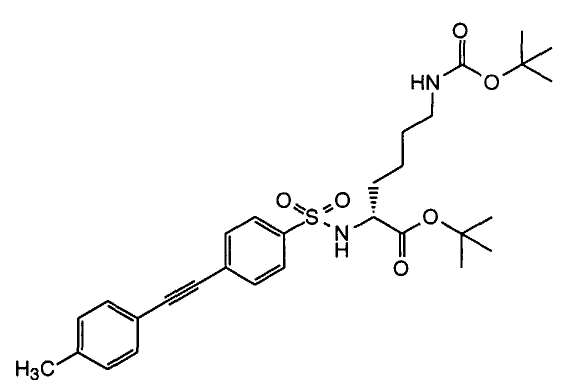

NMR (CDCl₃): δ 7.80 (2H, d, J = 8.2 Hz), 7.60 (2H, d, J = 8.2 Hz), 7.43 (2 H, d, J=8.5 Hz), 7.17 (2 H, d, J=8.5 Hz), 5.23 (1 H, d, J = 9.5 Hz), 4.56 (1 H, m), 3.77 (1 H, m), 3.08 (2 H, m), 2.38 (3 H, s), 1.9-1.3 (6H, m), 1.45 (9H, s), 1.26 (9H, s).

### Example 1(23)

### N-[4-(6-Imidazolyl-1-hexynyl)phenylsulfonyl]-D-alanine t-butyl ester

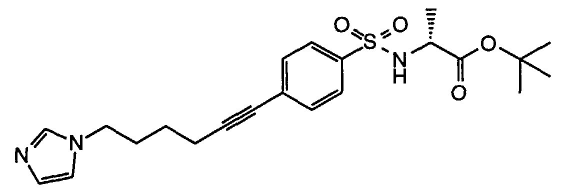

TLC : Rf 0.46 (chloroform : methanol = 9 : 1) ;
NMR (CDCl₃): δ 7.76 (2H, d, J=8.3Hz), 7.50 (1H, s), 7.46 (2H, d, J=8.3Hz), 7.08 (1H, s), 6.93 (1H, s), 5.36 (1H, d, J=8.5Hz), 4.01 (2H, t, J=6.9Hz), 3.95-3.78 (1H, m), 2.46 (2H, t, J=6.5Hz), 2.10-1.85 (2H, m), 1.70-1.50 (2H, m), 1.35 (3H, d, J=7.2Hz), 1.30 (9H, s).

### Example 1(24)

### N-[4-(6-Imidazolyl-1-hexynyl)phenylsulfonyl]-D-tryptophan methyl ester

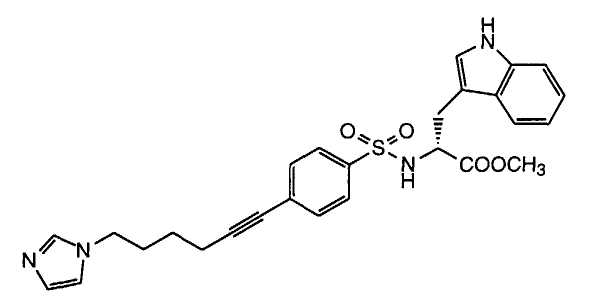

TLC : Rf 0.52 (chloroform : methanol = 85 : 15) ;
NMR (DMSO-d₆): δ 10.83 (1H, s), 8.55 (1H, s), 7.63 (1H, s), 7.52 (2H, d, J=8.4Hz), 7.38 (2H, d, J=8.4Hz), 7.33-7.23 (2H, m), 7.19-7.14 (1H, m), 7.10-6.82 (4H, m), 4.12-3.88 (3H, m), 3.06 (1H, dd, J=7.0, 14.0Hz), 2.93 (1H, dd, J=7.8, 14.0Hz), 2.60-2.40 (2H, m), 2.00-1.75 (2H, m), 1.60-1.38 (2H, m).

### Example 1(25)

### N-[4-[2-(4-Dimethylaminomethylphenyl)ethynyl]phenylsulfonyl]-D-tryptophan methyl ester

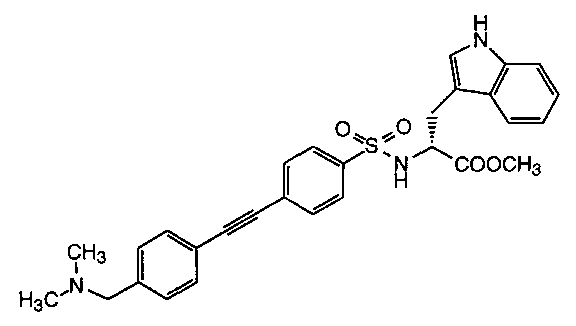

TLC : Rf 0.28 (chloroform : methanol = 10 : 1) ;
NMR (CDCl₃): δ 8.20-8.10 (1H, br.), 7.65 (2H, d, J=8.5Hz), 7.55-7.40 (5H, m), 7.32 (2H, d, J=8.5Hz), 7.35-6.95 (4H, m), 5.30-5.00 (1H, br.), 4.40-4.10 (1H, m), 3.48 (3H, s), 3.42 (2H, s), 3.24 (2H, d, J=6.0Hz), 2.25 (6H, s).

### Example 1(26)

### N-[4-[2-[4-(1-Imidazolylmethyl)phenyl]ethynyl]phenylsulfonyl]-D-tryptophan methyl ester

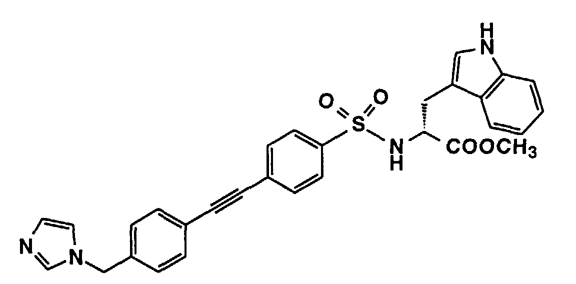

TLC : Rf 0.40 (chloroform : methanol = 10 : 1) ;
NMR (DMSO-d₆): δ 10.83 (1H, s), 8.60 (1H, d, J=7.5Hz), 7.77 (1H, m), 7.65-7.50 (6H, m), 7.30 (4H, d, J=8.0Hz), 7.21 (1H, t, J=1.0Hz), 7.10-6.90 (4H, m), 5.26 (2H, s), 4.10-3.90 (1H, m), 3.34 (3H, s), 3.08 (1H, dd, J=7.0, 14.0Hz), 2.81 (1H, dd, J=7.5, 14.0Hz).

### Example 1(27)

### N-[4-[5-(1-Imidazolyl)-1-pentynyl]phenylsulfonyl]-D-tryptophan methyl ester

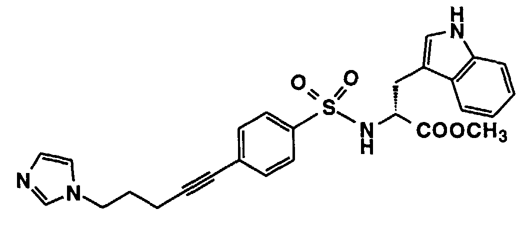

TLC : Rf 0.29 (chloroform : methanol = 9 : 1) ;
NMR (CDCl₃): δ 8.80-8.75 (1H, br.s), 7.62-7.52 (3H, m), 7.44 (1H, d, J=7.6Hz), 7.40-7.28 (3H, m), 7.22-7.02 (3H, m), 7.00-6.92 (2H, m), 5.39 (1H, d, J=7.0Hz), 4.34-4.20 (1H, m), 4.16 (2H, t, J=6.6Hz), 3.50 (3H, s), 3.32-3.14 (2H, m), 2.44 (2H, t, J=6.6Hz), 2.16-2.00 (2H, m).

### Example 1(28)

### N-[4-[2-[4-(2-Dimethylaminoethyl)phenyl]ethynyl]phenylsulfonyl]-D-tryptophan methyl ester

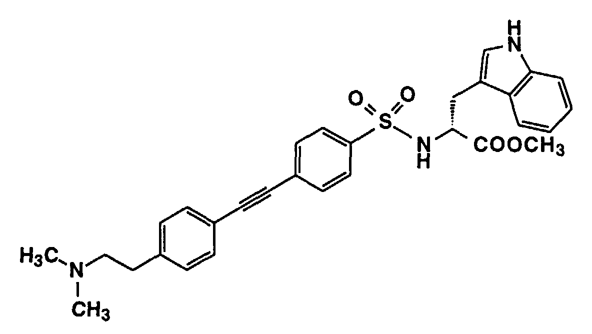

TLC : Rf 0.24 (chloroform : methanol : water = 100 : 10 : 1) ;
NMR (DMSO-d₆): δ 10.84 (1H, s), 8.61 (1H, d, J=8.5Hz), 7.65-6.90 (13H, m), 4.10-3.90 (1H, m), 3.36 (3H, s), 3.08 (1H, dd, J=7.0, 14.5Hz), 2.91 (1H, dd, J=7.5, 14.5Hz), 2.80-2.35 (4H, m), 2.19 (6H, s).

### Example 1(29)

### N-[4-[2-[4-[2-(1-Imidazolyl)ethyl]phenyl]ethynyl]phenylsulfonyl]-D-tryptophan methyl ester

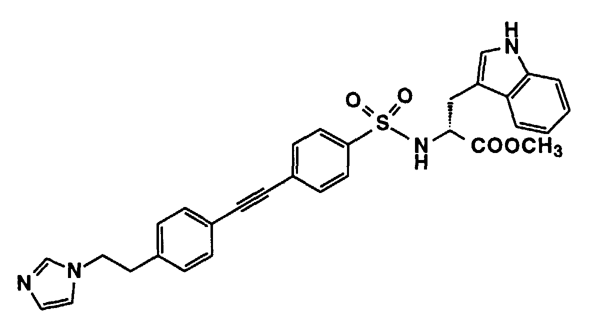

TLC : Rf 0.47 (chloroform : methanol : water = 100 : 10 : 1) ;
NMR (DMSO-d₆): δ 10.84 (1H, s), 8.61 (1H, d, J=8.5Hz), 7.65-7.45 (7H, m), 7.31 (2H, d, J=8.0Hz), 7.24 (2H, d, J=8.0Hz), 7.30-6.70 (5H, m), 4.23 (2H, t, J=7.0Hz), 4.10-3.90 (1H, s), 3.36 (3H, s), 3.20-3.00 (3H, m), 2.91 (1H, dd, J=8.0, 14.5Hz).

### Example 1(30)

### N-[4-[2-[4-(2-Methoxycarbonylethyl)phenyl]ethynyl]phenylsulfonyl]-D-tryptophan methyl ester

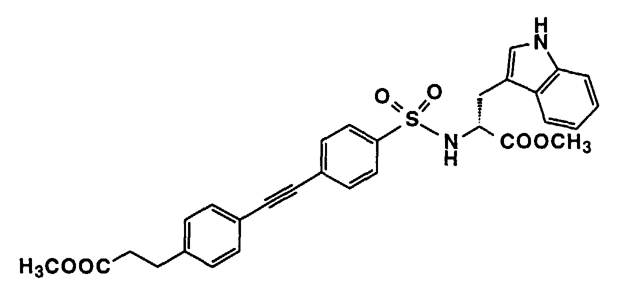

TLC : Rf 0.46 (hexane : ethyl acetate = 1 : 1) ;
NMR (CDCl₃): δ 8.08 (1H, s), 7.66 (2H, d, J=8.5Hz), 7.52-7.40 (5H, m), 7.34 (1H, d, J=8.0Hz), 7.21 (2H, d, J=8.5Hz), 7.2-7.0 (3H, m), 5.18 (1H, d, J=9.0Hz), 4.28 (1H, dt, J=9.0, 5.5Hz), 3.68 (3H, s), 3.48 (3H, s), 3.25 (2H, d, J=5.5Hz), 2.98 (2H, t, J=7.5Hz), 2.65 (2H, t, J=7.5Hz).

### Example 1(31)

### N-[4-[2-(4-Methylphenyl)ethynyl]phenylsulfonyl]-L-alanine t-butyl ester

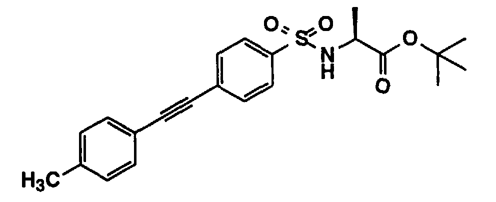

TLC : Rf 0.39 (hexane : ethyl acetate = 4 : 1) ;
NMR (CDCl₃): δ 7.82 (2H, d, J=8.8Hz), 7.61 (2H, d, J=8.8Hz), 7.44 (2H, d, J=8.0Hz), 7.18 (2H, d, J=8.0Hz), 5.25 (1H, d, J=8.4Hz), 3.96-3.78 (1H, m), 2.38 (3H, s), 1.36 (3H, d, J=7.4Hz), 1.31 (9H, s).

### Example 1(32)

### N-[4-[2-(4-Methylphenyl)ethynyl]phenylsulfonyl]-N'-methyl-N'-benzyl-D-glutamine t-butyl ester

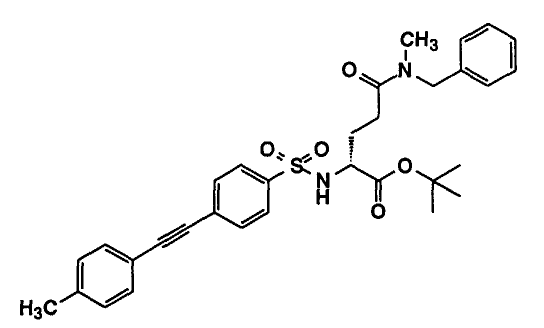

TLC : Rf 0.46 (hexane : ethyl acetate = 1 : 1) ;
NMR (CDCl₃): δ 7.80 and 7.79 (total 2H, d and d, J=8.8 and 8.8Hz), 7.59 and 7.58 (total 2H, d and d, J=8.8 and 8.8Hz), 7.44 (2H, d, J=8.0Hz), 7.40-7.22 (4H, m), 7.22-7.14 (3H, m), 5.49 and 5.44 (total 1H, d and d, J=9.2 and 9.8Hz), 4.62 and 4.53 (total 2H, s and s), 3.95-3.75 (1H, m), 2.97 and 2.93 (total 3H, s and s), 2.70-2.42 (2H, m), 2.38 (3H, s), 2.34-2.10 (1H, m), 1.96-1.74 (1H, m), 1.27 and 1.25 (total 9H, s and s).

### Example 1(33)

### N-[4-[2-(4-Methylphenyl)ethynyl]phenylsulfonyl]-D-alanine methyl ester

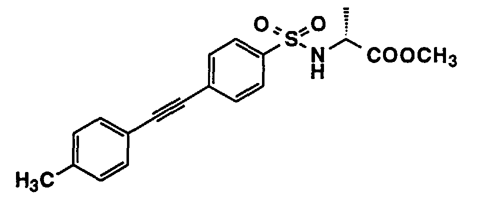

TLC : Rf 0.39 (hexane : ethyl acetate = 7 : 3) ;
NMR (CDCl₃): δ 7.81 (2H, d, J=8.8Hz), 7.62 (2H, d, J=8.8Hz), 7.44 (2H, d, J=8.0Hz), 7.18 (2H, d, J=8.0Hz), 5.27 (1H, d, J=8.6Hz), 4.10-3.93 (1H, m), 3.56 (3H, s), 2.38 (3H, s), 1.40 (3H, d, J=7.0Hz).

### Example 1(34)

### N-[4-(1-Propynyl)phenylsulfonyl]-D-alanine methyl ester

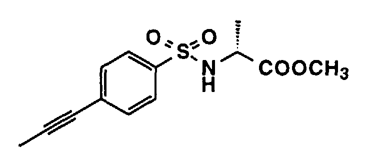

TLC : Rf 0.38 (hexane : ethyl acetate = 2 : 1) ;
NMR (CDCl₃): δ 7.75 (2H, d, J=8.4Hz), 7.48 (2H, d, J=8.4Hz), 5.21 (1H, br.d, J=8.4Hz), 3.99 (1H, dq, J=8.4, 7.2Hz), 3.55 (3H, s), 2.08 (3H, s), 1.39 (3H, d, J=7.2Hz).

### Reference Example 3

### N-[4-Trimethylsilylethynyl)phenylsulfonyl]-D-alanine t-butyl ester

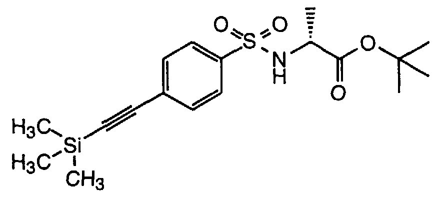

The same procedure as described in Example 1, using N-(4-bromophenylsulfonyl)-D-alanine t-butyl ester (prepared by the same procedure as Reference Example 1), and trimethylsilylacetylene gave the title compound having the following physical data.
TLC : Rf 0.20 (hexane : ethyl acetate = 10 : 1) ;
NMR (CDCl₃): δ 7.71 (2H, d, J=8.0Hz), 7.48 (2H, d, J=8.0Hz), 5.18 (1H, d, J=8.0Hz), 3.78 (1H, dq, J=8.0, 7.0Hz), 1.28 (3H, d, J=7.0Hz), 1.25 (9H, s), 0.26 (9H, s).

### Example 2

### N-[4-[2-(4-Methylphenyl)ethynyl]phenylsulfonyl]-D-phenylalanine

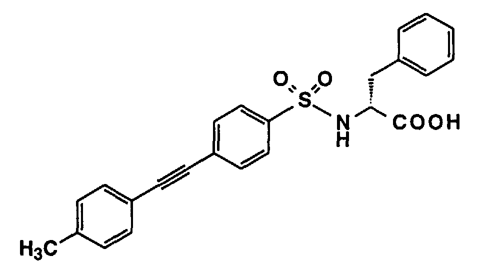

A solution of the compound prepared in Example 1 (90 mg) in trifluoroacetic acid (1 ml) was stirred at room temperature for 1 hour. The reaction mixture was concentrated to give the title compound (80 mg) having the following physical data.
TLC : Rf 0.37 (chloroform : methanol : acetic acid : water = 100 : 10 : 1 : 1) ;
NMR (CDCl₃): δ 7.63 (2H, d, J=8.8Hz), 7.55 (2H, d, J=8.8Hz), 7.43 (2H, d, J=8.0Hz), 7.30-7.00 (7H, m), 5.06 (1H, d, J=9.0Hz), 4.24 (1H, m), 3.13 (1H, dd, J=13.7Hz, 5.1Hz), 3.01 (1H, dd, J=13.7Hz, 6.6Hz), 2.39 (3H, s).

### Example 2(1) - 2(47)

By the same procedure as Example 2 (deprotection under acidic condition; for example, trifluoroacetic acid, a solution of hydrochloric acid in dioxane or in ethyl acetate, a solution of lithium hydroxide in dioxane, a solution of trimethylsilyl chloride and sodium iodide) or the procedure for the same purpose as Example 2 (e.g. alkali hydrolysis), using a compound prepared in Example 1(1)-1(25), Reference Example 3, Example 1(26)-1(32), 1(34), or a corresponding compound, following compounds were obtained.

### Example 2(1)

### N-[4-[2-(4-Methylphenyl)ethynyl]phenylsulfonyl]-D-alanine

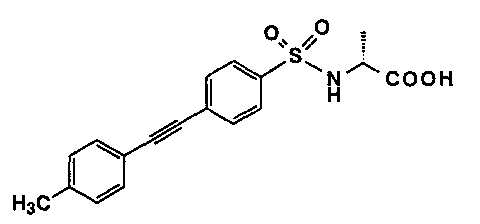

TLC : Rf 0.58 (chloroform : methanol: acetic acid = 9 : 1 : 0.1) ;
NMR (DMSO-d₆): δ 12.90-12.50 (1H, br.s), 8.26 (1H, d, J=8.0Hz), 7.80 (2H, d, J=8.4Hz), 7.69 (2H, d, J=8.4Hz), 7.48 (2H, d, J=8.0Hz), 7.26 (2H, d, J=8.0Hz), 3.92-3.68 (1H, m), 2.35 (3H, s), 1.57 (3H, d, J=7.0Hz).

### Example 2(2)

### N-[4-(1-Pentynyl)phenylsulfonyl]-D-alanine

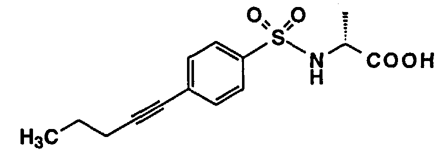

TLC : Rf 0.45 (chloroform : methanol = 2 : 1);
NMR (DMSO-d₆): δ 7.74 (2H, d, J=8.6Hz), 7.52 (2H, d, J=8.6Hz), 7.20-6.90 (1H, br.s), 3.20 (1H, m), 2.44 (2H, m), 1.57 (2H, m), 1.17 (3H, d, J=6.8Hz), 1.00 (3H, t, J=7.2Hz).

### Example 2(3)

### N-[4-(2-Phenylethynyl)phenylsulfonyl]glycine

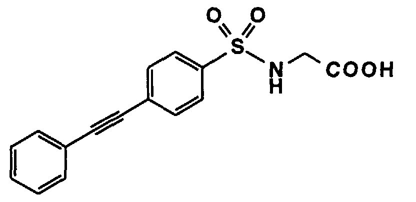

TLC : Rf 0.32 (chloroform : methanol: acetic acid = 9 : 1: 0.1) ;
NMR (DMSO-d₆): δ 12.90-12.50 (1H, br.s), 8.17 (1H, t, J=6.0Hz), 7.82 (2H, d, J=8. 6Hz), 7.72 (2H, d, J=8.6Hz), 7.66-7.52 (2H, m), 7.50-7.40 (3H, m), 3.62 (2H, d, J=6.0Hz).

### Example 2(4)

### N-[4-[2-(4-Methylphenyl)ethynyl]phenylsulfonyl]-D-tryptophan

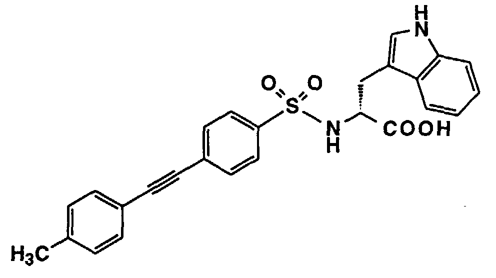

TLC: Rf 0.42 (chloroform : methanol : acetic acid = 9 : 1 : 0.1) ;
NMR (DMSO-d₆): δ 10.72 (1H, s), 7.65 (2H, d, J=8.4Hz), 7.52 (2H, d, J=8.4Hz), 7.47 (2H, d, J=8.8Hz), 7.32-7.20 (3H, m), 7.14-6.84 (3H, m), 3.74-3.58 (1H, m), 3.12 (1H, dd, J=5.4Hz, 14.8Hz), 3.03 (1H, dd, J=6.4Hz, 14.8Hz), 2.35 (3H, s).

### Example 2(5)

### N-[4-[2-(Pyridin-2-yl)ethynyl]phenylsulfonyl]glycine

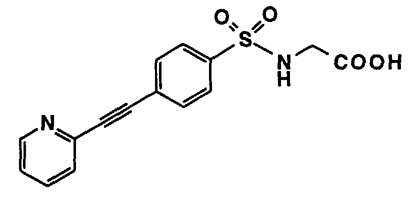

TLC : Rf 0.29 (chloroform : methanol : acetic acid : water = 50 : 10 : 1 : 1) ;
NMR (DMSO-d₆): δ 12.68 (1H, br.s), 8.64 (1H, m), 8.20 (1H, t, J=6.0Hz), 7.90-7.70 (6H, m), 7.45 (1H, m), 3.63 (2H, d, J=6.0Hz).

### Example 2(6)

### N-[4-[2-(4-Methoxyphenyl)ethynyl]phenylsulfonyl]glycine

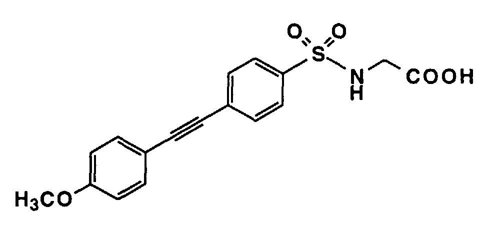

TLC : Rf 0.36 (chloroform : methanol : acetic acid : water = 50 : 10 : 1 : 1) ;
NMR (CD₃OD+CDCl₃): δ 7.86 (2H, d, J=8.6Hz), 7.63 (2H, d, J=8.6Hz), 7.49 (2H, d, J=8.8Hz), 6.94 (2H, d, J=8.8Hz), 3.85 (3H, s), 3.67 (2H, s).

### Example 2(7)

### N-[4-[2-(4-Methylphenyl)ethynyl]phenylsulfonyl]-D-valine

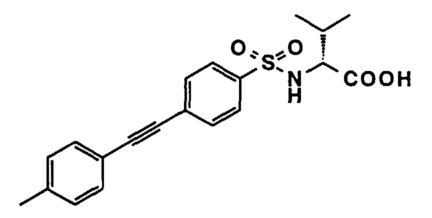

TLC : Rf 0.50 (ethyl acetate : acetic acid : water = 100 : 0.25) ;
NMR (CDCl₃): δ 7.80 (2H, d, J=9.0Hz), 7.59 (2H, d, J=9.0Hz), 7.43 (2H, d, J=9.0Hz), 7.17 (2H, d, J=9.0Hz), 5.18 (1H, br.d, J=10Hz), 3.71-3.86 (1H, m), 2.39 (3H, s), 2.00 - 2.21 (1H, m), 0.96 (3H, d, J=7.0Hz), 0.86 (3H, d, J=7.0Hz).

### Example 2(8)

### N-[4-[2-(2-Methylphenyl)ethynyl]phenylsulfonyl]-D-alanine

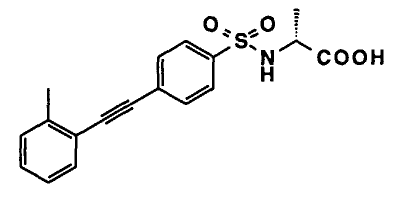

TLC : Rf 0.35 (chloroform : methanol : acetic acid : water = 100 : 10 : 1 : 1) ;
NMR (CDCl₃): δ 7.81 (2H, d, J=8.5Hz), 7.61 (2H, d, J=8.5Hz), 7.50 (1H, d, J=7.1Hz), 7.4-7.2 (3H, m), 5.33 (1H, d, J=8.1Hz), 4.05 (1H, m), 2.57 (3H, s), 1.44 (3H, d, J=7.1Hz).

### Example 2(9)

### N-[4-[4-(1-Heptenyl)]phenylsulfonyl]-D-alanine

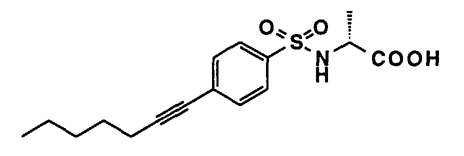

TLC : Rf 0.60 (chloroform : methanol = 3 : 1) ;
NMR (CDCl₃): δ 7.72-7.82 (m, 2H), 7.42-7.53 (m, 2H), 5.35 (d, 1H, J=8.6Hz), 4.03 (m, 1H), 2.42 (t, 2H, J=7.0Hz), 1.42 (d, 3H, J=7.0Hz), 1.20-1.72 (m, 6H), 0.92 (t, 3H, J=7.0Hz).

### Example 2(10)

### N-[4-[4-(1-Hexynyl)]phenylsulfonyl]-D-alanine

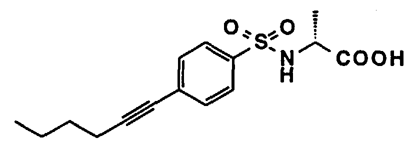

TLC : Rf 0.66 (chloroform : methanol = 3 : 1) ;
NMR (CD₃OD): δ 7.76 (d, 2H, J=8Hz), 7.58 (d, 2H, J=8Hz), 3.89 (q, 1H, J=7Hz), 2.43 (t, 2H, J=7Hz), 1.38-1.64 (m, 4H), 1.29 (d, 3H, J=7Hz), 0.95 (t, 3H, J=7Hz).

### Example 2(11)

### N-[4-(1-Octynyl)phenylsulfonyl]-D-alanine

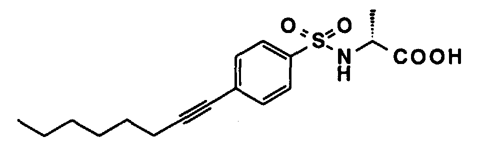

TLC : Rf 0.64 (chloroform : methanol = 3 : 1) ;
NMR (CDCl₃): δ 7.70-7.81 (m, 2H), 7.42-7.53 (m, 2H), 5.23-5.54 (m, 1H), 3.90-4.17 (m, 1H), 2.42 (t, 2H, J=7Hz), 1.42 (d, 3H, J=7Hz), 1.22-1.68 (m, 8H), 0.90 (t, 3H, J=7 Hz).

### Example 2(12)

### N-[4-[2-(3-Methylphenyl)ethynyl]phenylsulfonyl]-D-alanine

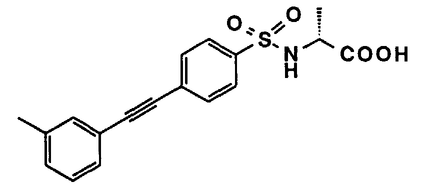

TLC : Rf 0.33 (chloroform : methanol : acetic acid : water = 100 : 10 : 1 : 1) ;
NMR (CDCl₃): δ 7.81 (2H, d, J=8.5Hz), 7.58 (2H, d, J=8.5Hz), 7.35-7.15 (4H, m), 5.75 (1H, br), 4.00 (1H, m), 2.34 (3H, s), 1.37 (3H, d, J=7.1Hz).

### Example 2(13)

### N-[4-[2-(2-Naphthyl)ethynyl]phenylsulfonyl]-D-alanine

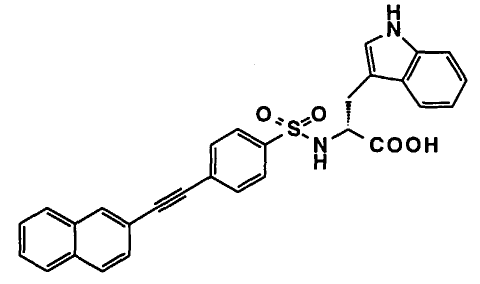

TLC : Rf 0.35 (chloroform : methanol : acetic acid = 90 : 10 : 1) ;
NMR (DMSO-d₆): δ 12.90-12.40 (1H, br), 10.80 (1H, s), 8.37 (1H, d, J=7.6Hz), 8,24 (1H, s), 8.05-7.90 (3H, m), 7.68-7.50 (7H, m), 7.40-7.28 (2H, m), 7.12-6.88 (3H, m), 4.02-3.86 (1H, m), 3.10 (1H, dd, J=14.4, 5.8Hz), 2.88 (1H, dd, J=14.4, 8.2Hz).

### Example 2(14)

### N-[4-[2-(4-Isobutylphenyl)ethynyl]phenylsulfonyl]-D-tryptophan

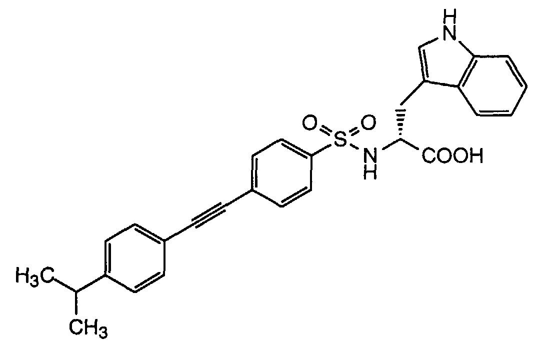

TLC : Rf 0.41 (chloroform : methanol : acetic acid = 90 : 10 : 1) ;
NMR (DMSO-d₆): δ 13.30-12.00 (1H, br), 10.80 (1H, s), 8.34 (1H, d, J=8.6Hz), 7.56 (2H, d, J=8.6Hz), 7.47 (2H, d, J=8.6Hz), 7.50-7.40 (2H, m), 7.40-7.28 (4H, m), 7.10-6.88 (3H, m), 4.02-3.86 (1H, m), 3.08 (1H, dd, J=15.0, 5.6Hz), 2.99-2.80 (2H, m), 1.22 (6H, d, J=6.8Hz).

### Example 2(15)

### N-[4-[2-(1,1'-Biphenyl-4-yl)ethynyl]phenylsulfonyl]-D-tryptophan

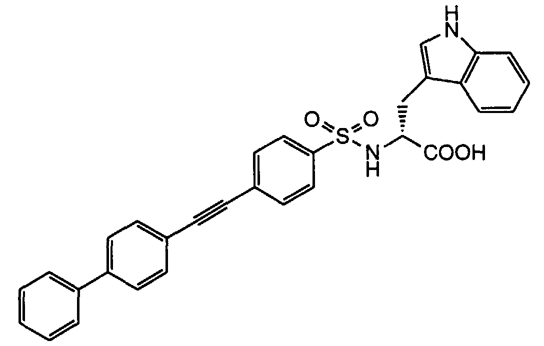

TLC : Rf 0.37 (chloroform : methanol : acetic acid = 90 : 10 : 1) ;
NMR (DMSO-d₆): δ 13.50-12.00 (1H, br), 10.80 (1H, s), 8.34 (1H, d, J=8.6Hz), 7.82-7.28 (15H, m), 7.14-6.90 (3H, m), 4.04-3.88 (1H, m), 3.10 (1H, dd, J=14.8, 6.0Hz), 2.89 (1H, dd, J=14.8, 8.4Hz).

### Example 2(16)

### N-[4-[2-(4-Benzyloxyphenyl)ethynyl]phenylsulfonyl]-D-tryptophan

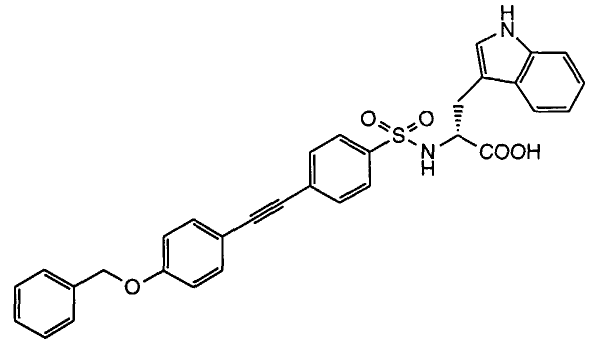

TLC : Rf 0.37 (chloroform : methanol : acetic acid = 90 : 10 : 1) ;
NMR (DMSO-d₆): δ 13.50-12.00 (1H, br), 10.80 (1H, s), 8.34 (1H, d, J=8.6Hz), 7.64-7.22 (13H, m), 7.18-6.84 (5H, m), 5.16 (2H, s), 4.02-3.88 (1H, m), 3.09 (1H, dd, J=14.4, 6.6Hz), 2.88 (1H, dd, J=14.4, 8.0Hz).

### Example 2(17)

### N-[4-[2-(4-Hydroxymethylphenyl)ethynyl]phenylsulfonyl]-D-alanine

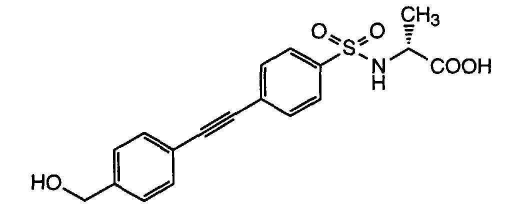

TLC : Rf 0.17 (chloroform : methanol : acetic acid = 90 : 10 : 1) ;
NMR (DMSO-d₆): δ 13.20-12.00 (1H, br), 8.27 (1H, d, J=8.4Hz), 7.81 (2H, d, J=8.6Hz), 7.71 (2H, d, J=8.6Hz), 7.55 (2H, d, J=8.4Hz), 7.39 (2H, d, J=8.4Hz), 4.54 (2H, s), 3.90-3.74 (1H, m), 1.18 (3H, d, J=7.4Hz).

### Example 2(18)

### N-[4-[2-(4-Imidazolylphenyl)ethynyl]phenylsulfonyl]-D-tryptophan

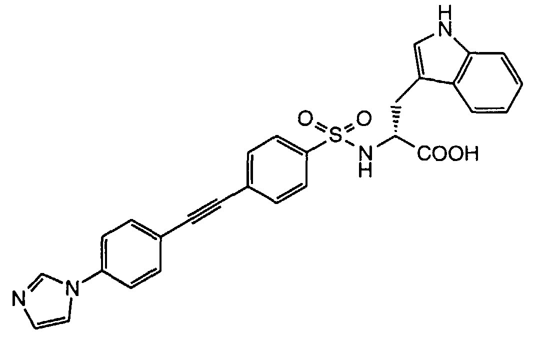

TLC : Rf 0.23 (chloroform : methanol : acetic acid = 90 : 10 : 1) ;
NMR (DMSO-d₆): δ 13.50-11.50 (1H, br), 10.80 (1H, s), 8.60-8.20 (1H, br), 8.00-7.65 (6H, m), 7.59 (2H, d, J=8.6Hz), 7.52 (2H, d, J=8.6Hz), 7.40-7.24 (2H, m), 7.24-6.85 (4H, m), 4.02-3.82 (1H, m), 3.09 (1 H, dd, J=14.0, 5.2Hz), 2.89 (1H, dd, J=14.0, 8.0Hz).

### Example 2(19)

### N-[4-[2-(4-Methylphenyl)ethynyl]phenylsulfonyl]-L-tryptophan

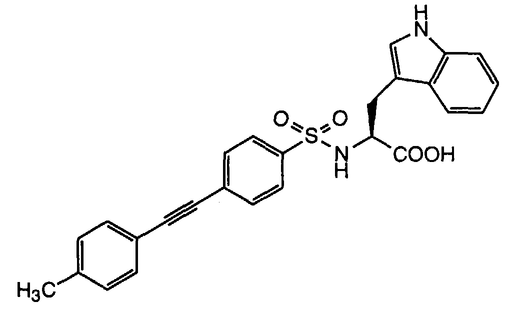

TLC: Rf 0.42 (chloroform : methanol : acetic acid = 90 : 10 : 1) ;
NMR (DMSO-d₆): δ 13.20-12.20 (1H, br), 10.80 (1H, s), 8.35 (1H, d, J=8.6Hz), 7.56 (2H, d, J=8.6Hz), 7.52-7.43 (4H, m), 7.39-7.22 (4H, m), 7.10-6.90 (3H, m), 4.02-3.82 (1H, m), 3.08 (1H, dd, J=14.4, 5.8Hz), 2.88 (1 H, dd, J=14.4, 8.2Hz), 2.37 (3H, s).

### Example 2(20)

### N-[4-[2-(4-Methylphenyl)ethynyl]phenylsulfonyl]-D-serine

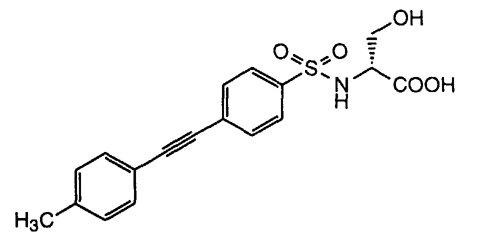

TLC : Rf 0.69 (ethyl acetate : acetic acid : water = 8 : 1 : 1) ;
NMR (CD₃OD+DMSO-d₆): δ 7.86 (2H, d, J=9.0Hz), 7.64 (2H, d, J=9.0Hz), 7.43 (2H, d, J=9.0), 7.23 (2H, d, J=9.0Hz), 3.52 - 3.82 (3H. m), 2.39 (3H, s).

### Example 2(21)

### N-[4-[2-(4-Methylphenyl)ethynyl]phenylsulfonyl]-D-glutamic acid

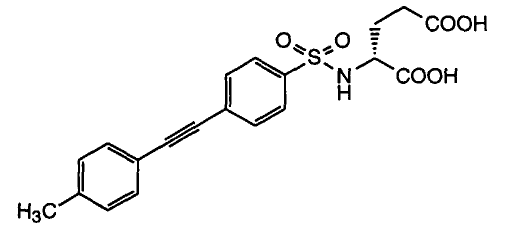

TLC : Rf 0.62 (ethyl acetate : acetic acid : water = 20 : 1) ;
NMR (CD₃OD): δ 7.82 (2H, d, J=9.0Hz), 7.61 (2H, d, J=9.0Hz), 7.42 (2H, d, J=9.0Hz), 7.23 (2H, d, J=9.0Hz), 3.84 - 4.00 (1H, m), 2.36 (2H, s), 2.40 (2H, t, J=6.0Hz), 1.45 - 2.18 (1H, m), 1.71 - 1.94 (1H, m).

### Example 2(22)

### N-[4-[2-(4-Methylphenyl)ethynyl]phenylsulfonyl]-D-lysine

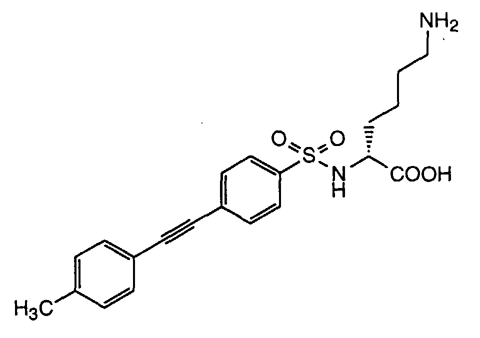

TLC : Rf 0.27 (ethyl acetate : acetic acid : water = 8 : 1 : 1) ;
NMR (CD₃OD): δ 7.84 (2H, d, J=9.0Hz), 7.63 (2H, d, J=9.0Hz), 7.43 (2H, d, J=9.0Hz), 7.21 (2H, d, J=9.0Hz), 3.78 - 3.94 (1H, m), 2.89 (2H, t, J=8.0Hz), 1.39 - 1.94 (6H, m).

### Example 2(23)

### N-[4-(6-Imidazolyl-1-hexynyl)phenylsulfonyl]-D-alanine

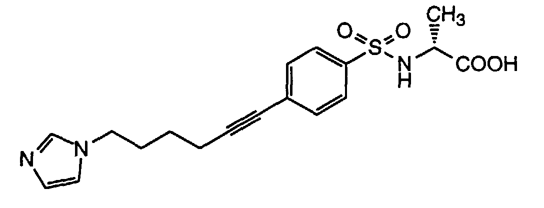

TLC : Rf 0.27 (chloroform : methanol : acetic acid = 80 : 20 : 1) ;
NMR (DMSO-d₆): δ 9.02 (1H, s), 8.21 (1H, d, J=8.6Hz), 7.74 (2H, d, J=8.6Hz), 7.73-7.68 (1H), 7.60-7.56 (1H), 7.53 (2H, d, J=8.6Hz), 4.24 (2H, t, 7.0Hz), 3. 84-3.68 (1H, m), 2.60-2.44 (2H), 2.02-1.86 (2H, m), 1.62- 1 .42 (2H, m), 1.15 (3H, d, J=7.2Hz).

### Example 2(24)

### N-[4-(6-Imidazolyl-1-hexynyl)phenylsulfonyl]-D-tryptophan

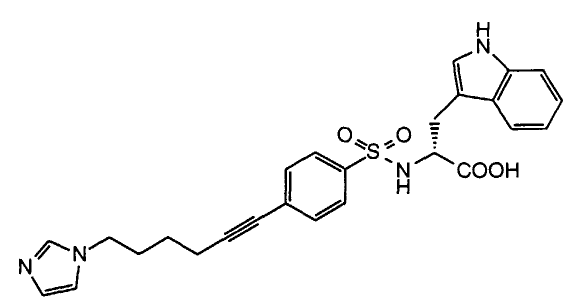

TLC : Rf 0.50 (chloroform : methanol : acetic acid = 80 : 20 : 1) ;
NMR (DMSO-d₆): δ 10.80 (1H, s), 8.50-8.00 (1H, br), 7.67 (1H, s), 7.50 (2H, d, J=8.2Hz), 7.44-7.16 (4H, m), 7.12-6.80 (5H, m), 4.03 (2H, t, J=6.8Hz), 4.00-3.80 (1H, m), 3.07 (1H, dd, J=14.6, 6.0Hz), 2.86 (1H, dd, J=14.6, 7.6Hz), 2.60-2.30 (2H, m), 2.08-1.75 (2H, m), 1.70-1.40 (2H, m).

### Example 2(25)

### N-[4-[2-(4-Dimethylaminomethylphenyl)ethynyl]phenylsulfonyl]-D-tryptophan

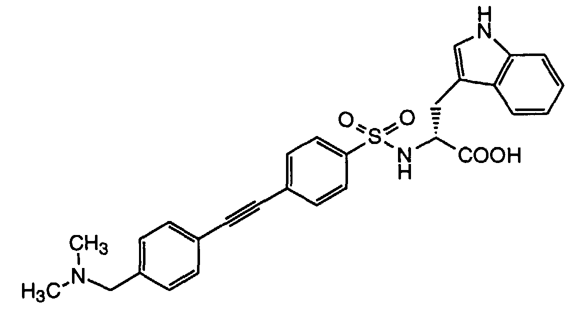

TLC : Rf 0.17 (chloroform : methanol : acetic acid = 40 : 10 : 1) ;
NMR (DMSO-d₆): δ 10.8 (1H, s), 8.4-8.0 (1H, br.), 7.65-7.45 (6H, m), 7.37 (2H, d, J=8.5Hz), 7.31 (2H, d, J=8.0Hz), 7.15-6.85 (3H, m), 4.0-3.8 (2H, m), 3.51 (2H, s), 3.09 (1H, dd, J=6.0, 14.5Hz), 2.90 (1H, dd, J=8.0, 14.5Hz), 2.21 (6H, s).

### Example 2(26)

### N-[(4-Ethynyl)phenylsulfonyl]-D-alanine

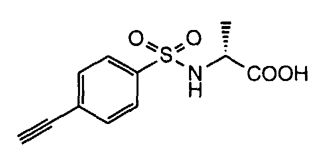

TLC : Rf 0.20 (chloroform : methanol = 3 : 1) ;
NMR (CDCl₃+CD₃OD (3 drops)): δ 7.68-7.54 (2H, m), 7.55-7.62 (2H, m), 5.24 (1H, d, J=9.0Hz), 3.85 (1H, qd, J=7.2, 9.0Hz), 3.23 (1H, s), 1.36 (3H, d, J=7.2Hz).

### Example 2(27)

### N-[4-[2-(4-Imidazolylphenyl)ethynyl]phenylsulfonyl]-N'-methyl-N'-(2-phenylethyl)-D-glutamine

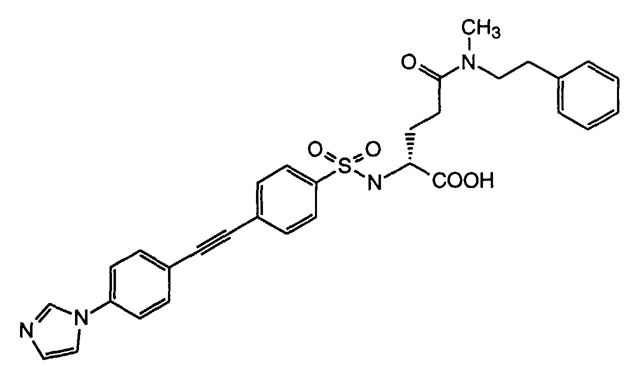

TLC : Rf 0.31 (chloroform : methanol : acetic acid = 90 : 10 : 1) ;
NMR (DMSO-d₆+CD₃OD (5 drops)): δ 8.36 (1H, s), 7.90-7.66 (9H, m), 7.40-7.18 (5H, m), 7,14 (1H, s), 3.56-3.32 (2H, m), 3.30-3.18 (1H,m), 2.88-2.60 (5H, m), 2.40-2.00 (2H, m), 1.92-1.68 (2H, m).

### Example 2(28)

### N-[4-[2-[4-(1-Imidazolylmethyl)phenylethynyl]phenylsulfonyl]-D-tryptophan

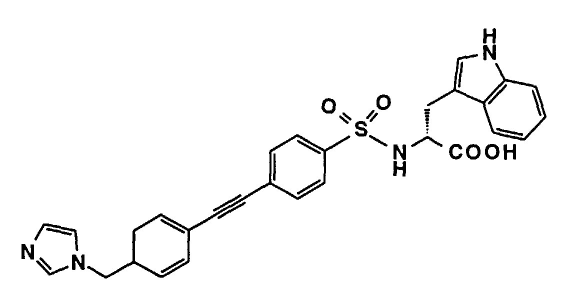

TLC : Rf 0.11 (chloroform : methanol : water = 40 : 10 : 1) ;
NMR (DMSO-d₆): δ 10.80 (1H, s), 8.37 (1H, d, J=9.0Hz), 7.81 (1H, s), 7.59 (2H, d, J=8.0Hz), 7.56 (2H, d, J=8.0Hz), 7.48 (2H, d, J=8.0Hz), 7.40-7.25 (4H, m), 7.22 (1H, s), 7.15-6.85 (4H, m), 5.27 (2H, s), 4.05-3.95 (1H, m), 3.08 (1H, dd, J=6.0, 14.5Hz), 2.87 (1H, dd, J=8.0, 14.5Hz).

### Example 2(29)

### N-[4-[5-(1-Imidazolyl)-1-pentynyl]phenylsulfonyl]-D-tryptophan

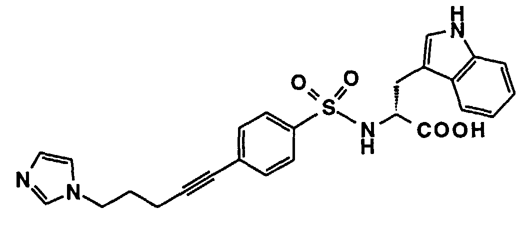

TLC : Rf 0.39 (chloroform : methanol : acetic acid = 80 : 20 : 1) ;
NMR (DMSO-d₆): δ 10.74 (1H, s), 7.66 (1H, s), 7.59 (2H, d, J=8.2Hz), 7.50-7.34 (3H, m), 7.28 (1H, d, J=7.8Hz), 7.20 (1H, s), 7.10-6.86 (4H, m), 4.11 (2H, t, J=6.8Hz), 3.70-3.58 (1H, m), 3.15-2.90 (2H, m), 2.41 (2H , t, J=7.0Hz), 2.08-1.90 (2H, m).

### Example 2(30)

### N-[4-[2-[4-(2-Dimethylaminoethyl)phenyl]ethynyl]phenylsulfonyl]-D-tryptophan hydrochloride

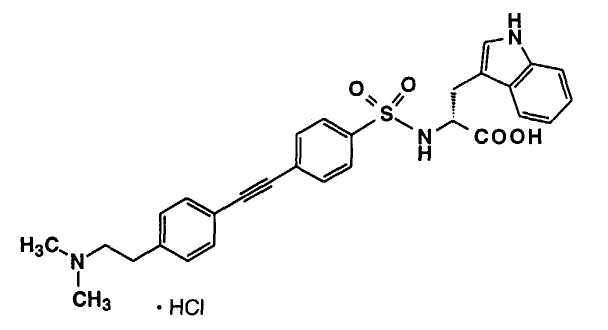

TLC : Rf 0.10 (chloroform : methanol : water = 40 : 10 : 1) ;
NMR (DMSO-d₆): δ 10.85 (1H, s), 8.40 (1H, d, J=8.5Hz), 7.64-7.44 (6H, m), 7.44-7.27 (4H, m), 7.12-6.90 (3H, m), 4.05-3.85 (1H, m), 3.90-3.25 (3H, m), 3.15-3.00 (2H, m), 2.87 (1H, dd, J=8.5, 15.0Hz), 2.81 (6H, s).

### Example 2(31)

### N-[4-[2-[4-[2-(1-Imidazolyl)ethyl]phenyl]ethynyl]phenylsulfonyl]-D-tryptophan

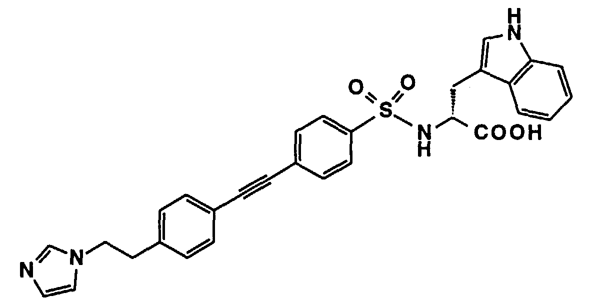

TLC : Rf 0.16 (chloroform : methanol : water = 40 : 10 : 1) ;
NMR (DMSO-d₆): δ 10.80 (1H, s), 8.5-8.1 (1H, br.), 7.65-7.42 (7H, m), 7.40-6.70 (9H, m), 4.24 (2H, t, J=7.0Hz), 4.00-3.95 (1H, m), 3.20-3.00 (3H, m), 2.88 (1H, dd, J=8.0, 14.0Hz).

### Example 2(32)

### N-[4-[2-[4-(2-Carboxyethyl)phenyl]ethynyl]phenylsulfonyl]-D-tryptophan

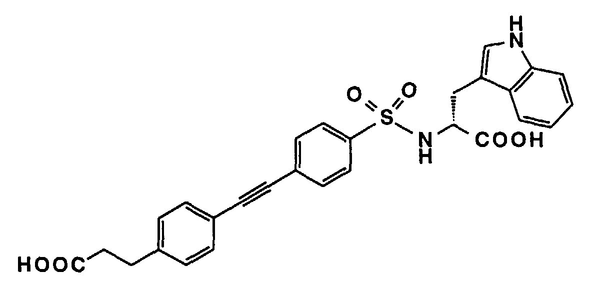

TLC : Rf 0.12 (chloroform : methanol : water = 40 : 10 : 1) ;
NMR (DMSO-d₆): δ 10.68 (1H, s), 7.69 (2H, d, J=8.5Hz), 7.62-7.42 (5H, m), 7.35-7.22 (3H, m), 7.10-6.85 (3H, m), 3.52 (1H, t, J=4.0Hz), 3.05 (2H, d, J=4.0Hz), 2.85 (2H, t, J=7.5Hz), 2.51 (2H, t, J=7.5Hz).

### Example 2(33)

### N-[4-[2-(4-Methylphenyl)ethynyl]phenylsulfonyl]-L-alanine

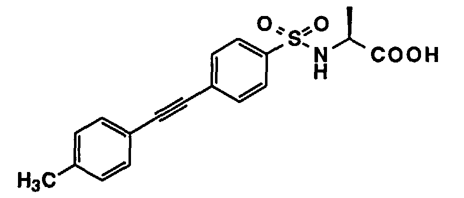

TLC : Rf 0.58 (chloroform : methanol : acetic acid = 90 : 10 : 1) ;
NMR (DMSO-d₆): δ 12.03 (1H, br.s), 8.26 (1H, d, J=8.8Hz), 7.81 (2H, d, J=8.4Hz), 7.69 (2H, d, J=8.4Hz), 7.48 (2H, d, J=8.2Hz), 7.26 (2H, d, J=8.2Hz), 3.90-3.70 (1H, m), 2.35 (3H, s), 1.18 (3H, d, J=7.4Hz).

### Example 2(34)

### N-[4-[2-(4-Methylphenyl)ethynyl]phenylsulfonyl]-N'-methyl-N'-benzyl-D-glutamine

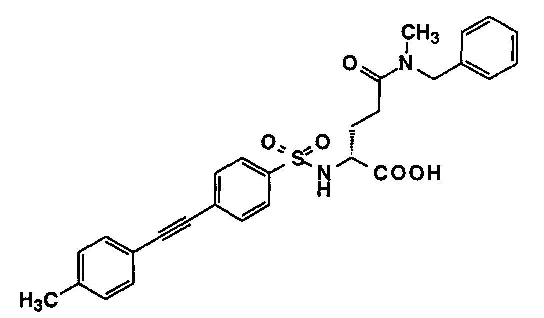

TLC : Rf 0.45 (chloroform : methanol : acetic acid = 90 : 10 : 1) ;
NMR (CDCl₃+CD₃OD): δ 7.80 (2H, d, J=8.4Hz), 7.59 (2H, d, J=8.4Hz), 7.43 (2H, d, J=8.0Hz), 7.40-7.24 (5H, m), 7.19 (2H, d, J=8.0Hz), 4.61 and 4.54 (total 2H, s and s), 3.92 (1H, dd, J=9.2 and 4.0Hz), 2.95 (3H, s), 2.85-2.40 (2H, m), 2.39 (3H, s), 2.38-2.10 (1H, m), 2.00-1.80 (1H, m).

### Example 2(35)

### N-[4-(1-Propynyl)phenylsulfonyl]-D-alanine

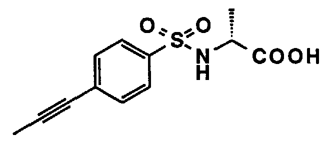

TLC : Rf 0.34 (chloroform : methanol : acetic acid = 100 : 10 : 1) ;
NMR (DMSO-d₆): δ 8.20 (1H, br.d, J=8.4Hz), 7.72 (2H, d, J=8.4Hz), 7.53 (2H, d, J=8.4Hz), 3.84-3.68 (1H, m), 2.07 (3H, s), 1.14 (3H, d, J=7.2Hz).

### Example 2(36)

### N-[4-[2-(4-Methylphenyl)ethynyl]phenylsulfonyl]-N'-methyl-N'-(2-phenylethyl)-D-glutamine

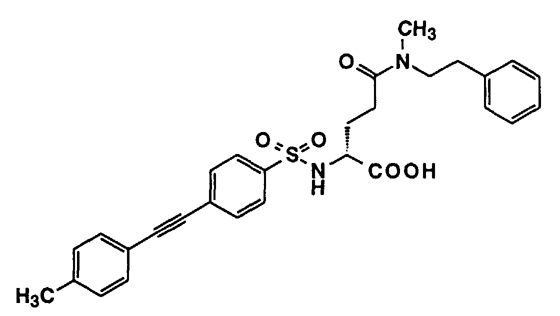

TLC : Rf 0.34 (chloroform : methanol = 4 : 1) ;
NMR (CDCl₃+CD₃OD): δ 7.80 (2H, d, J=8.0 Hz), 7.60 (2H, d, J=8.0 Hz), 7.40 (2H, d, J=7.0 Hz), 7.55-7.10 (7H, m), 3.40 and 3.80 (1H, m), 3.65-3.50 (2H, m), 2.95 (3H, s), 2.90 (3H, s), 2.85 (2H, m), 2.75-2.10 (2H, m), 2.10-1.60 (2H, m).

### Example 2(37)

### N-[4-(1-Heptynyl)phenylsulfonyl]-L-alanine

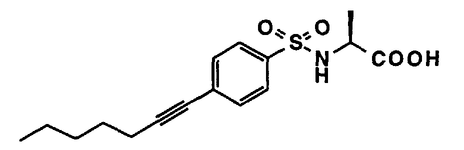

TLC : Rf 0.40 (chloroform : methanol : acetic acid = 90 : 10 : 1) ;
NMR (DMSO-d₆): δ 8.22 (1H, d, J=8.4Hz), 7.73 (2H, d, J=8.4Hz), 7.54 (2H, d, J=8.4Hz), 3.77 (1H, m), 2.55-2.40 (2H, m, overlap with DMSO), 1.65-1.48 (2H, m), 1.48-1.22 (4H, m), 1.15 (3H, d, J=7.2Hz), 0.89 (3H, t, J=7.2Hz).

### Example 2(38)

### N-[4-(1-Pentynyl)phenylsulfonyl]-L-alanine

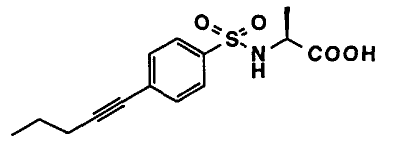

TLC : Rf 0.39 (chloroform : methanol : acetic acid = 90 : 10 : 1) ;
NMR (DMSO-d₆): δ 8.21 (1H, d, J=8.4Hz), 7.74 (2H, d, J=8.4Hz), 7.55 (2H, d, J=8.4Hz), 3.77 (1H, m), 2.44 (2H, t, J=7.0Hz), 1.68-1.48 (2H, m), 1.16 (3H, d, J=7.2Hz), 1.01 (3H, t, J=7.4Hz).

### Example 2(39)

### N-(4-Ethynyl)phenylsulfonyl-N'-methyl-N'-benzyl-D-glutamine

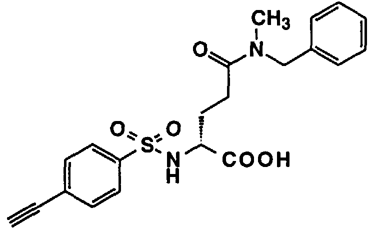

TLC : Rf 0.43 (chloroform : methanol = 9 : 1) ;
NMR (DMSO-d₆): δ 7.76-7.55 (4H, m), 7.36-7.12 (6H, m), 4.44-4.33 (3H, m), 3.83 (1H, dd, J=4.4, 4.6 Hz), 2.72 (3H, s), 2.33-2.20 (2H, m), 2.00-1.82 (1H, m), 1.76-1.58 (1H, m).

### Example 2(40)

### N-(4-Ethynyl)phenylsulfonyl-N'-(3-phenylpropyl)-D-glutamine

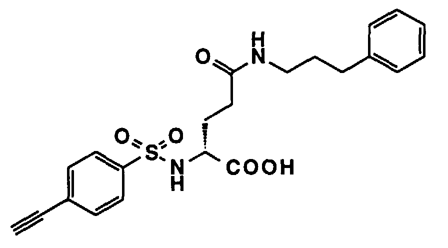

TLC : Rf 0.38 (chloroform : methanol = 9 : 1) ;
NMR (DMSO-d₆): δ 8.28 (1H, d, J=9.0 Hz), 7.74-7.58 (4H, m), 7.29-7.12 (6H, m), 4.44 (1H, m), 2.98 (2H, m), 2.53 (2H, t, J=7.6 Hz), 2.08 (2H, t, J=7.6 Hz), 1.94-1.76 (1H, m), 1.72-1.55 (3H, m).

### Example 2(41)

### N-(4-Ethynyl)phenylsulfonyl-N'-2-phenylethyl)-D-glutamine

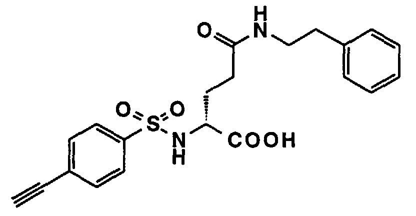

TLC : Rf 0.29 (chloroform : methanol : acetic acid = 90 : 10 : 1) ;
NMR (DMSO-d₆): δ 8.28 (1H, d, J=9.2Hz), 7.88 (1H, t, J=7.0Hz), 7.75 (2H, d, J=8.8Hz), 7.64 (2H, d, J=8.8Hz), 7.35-7.12 (5H, m), 4.44 (1H, s), 3.74 (1H, td, J=9.2 and 5.5Hz), 3.30-3.14 (2H, m), 2.67 (2H, t, J=7.3Hz), 2.08 (2H, t, J=7.5Hz), 1.98-1.76 (1H, m), 1.76-1.54 (1H, m).

### Example 2(42)

### N-(4-Ethynyl)phenylsulfonyl-N'-benzyl-D-glutamine

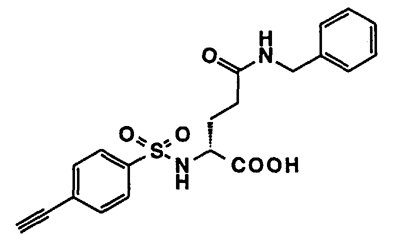

TLC : Rf 0.32 (chloroform : methanol : acetic acid = 100 : 10 : 5) ;
NMR (DMSO-d₆): δ 8.32 (1H, t, J=6.0Hz), 8.21-7.76 (1H, br.), 7.75 (2H, d, J=8.4Hz), 7.62 (2H, d, J=8.4Hz), 7.34-7.17 (5H, m), 4.46 (1H, s), 4.21 (2H, d, J=6.0Hz), 3.63 (1H, br.dd, J=5.4, 8.0Hz), 2.16 (2H ,t, J=7.8 Hz), 1.97-1.65 (2H, m).

### Example 2(43)

### N-(4-Ethynyl)phenylsulfonyl-N'-(1,2-diphenylethyl)-D-glutamine

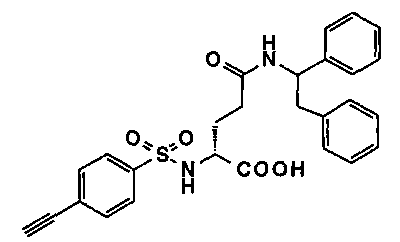

TLC : Rf 0.55 (chloroform : methanol acetic acid = 100 : 10 : 5) ;
NMR (DMSO-d₆): δ 8.38 (1H, d, J=8.4Hz), 8.23-7.95 (1H, br.), 7.72 (2H, d, J=8.8Hz), 7.60 (2H, d, J=8.8Hz), 7.30-7.16 (10H, m), 4.98 (1H, q, J=8.4Hz), 4.46 (1H, s), 3.68-3.55 (1H, m), 2.91 (2H, d, J=7.6Hz), 2.10-2.03 (2H ,m), 1.85-1.47 (2H, m).

### Example 2(44)

### N-(4-Ethynyl)phenylsulfonyl-N'-diphenylmethyl-D-glutamine

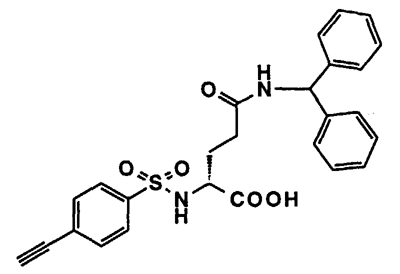

TLC : Rf 0.43 (chloroform : methanol : acetic acid = 100 : 10 : 5) ;
NMR (CDCl₃): δ 7.77 (2H, d, J=8.4Hz), 7.56 (2H, d, J=8.4Hz), 7.31-7.22 (10H, m), 6.16 (1H, s), 3.88 (1H, dd, J=5.2, 8.8Hz), 3.75 (1H, s), 2.48-2.39 (2H, m), 2.19-1.74 (2H, m).

### Example 2(45)

### N-(4-Ethynyl)phenylsulfonyl-N'-phenyl-D-glutamine

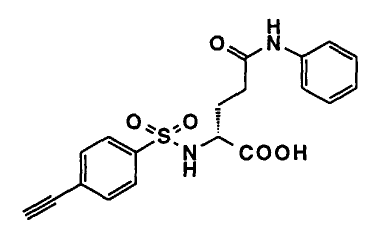

TLC : Rf 0.26 (chloroform : methanol : acetic acid = 100 : 10 : 5) ;
NMR (CD₃OD): δ 7.81 (2H, d, J=8.4Hz), 7.54 (2H, d, J=8.4Hz), 7.52 (2H, d, J=7.4Hz), 7.28 (2H, t, J=7.4Hz), 7.07 (1H, t, J=7.4Hz), 3.73 (1H, s), 3.67 (1H, dd, J=5.2, 7.4Hz), 2.51-2.43 (2H, m), 2.17-1.85 (2H, m).

### Example 2(46)

### N-[4-[2-(4-Methylphenyl)ethynyl]phenylsulfonyl]-L-proline

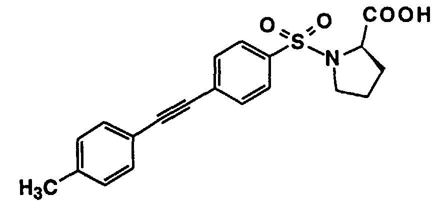

TLC : Rf 0.36 (chloroform : methanol : acetic acid = 100 : 10 : 1) ;
NMR (DMSO-d₆): δ 7.85 (2H, d, J=8.4Hz), 7.74 (2H, d, J=8.4Hz), 7.49 (2H, d, J=8.2Hz), 7.26 (2H, d, J=8.2Hz), 4.13 (1H, dd, J=4.4, 7.8Hz), 3.42-3.12 (2H, m), 2.34 (3H, s), 2.00-1.53 (4H, m).

### Example 2(47)

### N-(4-Ethynyl)phenylsulfonyl-N'-(4-methoxyphenyl)-D-glutamine

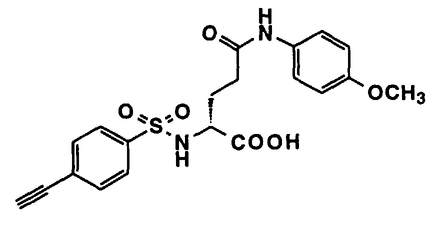

TLC : Rf 0.33 (chloroform : methanol : acetic acid = 100 : 10 : 5) ;
NMR (DMSO-d₆): δ 9.73 (1H, br.s), 8.31 (1H, d, J=8.4Hz), 7.75 (2H, d, J=8.2Hz), 7.61 (2H, d, J=8.2Hz), 7.44 (2H, d, J=8.8Hz), 6.84 (2H, d, J=8.8Hz), 4.45 (1H, s), 3.78 (1H, dt, J=5.4, 8.4Hz), 3.70 (3H, s), 2.32 (2H, t, J=6.8Hz), 2.05-1.63 (2H, m).

### Reference Example 4

### Phenyl 4-(chlorosulfonyl)phenyl methyl ketone

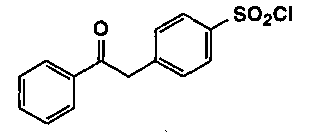

To chlorosulfonic acid (8.45 ml), phenyl benzyl ketone (5.0 g) was added slowly at 0 °C. The solution was stirred at 10-15 °C for 30 minutes, and at 50 °C for 15 minutes. The reaction solution was poured onto ice, and the mixture was extracted with ethyl acetate. The extract was washed with water, a saturated aqueous solution of sodium chloride, dried over anhydrous magnesium sulfate, and concentrated. Crystals obtained were then washed with ether, and dried to give the compound of the present invention (2.25 g). The residue remaining in ether layer was purified by column chromatography on silica gel (hexane : ethyl acetate = 17 : 3) to give the title compound (1.18 g; total 3.43 g) having the following physical data.
TLC : Rf 0.27 (hexane : ethyl acetate = 9 : 1).

### Reference Example 5

### N-[(4-Benzoylmethylphenylsulfonyl]glycine t-butyl ester

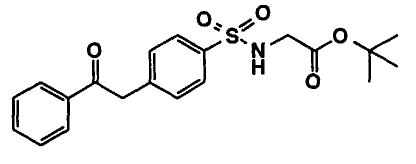

To a solution of glycine t-butyl ester hydrochloride (285 mg) and triethylamine (471 ul) in dichloromethane (10 ml), the compound prepared in Reference Example 4 (500 mg) was added. The mixture was stirred overnight at room temperature. The reaction mixture was concentrated, and the residue was extracted with ethyl acetate. The extract was washed with 1N hydrochloric acid, water, a saturated aqueous solution of sodium chloride, dried over anhydrous magnesium sulfate, and then concentrated to give the title compound (486 mg) having the following physical data.
TLC : Rf 0.21 (hexane : ethyl acetate = 7 : 3) ;
NMR (CDCl₃): δ 8.05-7.97 (2H, m), 7.83 (2H, d, J=8.6Hz), 7.65-7.45 (3H, m), 7.41 (2H, d, J=8.6Hz), 5.05 (1H, t, J=5.4Hz), 4.35 (2H, s), 3.67 (2H, d, J=5.4Hz), 1.34 (9H, s).

### Reference Example 6

### N-[[4-(4-Methylbenzoylmethyl)phenyl]sulfonyl]-D-alanine

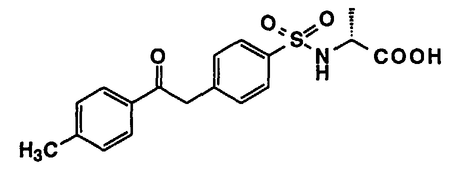

A solution of N-[4-[(4-methylphenyl)ethynyl]phenylsulfonyl]-D-alanine t-butyl ester (720 mg) (which was obtained by the same procedure as described in Example 1 using a corresponding compound) in formic acid (7 ml) was refluxed for 4 hours. To the reaction solution, TFA (7 ml) was added, and the mixture was refluxed for another 1 hour. The reaction solution was concentrated, and then washed with ether to give the title compound (627 mg) having the following physical data.
TLC : Rf 0.42 (ethyl acetate : acetic acid = 99 : 1) ;
NMR (DMSO-d₆): δ 12.90-12.50 (1H, br.s), 8.10 (1H, d, J=8.4Hz), 7.95 (2H, d, J=8.2Hz), 7.73 (2H, d, J=8.2Hz), 7.44 (2H, d, J=8.2Hz), 7.35 (2H, d, J=8.2Hz), 4.48 (2H, s), 3.85-3.68 (1H, m), 2.39 (3H, s), 1.13 (3H, d, J=7.2Hz).

### Reference Example 6(1)

### N-[[4-(4-Isopropylbenzoylmethyl)phenyl]sulfonyl]-D-tryptophan methyl ester

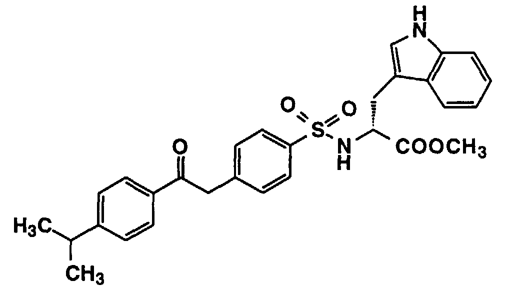

The same procedure as Reference Example 6 using a corresponding compound gave the title compound having the following physical data.
TLC : Rf 0.43 (hexane : ethyl acetate = 1 : 1).

### Reference Example 7

### N-[4-(2-Hydroxy-2-phenylethyl)phenylsulfonyl]glycine t-butyl ester

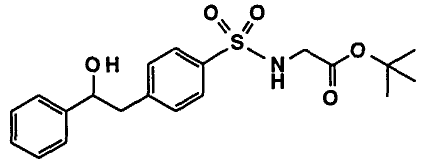

To a solution of the compound prepared in Reference Example 5 (390 mg) in ethanol (20 ml), sodium borohydride (111 mg) was added. The mixture was stirred at room temperature for 1 hour. The reaction mixture was concentrated. The residue was neutralized with 1N hydrochloric acid. The mixture was extracted with ethyl acetate. The extract was washed with a saturated aqueous solution of sodium chloride, dried over anhydrous magnesium sulfate, and concentrated. Crystals that appeared were washed with ether to give the title compound (307 mg) having the following physical data.
TLC : Rf 0.51 (hexane : ethyl acetate = 1 : 1) ;
NMR (CDCl₃): δ 7.78 (2H, d, J=8.8Hz), 7.40-7.26 (7H, m), 5.04-4.85 (2H, m), 3.66 (2H, d, J=5.4Hz), 3.12-3.04 (2H, m), 1.92 (1H, m), 1.36 (9H, s).

### Reference Example 7(1)-7(2)

The same procedure as Reference Example 7, using the compound prepared in Reference Example 6 and 6(1), was carried out to give the following compounds.

### Reference Example 7(1)

### N-[4-[2-Hydroxy-2-(4-methylphenyl)ethyl]phenylsulfonyl]-D-alanine

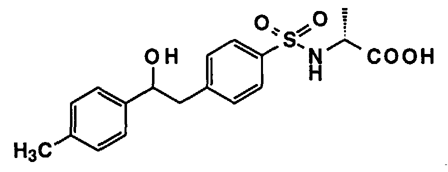

TLC : Rf 0.30 (chloroform : methanol : acetic acid = 90 : 10 : 1).

### Reference Example 7(2)

### N-[4-[2-Hydroxy-2-(4-isopropylphenyl)ethyl]phenylsulfonyl]-D-tryptophan methyl ester

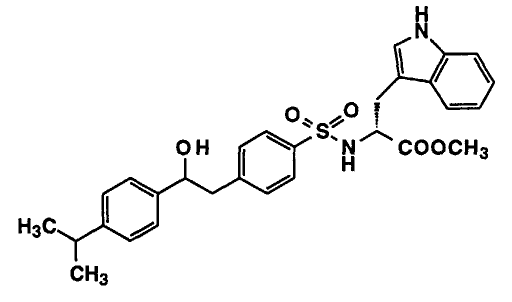

TLC : Rf 0.32 (hexane : ethyl acetate = 1 : 1).

### Example 3

### N-[4-(2-Phenylethenyl)phenylsulfonyl]glycine

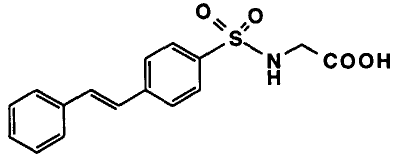

To a solution of the compound prepared in Reference Example 7 (307 mg) in toluene (10 ml), p-toluenesulfonic acid monohydrate (30 mg) was added. The mixture was stirred at 50 °C for 2 hours, at 70 °C for 2 hours, and at 90 °C for 4 hours. The reaction mixture was cooled to room temperature, and filtered to give the title compound (212 mg) having the following physical data.
TLC : Rf 0.58 (chloroform : methanol : acetic acid = 16 : 3 : 1) ;
NMR (CDCl₃): δ 12.80-12.50 (1H, br.s), 8.03 (1H, t, J=6.2Hz), 7.82-7.72 (4H, m), 7.64 (2H, d, J=7.3Hz), 7.43 (1H, d, J=16.6Hz), 7.41 (2H, t, J=8.7Hz), 7.33 (1H, d, J=16.6Hz), 7.31 (1H, t, J=7.3Hz), 3.59 (2H, d, J=6.2Hz).

### Example 3(1)-3(2)

The same procedure as Example 3, using a compound prepared in Reference Example 7(1)-7(2) gave the following compounds (optionally followed by the procedure for the same purpose as Example 2).

### Example 3(1)

### N-[4-[2-(4-Methylphenyl)ethenyl]phenylsulfonyl]-D-alanine

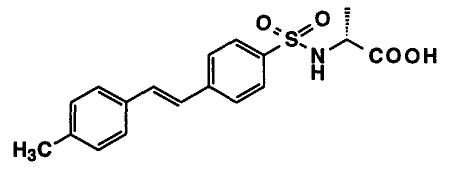

TLC : Rf 0.36 (chloroform : methanol : acetic acid = 90 : 10 : 1);
NMR (CD₃OD): δ 7.81 (2H, d, J=8.4Hz), 7.67 (2H, d, J=8.4Hz), 7.47 (2H, d, J=8.0Hz), 7.30 (1H, d, J=16.4Hz), 7.18 (2H, d, J=8.0Hz), 7.14 (1H, d, J=16.4Hz), 3.92 (1H, q, J=7.2Hz), 2.34 (3H, s), 1.31 (3H, d, J=7.2Hz).

### Example 3(2)

### N-[4-[2-(4-Isopropylphenyl)ethenyl]phenylsulfonyl]-D-tryptophan

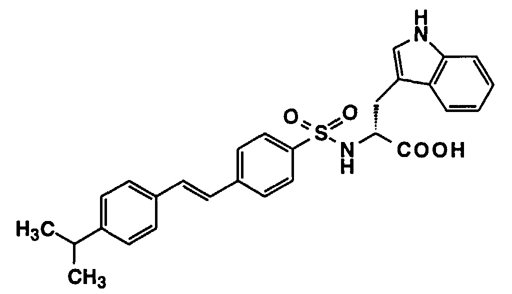

TLC : Rf 0.40 (chloroform : methanol : acetic acid = 90 : 10 : 1) ;
NMR (CD₃OD): δ 7.51 (2H, d, J=8.0Hz), 7.48 (2H, d, J=8.2Hz), 7.44-7.32 (3H, m), 7.30-6.90 (8H, m), 4.09 (1H, dd, J=8.4, 5.4Hz), 3.23 (1H, dd, J=14.2, 5.4Hz), 3.01 (1H, dd, J=14.2, 8.4Hz), 3.00-2.84 (1H, m), 1.27 (6H, d, J=7.0Hz).

### Reference Example 8

### 1-Hexenyl-bis(cyclopentadienyl)zirconium chloride

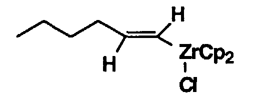

To 1-Hexyne (0.36 ml), a solution of bis(cyclopentadienyl)zirconium chloride hydride (805 mg) in benzene (6 ml) was added, and the mixture was stirred at room temperature for 2 hours. The reaction mixture was concentrated, and THF was added to give the title compound.

### Example 4

### N-[4-(1-Hexenyl)phenylsulfonyl]-N-t-butoxycarbonyl-D-alanine t-butyl ester

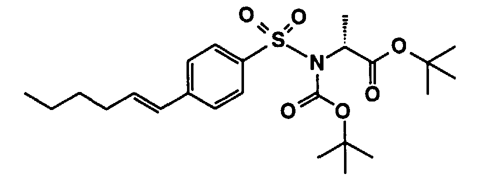

To the solution of the compound obtained in Reference Example 8 in THF, tetrakis(triphenylphosphine)palladium (120 mg) was added, and then a solution of N-(4-trifluoromethylsilyloxyphenylsulfonyl)-N-t-butoxycarbonyl-D-alanine t-butyl ester (555 mg), which was obtained by the same procedure as Reference Example 2 using corresponding compounds in THF (5 ml), was added, and the reaction mixture was stirred at room temperature for 16 hours. To the reaction solution, 1N hydrochloric acid was added, and the mixture was extracted with ethyl acetate. The extract was washed with 1N hydrochloric acid, a saturated aqueous solution of sodium bicarbonate, and a saturated aqueous solution of sodium chloride, dried over anhydrous magnesium sulfate, and concentrated. The residue was purified by column chromatography on silica gel (hexane : ethyl acetate = 4 : 1) to give the title compound (482 mg) having the following physical data.
TLC : Rf 0.60 (hexane : ethyl acetate = 4 : 1) ;
NMR (CDCl₃): δ 7.90 (2H, d, J=8.6Hz), 7.43 (2H, d, J=8.6Hz), 6.41 (2H, m), 5.07 (1H, q, J=7.0Hz), 2.25 (2H, m), 1.63 (3H, d, J=7.0Hz), 1.50-1.21 (22H, m), 0.94 (3H, t, J=7.2Hz).

### Example 5

### N-[4-(1-Hexenyl)phenylsulfonyl]-D-alanine

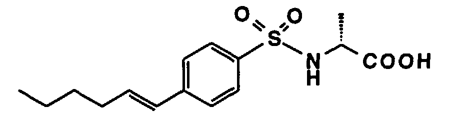

The same procedure as Example 2 using the compound prepared in Example 4 (138 mg) gave the title compound having the following physical data.
TLC : Rf 0.46 (chloroform : methanol : acetic acid = 90 : 10 : 1) ;
NMR (C₅D₅N): δ 9.95 (1H, d, J=8.6Hz), 8.18 (2H, d, J=8.4Hz), 7.48 (2H, d, J=8.4Hz), 6.36 (1H, d, J=15.8Hz), 6.33-6.20 (1H, m), 4.60 (1H, m), 2.09 (2H, m), 1.63 (3H, d, J=7.2Hz), 1.28 (4H, m), 0.85 (3H, t, J=7.2Hz).

### Example 6

### N-Hydroxy-[N-[4-[2-(4-methylphenyl)ethynyl]phenylsulfonyl]-D-tryptophyl]amide

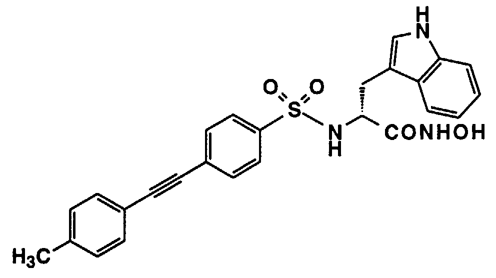

To a solution of the compound prepared in Example 2(4) (230 mg) in THF (5 ml), CDI (100 mg) was added. The mixture was stirred overnight at room temperature. To the reaction mixture hydroxylamine hydrochloride (80 mg) was added and then stirred at room temperature for 4 hours. To the reaction mixture, 1N hydrochloric acid was added. The mixture was extracted with ethyl acetate. The extract was washed with water, a saturated aqueous solution of sodium chloride, dried, and concentrated. The compound obtained was washed with ether, and dried to give the title compound (126 mg) having the following physical data.
TLC : Rf 0.35 (chloroform : methanol = 9 : 1) ;
NMR (DMSO-d₆): δ 10.73 (1H, s), 9.40-8.30 (1H, br), 7.54 (2H, d, J=8.6Hz), 7.49 (2H, d, J=8.0Hz), 7.43 (2H, d, J=8.0Hz), 7.36-7.23 (4H, m), 7.10-6.86 (3H, m), 3.83 (1H, t, J=7.2Hz), 2.96 (1H, dd, J=14.0, 6.6Hz), 2.72 (1H, dd, J=14.0, 8.0Hz), 2.36 (3H, s).

### Example 6(1)-6(23)

The same procedure as Example 6 using the compounds prepared in Example 2(2), 3(1), 5, 2(1), 2(36), 2(33), 2(26), 2(34), 2(11), 2(10), 2(9), 2(35), and 2(37)-2(47) gave the following compounds.

### Example 6(1)

### N-Hydroxy-[N-[4-(1-pentynyl)phenylsulfonyl]-D-alanyl]amide

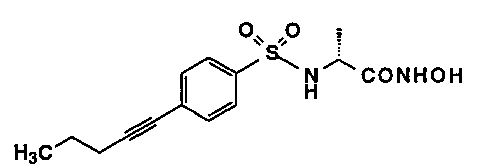

TLC : Rf 0.39 (chloroform : methanol = 9 : 1) ;
NMR (DMSO-d₆): δ 10.56 (1H, s), 8.82 (1H, s), 8.12 (1H, d, J=8.2 Hz), 7.73 (2H, d, J=8.4 Hz), 7.54 (2H, d, J=8.4 Hz), 3.64 (1H, m), 2.44 (2H, m, overlap with DMSO), 1.58 (2H, m), 1.01 (6H, m).

### Example 6(2)

### N-Hydroxy-[N-[4-[2-(4-methylphenyl)ethenyl]phenylsulfonyl]-D-alanyl]amide

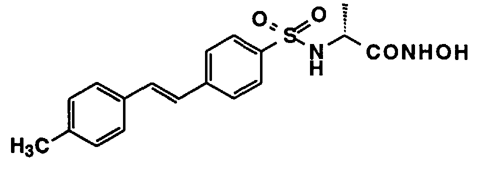

TLC : Rf 0.55 (chloroform : methanol = 9 : 1) ;
NMR (DMSO-d₆): δ 10.57 (1H, s), 8.94 (1H, s), 8.01 (1H, d, J=8.2Hz), 7.85-7.55 (4H, m), 7.53 (2H, d, J=8.2Hz), 7.39 (1H, d, J=16.2Hz), 7.25 (1H, d, J=16.2Hz), 7.21 (2H, d, J=8.2Hz), 3.78-3.58 (1H, m), 2.32 (3H, s), 1.02 (3H, d, J=7.2Hz).

### Example 6(3)

### N-Hydroxy-[N-[4-(1-hexenyl)phenylsulfonyl]-D-alanyl]amide

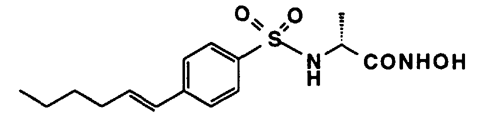

TLC : Rf 0.38 (chloroform : methanol : acetic acid = 90 : 10 : 1) ;
NMR (C₅D₅N): δ 12.35 (1H, br), 10.19 (1H, br), 8.08 (2H, d, J=8.6Hz), 7.41 (2H, d, J=8.6Hz), 6.32 (2H, m), 4.52 (1H, m), 2.10 (2H, m), 1.55 (3H, d, J=7.0Hz), 1.31 (4H, m), 0.85 (3H, t, J=6.8Hz).

### Example 6(4)

### N-Hydroxy-[N-[4-[2-(4-methylphenyl)ethynyl]phenylsulfonyl]-D-alanyl]amide

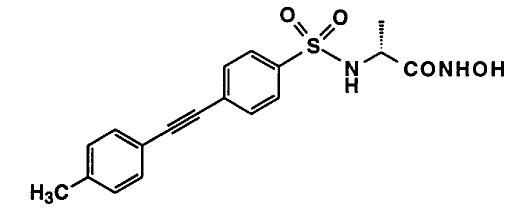

TLC : Rf 0.27 (chloroform : methanol : acetic acid = 100 : 10 : 1) ;
NMR (CDCl₃+CD₃OD): δ 7.82 (2H, d, J=8.0Hz), 7.63 (2H, d, J=8.0Hz), 7.44 (2H, d, J=8.0Hz), 7.18 (2H, d, J=8.0Hz), 3.9-3.7 (1H, m), 2.38 (3H, s), 1.22 (3H, d, J=8.0Hz).

### Example 6(5)

### N-Hydroxy-[N-[4-[2-(4-methylphenyl)ethynyl]phenylsulfonyl]-N'-methyl-N'-(2-phenylethyl)-D-glutaminyl]amide

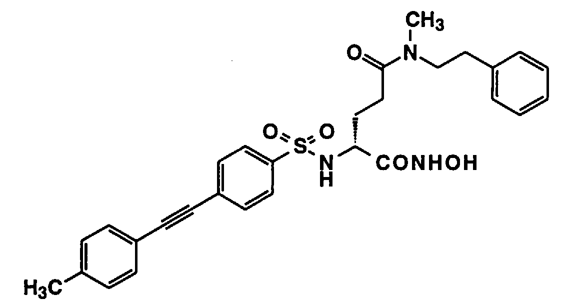

TLC : Rf 0.59 (chloroform : methanol = 9 : 1) ;
NMR (CD₃OD): δ 7.83 and 7.81 (2H, d, J=8.0 Hz), 7.63 and 7.62 (2H, d, J=8.0 Hz), 7.40 and 7.38 (2H, d, J=7.0 Hz), 7.35-7.10 (7H, m), 3.75 and 3.60 (1H, m), 3.55-3.40 (2H, m), 2.85 and 2.80 (3H, s), 2.40 (3H, s), 2.25 (1H, m), 2.05 (1H, m), 2.00-1.60 (2H, m).

### Example 6(6)

### N-Hydroxy-[N-[4-[2-(4-methylphenyl)ethynyl]phenylsulfonyl]-L-alanyl]amide

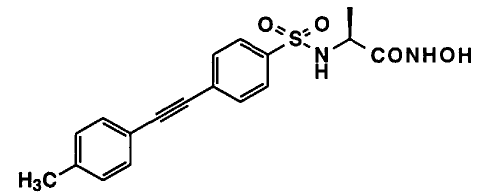

TLC : Rf 0.37 (chloroform : methanol = 9 : 1) ;
NMR (DMSO-d₆): δ 9.45-8.60 (2H, br), 7.81 (2H, d, J=8.4Hz), 7.69 (2H, d, J=8.4Hz), 7.48 (2H, d, J=8.0Hz), 7.26 (2H, d, J=8.0Hz), 3.68 (1H, q, J=7.0Hz), 2.35 (3H, s), 1.04 (3H, d, J=7.0Hz).

### Example 6(7)

### N-Hydroxy-[N-[(4-ethynyl)phenylsulfonyl]-D-alanyl]amide

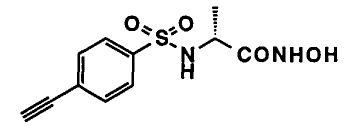

TLC : Rf 0.19 (chloroform : methanol : acetic acid = 100 : 10 : 1) ;
NMR (DMSO-d₆): δ 10.57 (1H, br.s), 8.82 (1H, br.s), 8.18 (1H, d, J=8.0Hz), 7.77 (2H, d, J=8.4Hz), 7.64 (2H, d, J=8.4Hz), 4.46 (1H, s), 3.72-3.58 (1H, m), 1.01 (3H, d, J=7.0Hz).

### Example 6(8)

### N-Hydroxy-[N-[4-[2-(4-methylphenyl)ethynyl]phenylsulfonyl]-N'-methyl-N'-benzyl-D-glutaminyl]amide

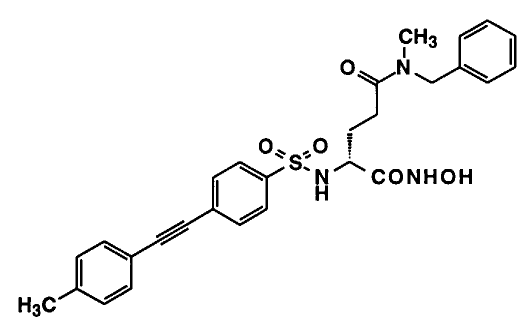

TLC : Rf 0.49 (chloroform : methanol = 9 : 1) ;
NMR (DMSO-d₆): δ 10.00-8.40 (2H, br), 7.81 and 7.77 (total 2H, d and d, J=8.4 and 8.4Hz), 7.68 and 7.61 (total 2H, d and d, J=8.4 and 8.4Hz), 7.49 and 7.48 (total 2H, d and d, J=8.2 and 8.2Hz), 7.44-7.22 (5H, m), 7.22-7.10 (2H, m), 4.46 and 4.37 (total 2H, s and s), 3.75-3.55 (1H, m), 2.75 and 2.73 (total 3H, s and s), 2.36 (3H, s), 2.30-2.00 (2H, m), 1.90-1.55(2H, m).

### Example 6(9)

### N-Hydroxy-[N-[4-(1-octynyl)phenylsulfonyl]-D-alanyl]amide

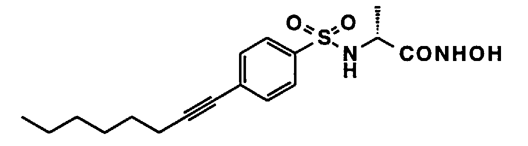

TLC : Rf 0.30 (chloroform : methanol = 9 : 1) ;
NMR (DMSO-d₆): δ 10.57 (1H, s), 9.00-8.60 (1H, br), 8.11 (1H, d, J=8.4Hz), 7.74 (2H, d, J=8.4Hz), 7.53 (2H, d, J=8.4Hz), 3.80-3.55 (1H, m), 2.65-2.30 (2H, m, overlap with DMSO), 1.70-1.15 (8H, m), 1.02 (3H, d, J=7.0Hz), 0.88 (3H, t, J=6.6Hz).

### Example 6(10)

### N-Hydroxy-[N-[4-[4-(1-hexynyl)]phenylsulfonyl]-D-alanyl]amide

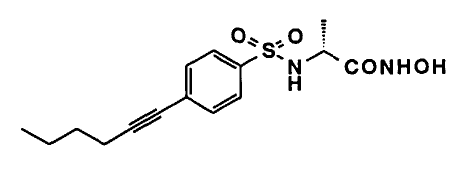

TLC : Rf 0.32 (chloroform : methanol = 9 : 1) ;
NMR (DMSO-d₆): δ 10.70-10.40 (1H, br), 9.00-8.65 (1H, br), 8.30-8.00 (1H, br), 7.74 (2H, d, J=8.4Hz), 7.54 (2H, d, J=8.4Hz), 3.75-3.55 (1H, m), 2.60-2.40 (2H, m, overlap with DMSO), 1.65-1.30 (4H, m), 1.02 (3H, d, J=7.0Hz), 0.92 (3H, t, J=7.2Hz).

### Example 6(11)

### N-Hydroxy-[N-[4-[4-(1-heptynyl)]phenylsulfonyl]-D-alanyl]amide

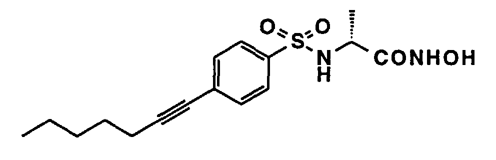

TLC : Rf 0.37 (chloroform : methanol = 9 : 1) ;
NMR (DMSO-d₆): δ 9.60-8.70 (3H, br), 7.74 (2H, d, J=8.4Hz), 7.53 (2H, d, J=8.4Hz), 3.64 (1H, q, J=7.0Hz), 2.55-2.40 (2H, m, overlap with DMSO), 1.68-1.47 (2H, m), 1.47-1.20 (4H, m), 1.01 (3H, d, J=7.0Hz), 0.90 (3H, t, J=7.0Hz).

### Example 6(12)

### N-Hydroxy-[N-[4-(1-propynyl)phenylsulfonyl]-D-alanyl]amide

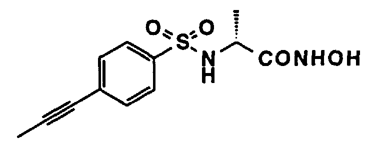

TLC : Rf 0.25 (chloroform : methanol : acetic acid = 100 : 10 : 1) ;
NMR (DMSO-d₆): δ 8.20 (1H, br.d, J=8.4Hz), 7.72 (2H, d, J=8.4Hz), 7.53 (2H, d, J=8.4Hz), 3.84-3.68 (1H, m), 2.07 (3H, s), 1.14 (3H, d, J=7.2Hz).

### Example 6(13)

### N-Hydroxy-[N-[4-(1-heptynyl)phenylsulfonyl]-L-alanyl]amide

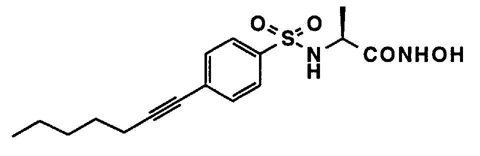

TLC : Rf 0.34 (chloroform : methanol = 9 : 1) ;
NMR (DMSO-d₆): δ 10.58 (1H, s), 8.83 (1H, s), 8.12 (1H, d, J=8.4Hz), 7.74 (2H, d, J=8.8Hz), 7.54 (2H, d, J=8.8Hz), 3.75-3.55 (1H, m), 2.55-2.38 (2H, m, overlap with DMSO), 1.65-1.46 (2H, m), 1.46-1.20 (4H, m), 1.02 (3H, d, J=7.0Hz), 0.90 (3H, t, J=7.2Hz).

### Example 6(14)

### N-Hydroxy-[N-[4-(1-pentynyl)phenylsulfonyl]-L-alanyl]amide

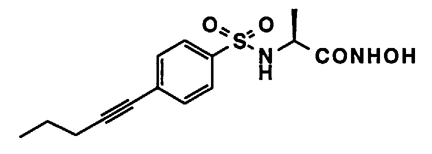

TLC : Rf 0.32 (chloroform : methanol = 9 : 1) ;
NMR (DMSO-d₆): δ 9.40-8.80 (2H, br), 7.75 (2H, d, J=8.4Hz), 7.54 (2H, d, J=8.4Hz), 3.64 (1H, q, J=6.8Hz), 2.44 (2H, t, J=7.0Hz), 1.70-1.50 (2H, m), 1.15-0.95 (6H, m).

### Example 6(15)

### N-Hydroxy-[N-(4-ethynyl)phenylsulfonyl-N'-methyl-N'-benzyl-D-glutaminyl]amide

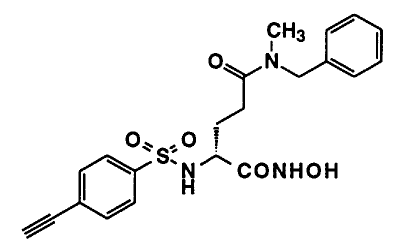

TLC : Rf 0.59 (chloroform : methanol : acetic acid = 90 : 10 : 5) ;
NMR (DMSO-d₆): δ 9.19 (1H, br s), 7.78-7.70 (2H, m), 7.63-7.53 (2H, m), 7.36-7.11(6H, m), 4.55-4.35 (3H, m), 3.60(1H, q, J=6.9Hz), 2.70 (3H, s), 2.22-2.07 (2H, m), 1.76-1.58 (2H, m).

### Example 6(16)

### N-Hydroxy-[N-(4-ethynyl)phenylsulfonyl-N'-(3-phenylpropyl)-D-glutaminyl]amide

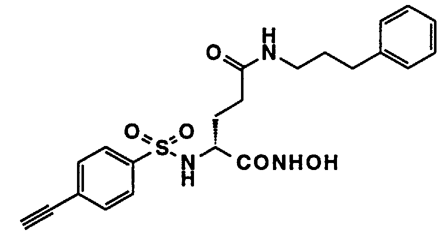

TLC : Rf 0.50 (chloroform : methanol : acetic acid = 90 : 10 : 5) ;
NMR (DMSO-d₆): δ 9.10 (1H, br s), 7.87-7.68 (3H, m), 7.59 (2H, d, J=8.4Hz), 7.31-7.12 (6H, m), 4.43 (1H, s), 3.58-3.47 (1H, m), 3.06-2.92 (2H, m), 2.60-2.50 (2H, m), 2.02-1.90 (2H, m), 1.74-1.54 (2H, m).

### Example 6(17)

### N-Hydroxy-[N-(4-ethynyl)phenylsulfonyl-N'-(2-phenylethyl)-D-glutaminyl]amide

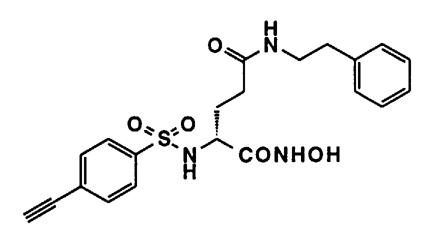

TLC : Rf 0.32 (chloroform : methanol = 9 : 1) ;
NMR (DMSO-d₆): δ 10.60 (1H, s), 8.21 (1H, d, J=8.4Hz), 7.87 (1H, t, J=5.6Hz), 7.77 (2H, d, J=8.4Hz), 7.63 (2H, d, J=8.4Hz), 7.35-7.10 (5H, m), 4.45 (1H, s), 3.58 (1H, m), 3.30-3.15 (2H, m), 2.67 (2H, t, J=7.4Hz), 2.20-1.80 (2H, m), 1.80-1.45 (2H, m).

### Example 6(18)

### N-Hydroxy-[N-(4-ethynyl)phenylsulfonyl-N'-benzyl-D-glutaminyl]amide

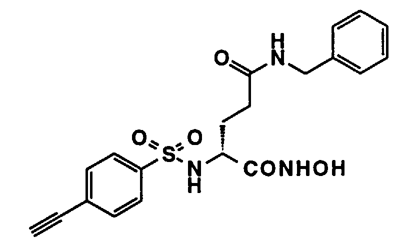

TLC : Rf 0.26 (chloroform : methanol : acetic acid = 100 : 10 : 5) ;
NMR (DMSO-d₆): δ 10.60 (1H, br.s), 9.20-8.50 (1H, br.), 8.30 (1H, t, J=5.8Hz), 8.21 (1H, d, J=8.4Hz), 7.75 (2H, d, J=8.2Hz), 7.62 (2H, d, J=8.2Hz), 7.35-7.18 (5H, m), 4.47 (1H; s), 4.20 (2H, d, J=5.8Hz), 3.59 (1H, br.q, J=7.6Hz), 2.20-1.93 (2H, m), 1.82-1.51 (2H, m).

### Example 6(19)

### N-Hydroxy-[N-(4-ethynyl)phenylsulfonyl-N'-(1,2-diphenylethyl)-D-glutaminyl]amide

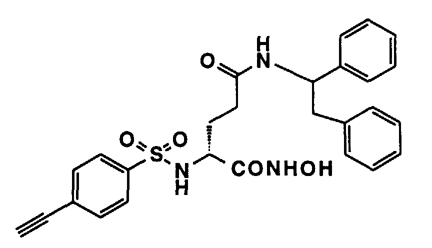

TLC : Rf 0.35 (chloroform : methanol : acetic acid = 100 : 10 : 5) ;
NMR (DMSO-d₆): δ 10.54 (1H, br.s), 8.36 (1H, br.d, J=8.4Hz), 8.18 (1H, br.d, J=8.4Hz), 7.73 (2H, d, J=8.4Hz), 7.60 (2H, d, J=8.4Hz), 7.30-7.15 (10H, m), 4.97 (1H, m), 4.46 (1H, s), 3.53 (1H, m), 2.90 (2H, d, J=7.6Hz), 2.04-1.81 (2H, m), 1.69-1.38 (2H, m).

### Example 6(20)

### N-Hydroxy-[N-(4-ethynyl)phenylsulfonyl-N'-(diphenylmethyl)-D-glutaminyl]amide

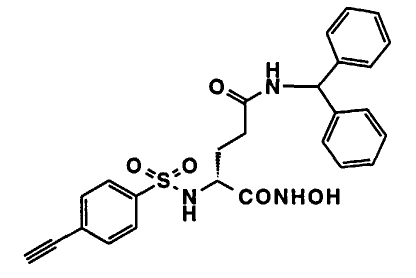

TLC : Rf 0.50 (chloroform : methanol : acetic acid = 100 : 10 : 5) ;
NMR (DMSO-d₆): δ 10.60 (1H, br.s), 8.79 (1H br.d, J=8.8Hz), 8.20 (1H, br.d, J=8.8Hz), 7.74 (2H, d, J=8.4Hz), 7.60 (2H, d, J=8.4Hz), 7.35-7.22 (10H, m), 6.07 (1H, d, J=8.4Hz), 4.47 (1H, s), 3.65-3.54 (1H, m), 2.18-2.08 (2H, m), 1.79-1.55 (2H, m).

### Example 6(21)

### N-Hydroxy-[N-(4-ethynyl)phenylsulfonyl-N'-phenyl-D-glutaminyl]amide

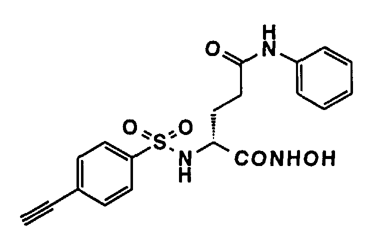

TLC : Rf 0.33 (chloroform : methanol : acetic acid = 100 : 10 : 5) ;
NMR (DMSO-d₆): δ 10.64 (1H, br.s), 9.88 (1H, br.s), 8.84 (1H, br.s), 7.76 (2H, d, J=8.2Hz), 7.61 (2H, d, J=8.2Hz), 7.54 (2H, d, J=7.4Hz), 7.27 (2H, t, J=7.4Hz), 7.00 (1H, t, J=7.4Hz), 4.45 (1H, s), 3.70-3.59 (1H, m), 2.30-2.20 (2H, m), 1.91-1.62 (2H, m).

### Example 6(22)

### N-Hydroxy-[N-[4-[2-(4-methylphenyl)ethynyl]phenylsulfonyl-D-prolyl]amide

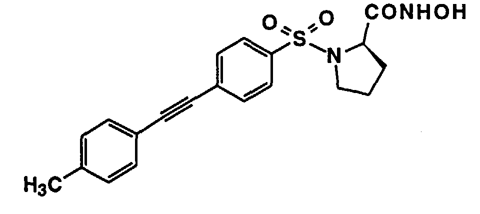

TLC : Rf 0.24 (chloroform : methanol : acetic acid =100 : 10 : 1) ;
NMR (DMSO-d₆): δ 10.70 (1H, br.s), 8.96 (1H, br.s), 7.87 (2H, d, J=8.6Hz), 7.76 (2H, d, J=8.6Hz), 7.49 (2H, d, J=8.4Hz), 7.26 (2H, d, J=8.4Hz), 3.92 (1H, dd, J=4.8, 7.4Hz), 3.46-3.11 (2H, m), 2.34 (3H, s), 1.92-1.45 (4H, m).

### Example 6(23)

### N-Hydroxy-[N-(4-ethynyl)phenylsulfonyl-N'-(4-methoxyphenyl)-D-glutaminyl]amide

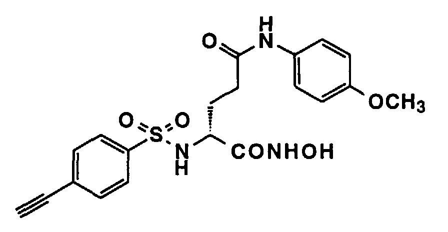

TLC : Rf 0.29 (chloroform : methanol acetic acid = 100 : 10 : 5) ;
NMR (DMSO-d₆): δ 10.63 (1H, br.s), 9.72 (1H, br.s), 9.02-8.64 (1H, br.), 8.22 (1H, d, J=8.8Hz), 7.76 (2H, d, J=8.4Hz), 7.61 (2H, d, J=8.4Hz), 7.44 (2H, d, J=9.2Hz), 6.84 (2H, d, J=9.2Hz), 3.92 (1H, dd, J=4.8, 7.4Hz), 4.45 (1H, s), 3.70 (3H, s), 3.69-3.58 (1H, m), 2.26-2.16 (2H, m), 1.90-1.62 (2H, m).

### Example 7

### N-Methyl-N-[4-[2-(4-methylphenyl)ethynyl]phenylsulfonyl]-D-alanine methyl ester

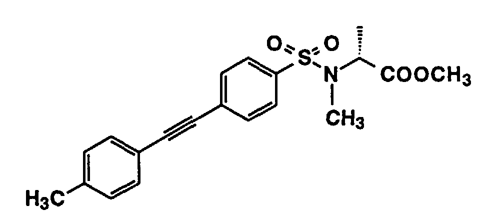

To a solution of the compound (3.0 g) prepared in Example 1(33) in N,N-dimethylformamide (20 ml) , methyl iodide (1.07 ml) and potassium carbonate (2.32 g) were added, and the mixture was stirred at room temperature for 1 hour. The reaction mixture was neutralized with 1N hydrochloric acid, and extracted with ethyl acetate. The extract was washed with a saturated aqueous solution of sodium chloride, and dried over anhydrous magnesium sulfate, and then concentrated. Crystals that appeared were washed with hexane to give the title compound (2.89 g) having the following physical data.
TLC : Rf 0.47 (hexane : ethyl acetate = 7 : 3) ;
NMR (CDCl₃): δ 7.78 (2H, d, J=8.2Hz), 7.62 (2H, d, J=8.2Hz), 7.44 (2H, d, J=8.0Hz), 7.18 (2H, d, J=8.0Hz), 4.78 (1H, q J=7.0Hz), 3.55 (3H, s), 2.86 (3H, s), 2.38 (3H, s), 1.37 (3H, d, J=7.0Hz).

### Example 8

### N-2-(1-Imidazolyl)ethyl-N-[4-[2-(4-methylphenyl)ethynyl]phenylsulfonyl]-D-alanine methyl ester

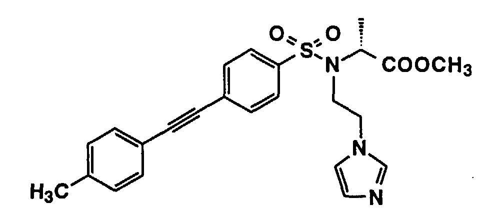

To a solution of the compound prepared in Example 1(33) (500 mg) in THF (15 ml), 1-(2-hydroxyethyl)imidazole (313 mg) and triphenyl phosphine (730 mg) were added. To the mixture, diethylazodicarboxylate (1.1 ml; 40% toluene solution) was added at 0 °C, and the mixture was stirred at room temperature for 16 hours. The reaction mixture was concentrated, and the residue was purified by column chromatography on silica gel (ethyl acetate → ethyl acetate: methanol = 19 : 1 → chloroform : ethyl acetate = 1 : 1 → chloroform : methanol = 9 : 1) to give the title compound (638 mg) having the following physical data.
TLC : Rf 0.32 (chloroform : methanol = 19 : 1) ;
NMR (CDCl₃): δ 7.76 (2H, d, J=8.8Hz), 7.63 (2H, d, J=8.8Hz), 7.54 (1H, s), 7.44 (2H, d, J=8.4Hz), 7.18 (2H, d, J=8.4Hz), 7.07 (1H, s), 6.98 (1H, s), 4.60 (1H, q, J=7.4Hz), 4.45-4.15 (2H, m), 3.59 (1H, ddd, J=15.6, 7.4, 4.4Hz), 3.46 (3H, s), 3.37 (1H, dd, J=15.6, 8.0Hz), 2.38 (3H, s), 1.09 (3H, d, J=7.4Hz).

### Example 9

### N-Methyl-N-[4-[2-(4-methylphenyl)ethynyl]phenylsulfonyl]-D-alanine

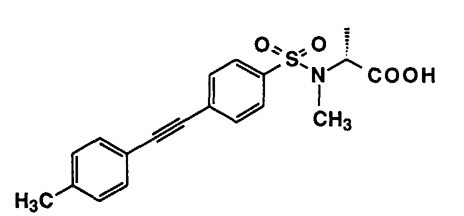

To a solution of the compound prepared in Example 7 (1.48 g) in THF (20 ml), a 1N aqueous solution of sodium hydroxide (4.8 ml) was added, and the mixture was stirred at room temperature for 3 hours. The reaction mixture was neutralized with a 1N hydrochloric acid, and extracted with ethyl acetate. The extract was washed with a saturated aqueous solution of sodium chloride, dried over anhydrous sodium sulfate, and concentrated to give the title compound having the following physical data.
TLC : Rf 0.44 (chloroform : methanol = 4 : 1) ;
NMR (CDCl3+CD3OD): δ 7.80 (2H, d, J=7.0Hz), 7.60 (2H, d, J=7.0Hz), 7.45 (2H, d, J=7.0Hz), 7.20 (2H, d, J=7.0Hz), 4.75 (1H, q, J=7.0Hz), 2.85 (3H, s), 2.35 (3H, s), 1.35 (3H, d, J=7.0Hz).

### Example 9(1) - 9(15)

The following compounds were obtained by the same procedure as Example 7 → Example 9 using a corresponding halogen compound, or by the same procedure as Example 8 → Example 9 using a corresponding alcohol compound.

### Example 9(1)

### N-Benzyl-N-[4-[2-(4-methylphenyl)ethynyl]phenylsulfonyl]-D-alanine

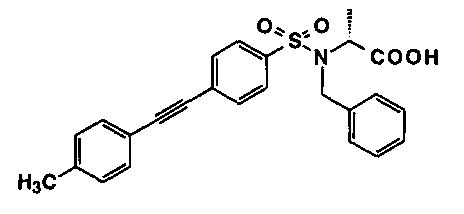

TLC : Rf 0.44 (chloroform : methanol = 9 : 1) ;
NMR (DMSO-d6): δ 12.79 (1H, br.s), 7.83 (2H, d, J=8.4Hz), 7.68 (2H, d, J=8.4Hz), 7.50 (2H, d, J=8.2Hz), 7.40-7.16 (7H, m), 4.65 (1H, d, J=16.6Hz), 4.52 (1H, q, J=7.2Hz), 4.36 (1H, d, J=16.6Hz), 2.35 (3H, s), 1.20 (3H, d, J=7.2Hz).

### Example 9(2)

### N-Carboxymethyl-N-[4-[2-(4-methylphenyl)ethynyl]phenylsulfonyl]-D-alanine

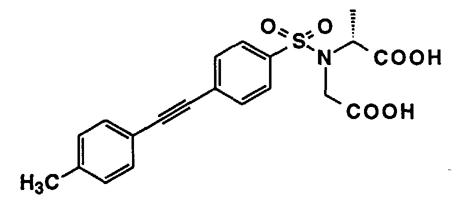

TLC : Rf 0.42 (chloroform : methanol : acetic acid : water = 16 : 4 : 1 : 1) ;
NMR (DMSO-d₆): δ 12.76 (2H, br.s), 7.87 (2H, d, J=8.4Hz), 7.70 (2H, d, J=8.4Hz), 7.49 (2H, d, J=8.2Hz), 7.26 (2H, d, J=8.2Hz), 4.41 (1H, d, J=7.4Hz), 4.15 (1H, d, J=19.2Hz), 3.97 (1H, d, J=19.2Hz), 2.35 (3H, s), 1. 33 (3H, d, J=7.4Hz).

### Example 9(3)

### N-Methoxymethyl-N-[4-[2-(4-methylphenyl)ethynyl]phenylsulfonyl]-D-alanine

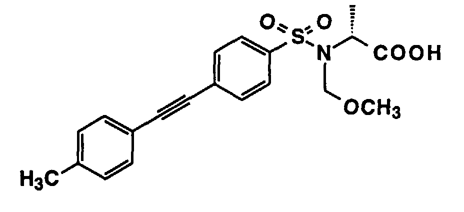

TLC : Rf 0.37 (chloroform : methanol = 9 : 1) ;
NMR (DMSO-d₆): δ 12.82 (1H, s), 7.86 (2H, d, J=8.4Hz), 7.71 (2H, d, J=8.4Hz), 7.49 (2H, d, J=8.0Hz), 7.26 (2H, d, J=8.0Hz), 4.89 (1H, d, J=11.2Hz), 4.72 (1H, d, J=11.2Hz), 4.43 (1H, q, J=7.4Hz), 3.20 (3H, s), 2.35 (3H, s), 1.32 (3H, d, J=7.4Hz).

### Example 9(4)

### N-Methyl-N-[4-[2-[4-(1-imidazolyl)phenyl]ethynyl]phenylsulfonyl]-D-alanine

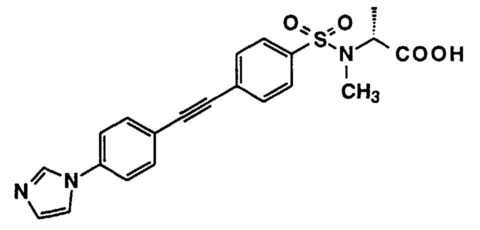

TLC : Rf 0.39 (chloroform : methanol : acetic acid = 90 : 10 : 1).

### Example 9(5)

### N-Methyl-N-[4-(1-pentynyl)phenylsulfonyl]-D-alanine

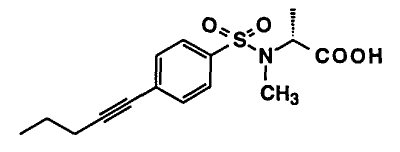

TLC : Rf 0.49 (chloroform : methanol : acetic acid = 90 : 10 : 1) ;
NMR (CDCl₃): δ 7.73 (2H, d, J=8.4Hz), 7.49 (2H, d, J=8.4Hz), 4.79 (1H, q, J=7.4Hz), 2.85 (3H, s), 2.42 (2H, t, J=7.0Hz), 1.74-1.56 (2H, m), 1.37 (3H, d, J=7.4Hz), 1.06 (3H, t, J=7.3Hz).

### Example 9(6)

### N-(2-Benzoyloxyethyl)-N-[4-[2-(4-methylphenyl)ethynyl]phenylsulfonyl]-D-alanine

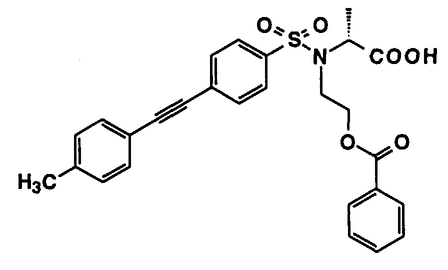

TLC: Rf 0.44 (chloroform : methanol = 9 : 1) ;
NMR (CDCl₃): δ 8.10-7.90 (2H, m), 7.78 (2H, d, J=8.4Hz), 7.60-7.48 (3H, m), 7.46-7.32 (4H, m), 7.15 (2H, d, J=8.0Hz), 4.61 (1H, q, J=7.4Hz), 4.51 (2H, t, J=6.4Hz), 3.82-3.45 (2H, m), 2.37 (3H, s), 1.48 (3H, d, J=7.4Hz).

### Example 9(7)

### N-2-(1-Imidazolyl)ethyl-N-[4-[2-(4-methylphenyl)ethynyl]phenylsulfonyl]-D-alanine

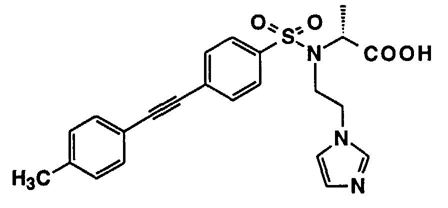

TLC : Rf 0.21 (chloroform : methanol : acetic acid = 80 : 20 : 1).

### Example 9(8)

### N-2-Morpholinoethyl-N-[4-[2-(4-methylphenyl)ethynyl]phenylsulfonyl]-D-alanine

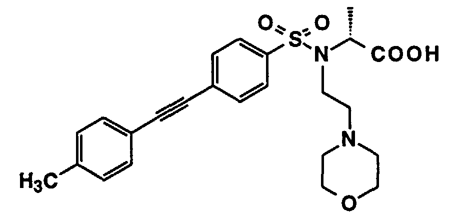

TLC : Rf 0.47 (chloroform : methanol : water = 40 : 10 : 1) ;
NMR (DMSO-d6): δ 7.86 (2H, d, J=8.4Hz), 7.71 (2H, d, J=8.4Hz), 7.48 (2H, d, J=8.0Hz), 7.26 (2H, d, J=8.0Hz), 4.39 (1H, q, J=7.0Hz), 3.61 (4H, t, J=4.4Hz), 3.50-3.20 (2H, m), 2.90-2.40 (6H, m), 2.36 (3H, s), 1.22 (3H, d, J=7.0Hz).

### Example 9(9)

### N-3-Dimethylaminopropyl-N-[4-[2-(4-methylphenyl)ethynyl]-phenylsulfonyl]-D-alanine

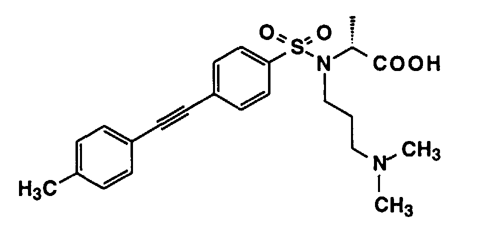

TLC : Rf 0.24 (chloroform : methanol : acetic acid = 80 : 20 : 1).

### Example 9(10)

### N-3-Hydroxypropyl-N-[4-[2-(4-methylphenyl)ethynyl]phenylsulfonyl]-D-alanine

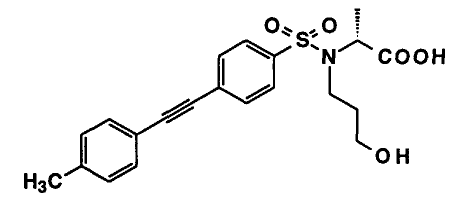

TLC : Rf 0.32 (chloroform : methanol : acetic acid = 90 : 10 : 1).

### Example 9(11)

### N-3-Pyridylmethyl-N-[4-[2-(4-methylphenyl)ethynyl]phenylsulfonyl-D-alanine

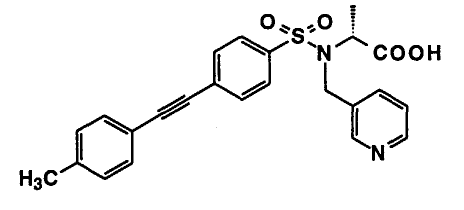

TLC : Rf 0.26 (chloroform : methanol = 9 : 1).

### Example 9(12)

### N-2-t-butoxyethyl-N-[4-[2-(4-methylphenyl)ethynyl]phenylsulfonyl]-D-alanine

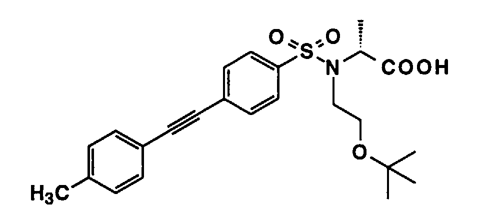

TLC : Rf 0.52 (chloroform : methanol = 9 : 1) ;
NMR (CDCl₃): δ 7.82 (2H, d, J=8.4Hz), 7.44 (2H, d, J=8.0Hz), 7.18 (2H, d, J=8.0Hz), 4.55 (1H, q, J=7.4Hz), 3.94-3.77 (1H, m), 3.62-3.18 (3H, m), 2.38 (3H, s), 1.36 (3H, d, J=7.4Hz), 1.22 (9H, s).

### Example 9(13)

### N-Methyl-N-(4-ethynylphenylsulfonyl)-N'-methyl-N'-phenyl-D-glutamine

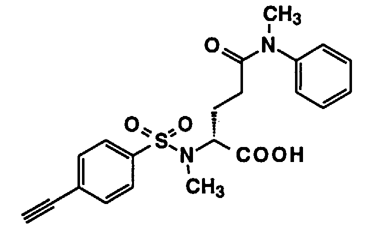

TLC : Rf 0.52 (chloroform : methanol : acetic acid = 90 : 10 : 1) ;
NMR (DMSO-d₆): δ 7.71 (2H, d, J=8.4Hz), 7.62 (2H, d, J=8.4Hz), 7.52-7.32 (3H, m), 7.32-7.22 (2H, m), 4.49 (1H, s), 4.40 (1H, m), 3.15 (3H, s), 2.61 (3H, s), 2.20-1.85 (3H, m), 1.85-1.60 (1H, m).

### Example 9(14)

### N-Methyl-N-[4-(1-propynyl)phenylsulfonyl]-D-alanine

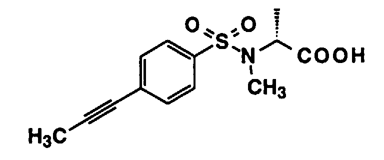

TLC : Rf 0.34 (chloroform : methanol : acetic acid = 100 : 10 : 1) ;
NMR (DMSO-d₆): δ 7.73 (2H, d, J=8.4Hz), 7.55 (2H, d, J=8.4Hz), 4.54 (1H, q, J=7.2Hz), 2.74 (3H, s), 2.08 (3H, s), 1.01 (3H, d, J=7.2Hz).

### Example 9(15)

### N-Methyl-N-(4-ethynylphenylsulfonyl)-D-alanine

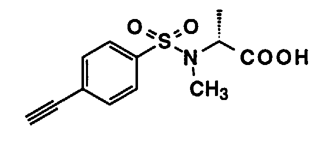

TLC : Rf 0.14 (chloroform : methanol : acetic acid = 100 : 10 : 1) ;
NMR (DMSO-d₆): δ 7.78 (2H, d, J=8.4Hz), 7.66 (2H, d, J=8.4Hz), 4.55 (1H, q, J=7.2Hz), 4.49 (1H, s), 2.75 (3H, s), 1.18 (3H, d, J=7.2Hz).

### Example 10

### N-Hydroxy-[N-methyl-N-[4-[2-(4-methylphenyl)ethynyl]phenylsulfonyl]-D-alanyl]amide

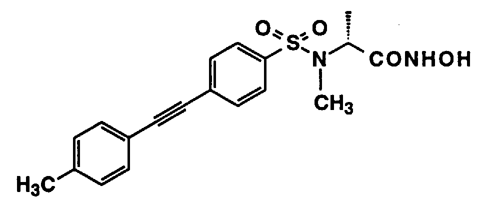

To a solution of the compound prepared in Example 9 (1.70 g) in N,N-dimethylformamide (15 ml), 1-ethyl-3-(3-dimethylaminopropyl)carbodiimide hydrochloride (882 mg), 1-hydroxybenzotriazole monohydrate (684 mg) and N-(1-methoxy-1-methylethoxy)amine (780 mg) were added, and the mixture was stirred at room temperature for 1 hour. To the reaction mixture, water was added. The mixture was extracted with ethyl acetate. The extract was washed with a saturated aqueous solution of sodium chloride, and dried over anhydrous sodium sulfate, and then concentrated. The residue was washed with a mixed solution of ether / hexane to give N-(1-methoxy-1-methylethoxy)-[N-[4-[2-(4-methyl phenyl)ethynyl]phenylsulfonyl]-N-methyl-D-alanyl]amide. To a solution of this compound in methanol (20 ml), 1N hydrochloric acid (0.1 ml) was added, and the mixture was stirred at room temperature for 1 hour. The reaction mixture was neutralized, and concentrated. The residue was extracted with ethyl acetate. The extract was washed with a saturated aqueous solution of sodium chloride, and dried over anhydrous sodium sulfate, and then concentrated. The residue was washed with ether to give the title compound (836 mg) having the following physical data.
TLC : Rf 0.31 (chloroform : methanol = 19 : 1) ;
NMR (DMSO-d₆): δ 10.72 (1H, s), 8.89 (1H, s), 7.81 (2H, d, J=8.4Hz), 7.73 (2H, d, J=8.4Hz), 7.49 (2H, d, J=8.0Hz), 7.26 (2H, d, J=8.0Hz), 4.38 (1H, q, J=7.0Hz), 2.85 (3H, s), 2.36 (3H, s), 1.06 (3H, d, J=7.0Hz).

### Example 10(1)-10(15)

The following compounds were obtained by the same procedure as Example 10 or Example 6 using the compound prepared in Example 9 (1), 9(3)-9(15), or by the same procedure as Example 10 using the compound prepared in Example 9(6) with extra operation same as Example 9 en route.

### Example 10(1)

### N-Hydroxy-[N-benzyl-N-[4-[2-(4-methylphenyl)ethynyl]phenylsulfonyl]-D-alanyl]amide

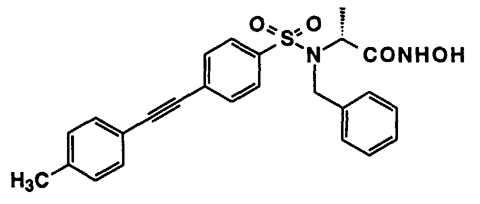

TLC : Rf 0.60 (chloroform : methanol = 9 : 1) ;
NMR (DMSO-d₆): δ 10.70 (1H, s), 8.69 (1H, s), 7.77 (2H, d, J=8.4Hz), 7.65 (2H, d, J=8.4Hz), 7.49 (2H, d, J=8.0Hz), 7.42-7.16 (7H, m), 4.80-4.58 (2H, m), 4.46 (1H, q, J=7.0Hz), 2.36 (3H, s), 1.12 (3H, d, J=7.0Hz).

### Example 10(2)

### N-Hydroxy-[N-methoxymethyl-N-[4-[2-(4-methylphenyl)ethynyl]phenylsulfonyl]-D-alanyl]amide

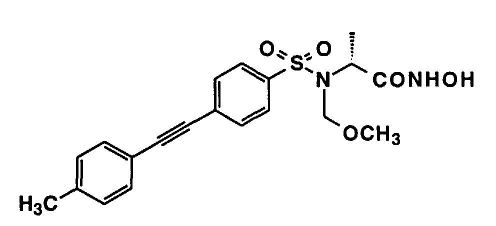

TLC : Rf 0.45 (chloroform : methanol = 9 : 1) ;
NMR (DMSO-d₆): δ 10.60 (1H, s), 9.20-8.60 (1H, br), 7.85 (2H, d, J=8.4Hz), 7.71 (2H, d, J=8.4Hz), 7.49 (2H, d, J=8.0Hz), 7.26 (2H, d, J=8.0Hz), 5.03 (1H, d, J=11.4Hz), 4.87 (1H, d, J=11.4Hz), 4.21 (1H, q, J=7.4Hz) , 3.21 (3H, s), 2.35 (3H, s), 1.23 (3H, d, J=7.4Hz).

### Example 10(3)

### N-Hydroxy-[N-methyl-N-[4-[2-[4-(1-imidazolyl)phenyl]ethynyl]phenylsulfonyl]-D-alanyl]amide hydrochloride

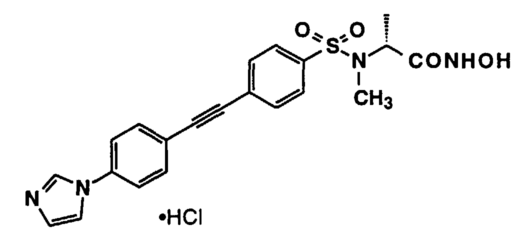

TLC : Rf 0.38 (chloroform : methanol = 9 : 1) ;
NMR (DMSO-d₆): δ 10.78 (1H, br.s), 9.83 (1H, s), 8.41-8.37 (1H, m), 8.02-7.70 (10H, m), 4.40 (1H, q, J=7.0Hz), 2.86 (3H, s), 1.08 (3H, d, J=7.0Hz).

### Example 10(4)

### N-Hydroxy-N-methyl-N-[4-(1-pentynyl)phenylsulfonyl]-D-alanyl]amide

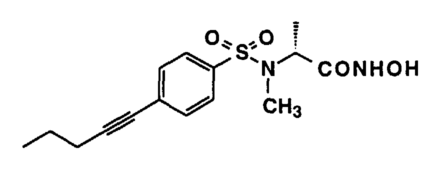

TLC : Rf 0.54 (chloroform : methanol = 9 : 1) ;
NMR (DMSO-d₆): δ 11.00-8.40 (2H, br), 7.74 (2H, d, J=8.4Hz), 7.57 (2H, d, J=8.4Hz), 4.35 (1H, q, J=7.0Hz), 2.82 (3H, s), 2.45 (2H, t, J=7.0Hz), 1.67-1.50 (2H, m), 1.05-0.98 (5H, m).

### Example 10(5)

### N-Hydroxy-[N-(2-benzoyloxyethyl)-N-[4-[2-(4-methylphenyl)ethynyl]phenylsulfonyl]-D-alanyl]amide

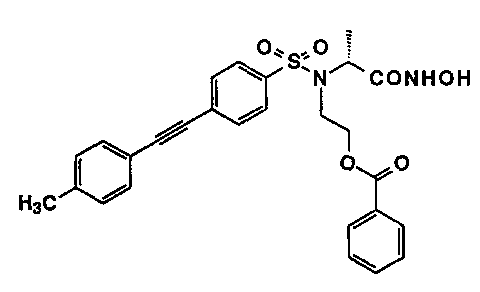

TLC : Rf 0.47 (chloroform : methanol = 9 : 1) ;
NMR (DMSO-d₆): δ 10.73 (1H, s), 9.30-8.70 (1H, br), 7.98 (2H, d, J=7.4Hz), 7.86 (2H, d, J=8.4Hz), 7.80-7.60 (3H, m), 7.58-7.40 (4H, m), 7.26 (2H, d, J=8.0Hz), 4.58-4.28 (3H, m), 4.00-3.80 (1H, m), 3.74-3.54 (1H, m), 2.36 (3H, s), 1.24 (3H, d, J=7.0Hz).

### Example 10(6)

### N-Hydroxy-[N-2-(1-imidazolyl)ethyl-N-[4-[2-(4-methylphenyl)ethynyl]phenylsulfonyl]-D-alanyl]amide hydrochloride

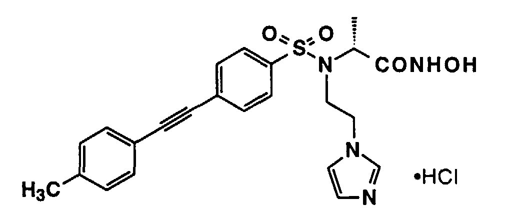

TLC : Rf 0.54 (chloroform : methanol = 4 : 1) ;
NMR (DMSO-d₆): δ 10.92 (1H, s), 9.16 (1H, s), 9.12-8.92 (1H, br), 7.83 (2H, d, J=8.8Hz), 7.82-7.78 (1H, m), 7.73 (2H, d, J=8.8Hz), 7.68-7.64 (1H, m), 7.48 (2H, d, J=8.2Hz), 7.26 (2H, d, J=8.2Hz), 4.65-4.24 (3H, m) , 4.02-3.81 (1H, m), 3.68-3.52 (1H, m), 2.36 (3H, s), 1.09 (3H, d, J=7.0Hz).

### Example 10(7)

### N-Hydroxy-[N-2-morpholinoethyl-N-[4-[2-(4-methylphenyl)ethynyl]phenylsulfonyl]-D-alanyl]amide hydrochloride

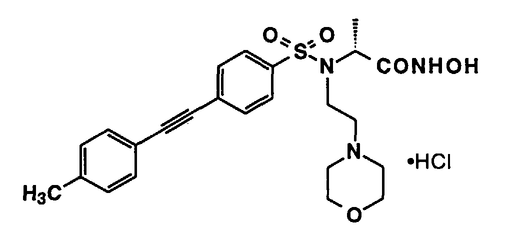

TLC : Rf 0.52 (chloroform : methanol = 9 : 1) ;
NMR (DMSO-d₆): δ 11.22-10.98 (1H, br), 10.90 (1H, s), 9.20-8.88 (1H, br), 7.91 (2H, d, J=8.4Hz), 7.75 (2H, d, J=8.4Hz), 7.49 (2H, d, J=8.0Hz), 7.27 (2H, d, J=8.0Hz), 4.39 (1H, q, J=7.4Hz), 4.10-3.62 (6H, m), 3.60-3.00 (6H, m), 2.36 (3H, s), 1.18 (3H, d, J=7.4Hz).

### Example 10(8)

### N-Hydroxy-[N-3-dimethylaminopropyl-N-[4-[2-(4-methylphenyl)ethynyl]phenylsulfonyl]-D-alanyl]amide hydrochloride

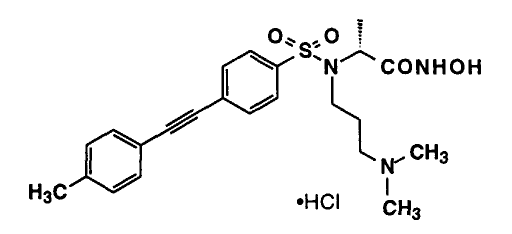

TLC : Rf 0.42 (chloroform : methanol : acetic acid : water = 14 : 6 : 1 : 1) ;
NMR (DMSO-d₆): δ 10.77 (1H, s), 10.25-10.02 (1H, br.s), 8.93 (1H, s), 7.86 (2H, d, J=8.4Hz), 7.73 (2H, d, J=8.4Hz), 7.48 (2H, d, J=8.0Hz), 7.26 (2H, d, J=8.0Hz), 4.34 (1H, q, J=7.0Hz), 3.60-3.20 (2H, m), 3.09 (2H, t, J=7.2Hz), 2.76 (6H, s), 2.36 (3H, s), 2.12-1.90 (2H, m), 1.15 (3H, d, J=7.0Hz).

### Example 10(9)

### N-Hydroxy-[N-3-hydroxypropyl-N-[4-[2-(4-methylphenyl)ethynyl]phenylsulfonyl]-D-alanyl]amide

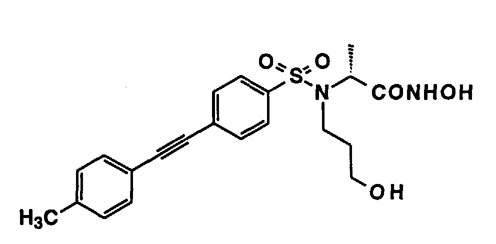

TLC : Rf 0.43 (chloroform : methanol = 9 : 1) ;
NMR (DMSO-d₆): δ 10.64 (1H, s), 8.86 (1H, s), 7.82 (2H, d, J=8.4Hz), 7.70 (2H, d, J=8.4Hz), 7.48 (2H, d, J=8.0Hz), 7.26 (2H, d, J=8.0Hz), 4.44 (1H, t, J=5.0Hz), 4.35 (1H, q, J=6.8Hz), 3.61-3.12 (4H, m), 2.36 (3H, s), 1.94-1.56 (2H, m), 1.19 (3H, d, J=6.8Hz).

### Example 10(10)

### N-Hydroxy-[N-3-pyridylmethyl-N-[4-[2-(4-methylphenyl)ethynyl]phenylsulfonyl]-D-alanyl]amide hydrochloride

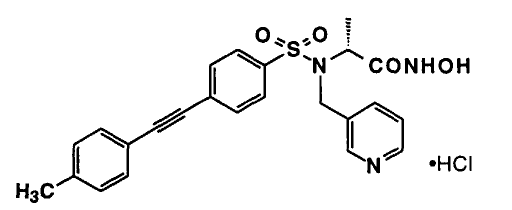

TLC : Rf 0.36 (chloroform : methanol = 9 : 1) ;
NMR (DMSO-d₆): δ 10.86 (1H, s), 8.88 (1H, s), 8.80 (1H, d, J=5.6Hz), 8.49 (1H, d, J=8.0Hz), 7.96 (1H, dd, J=8.0, 5.6Hz), 7.87 (2H, d, J=8.4Hz), 7.74 (2H, d, J=8.4Hz), 7.50 (2H, d, J=8.2Hz), 7.27 (2H, d, J=8.2Hz), 4.88 (1H, d, J=17.6Hz), 4.76 (1H, d, J=17.6Hz), 4.51 (1H, q, J=6.8Hz), 2.36 (3H, s), 1.13 (3H, d, J=6.8Hz).

### Example 10(11)

### N-Hydroxy-[N-2-t-butoxyethyl-N-[4-[2-(4-methylphenyl)ethynyl]phenylsulfonyl]-D-alanyl]amide

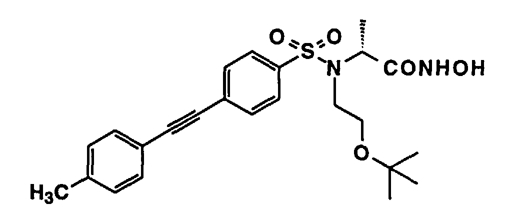

TLC : Rf 0.44 (chloroform : methanol = 19 : 1) ;
NMR (DMSO-d₆): δ 10.63 (1H, s), 8.88 (1H, s), 7.83 (2H, d, J=8.4Hz), 7.71 (2H, d, J=8.4Hz), 7.48 (2H, d, J=8.0Hz), 7.26 (2H, d, J=8.0Hz), 4.32 (1H, q, J=7.4Hz), 3.60-3.18 (4H, m), 2.35 (3H, s), 1.17 (3H, d, J=7.4Hz), 1.12 (9H, s).

### Example 10(12)

### N-Hydroxy-[N-methyl-N-(4-ethynylphenylsulfonyl)-N'-methyl-N'-phenyl-D-glutaminyl]amide

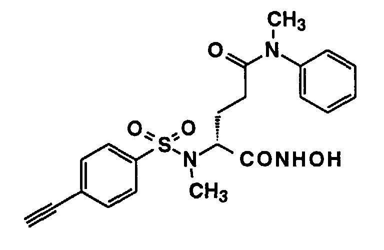

TLC : Rf 0.46 (chloroform : methanol = 9 : 1) ;
NMR (DMSO-d₆): δ 10.71 (1H, s), 8.85 (1H, s), 7.74 (2H, d, J=8.4Hz), 7.63 (2H, d, J=8.4Hz), 7.55-7.34 (3H, m), 3.34-7.22 (2H, m), 4.49 (1H, s), 4.15 (1H, m), 3.16 (3H, s), 2.70 (3H, s), 2.08-1.60 (4H, m).

### Example 10(13)

### N-Hydroxy-[N-methyl-N-[4-(1-propynyl)phenylsulfonyl]-D-alanyl]amide

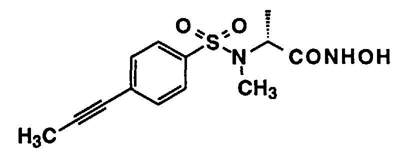

TLC : Rf 0.25 (chloroform : methanol : acetic acid = 100 : 10 : 1) ;
NMR (DMSO-d₆): δ 10.69 (1H, br.s), 8.87 (1H, br.s), 7.72 (2H, d, J=8.4Hz), 7.57 (2H, d, J=8.4Hz), 4.33 (1H, q, J=7.2Hz), 2.81 (3H, s), 2.08 (3H, s), 1.02 (3H, d, J=7.2Hz).

### Example 10(14)

### N-Hydroxy-[N-methyl-N-(4-ethynylphenylsulfonyl)-D-alanyl]amide

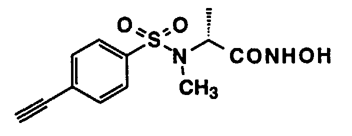

TLC : Rf 0.45 (chloroform : methanol : acetic acid = 100 : 10 : 1) ;
NMR (DMSO-d₆): δ 10.70 (1H, br.s), 9.30-8.00 (1H, br.), 7.77 (2H, d, J=8.6Hz), 7.68 (2H, d, J=8.6Hz), 4.51 (1H, s), 4.34 (1H, q, J=7.0Hz), 2.82 (3H, s), 1.04 (3H, d, J=7.0Hz).

### Example 10(15)

### N-Hydroxy-[N-2-hydroxyethyl-N-[4-[2-(4-methylphenyl)ethynyl]phenylsulfonyl]-D-alanyl]amide

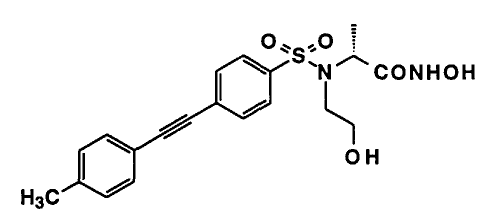

TLC : Rf 0.35 (chloroform : methanol = 9 : 1) ;
NMR (CD₃OD): δ 7.85 (2H, d, J=8.4Hz), 7.68 (2H, d, J=8.4Hz), 7.43 (2H, d, J=8.0Hz), 7.21 (2H, d, J=8.0Hz), 4.44 (1H, q, J=7.0Hz), 3.90-3.50 (3H, m), 3.39-3.20 (1H, m), 2.37-(3H, s), 1.26 (3H, d, J=7.0Hz).

### [Formulation Example]

### Formulation Example 1

The following components were admixed in conventional method and punched out to obtain 100 tablets each containing 50 mg of active ingredient.

| | |
|---|---|
| N-[4-[2-(4-Methylphenyl)ethynyl]phenylsulfonyl]-D-phenylalanine | 5.0 g |
| Carboxymethyl cellulose calcium (disintegrating agent) | 0.2 g |
| Magnesium stearate (lubricating agent) | 0.1 g |
| Micro crystalline cellulose | 4.7 g |

### Formulation Example 2

The following components were admixed in conventional method. The solution was sterilized in conventional manner, placed 5 ml portions into ampoules and freeze-dried in conventional method to obtain 100 ampoules each containing 20 mg of active ingredient.

| | |
|---|---|
| N-[4-[2-(4-Methylphenyl)ethynyl]phenylsulfonyl]-D-phenylalanine | 2.0 g |
| Mannitol | 20 g |
| Distilled water | 500 ml |

## Claims

1. A phenylsulfonamide derivative of formula (I): 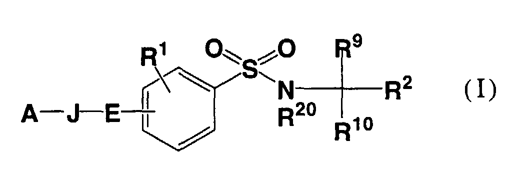
(wherein R¹ is hydrogen, or C₁₋₄alkyl;
R² is -COOR³, or -CONHOR⁴;
R³ is
1) hydrogen,
2) C₁₋₈alkyl,
3) phenyl, or
4) C₁₋₄alkyl substituted by a group selected from phenyl, -OCOR¹⁵ (wherein R¹⁵ is C₁₋₄ alkyl) and -CONR¹⁶R¹⁷ (wherein R¹⁶ and R¹⁷ are each independently, hydrogen or C₁₋₄ alkyl);
R⁴ is hydrogen, C₁₋₈alkyl, phenyl, or C₁₋₄alkyl substituted by phenyl;
E is
―C≡C― or ―CH=CH―;
A is hydrogen, C₁₋₈alkyl, a carbocyclic ring or a heterocyclic ring, in which the said rings may be substituted by 1-3 groups selected from:
C₁₋₁₅alkyl, C₁₋₁₅alkoxy, halogen, nitro, cyano, guanidino, amidino, hydroxy, benzyloxy, trifluoromethyl, NR⁵R⁶ (wherein R⁵ and R⁶ are each independently, hydrogen, C₁₋₄alkyl, or -COOR⁷ (wherein R⁷ is C₁₋₄alkyl or benzyl)), -COOR⁸ (wherein R⁸ is hydrogen, C₁₋₄ alkyl, phenyl, or C₁₋₄alkyl substituted by phenyl), a carbocyclic ring and a heterocyclic ring, or C₁₋₄alkyl substituted by hydroxy, C₁₋₄alkoxy, NR⁵R⁶, a carbocyclic ring or a heterocyclic ring;
J is a single bond or C₁₋₈alkylene;
R⁹ and R¹⁰ are each independently,
(1) hydrogen,
(2) C₁₋₈alkyl (wherein one carbon atom may be replaced by a sulfur atom),
(3) -COR¹¹ (wherein R¹¹ is hydroxy, C₁₋₈alkyl, C₁₋₈alkoxy, phenoxy, C₁₋₄alkoxy substituted by phenyl, or NR¹⁸R¹⁹ (wherein R¹⁸ and R¹⁹ are each independently, hydrogen, C₁₋₄alkyl, phenyl, or C₁₋₄alkyl substituted by 1-2 phenyl, in which the said phenyl may be substituted by 1-3 groups selected from C₁₋₄alkyl, C₁₋₄alkoxy, halogen, hydroxy, and trifluoromethyl)),
(4) a carbocyclic ring,
(5) a heterocyclic ring, or
(6) C₁₋₈alkyl substituted by a group selected from (i)-(viii) below:
(i) -COR¹¹, wherein R¹¹ is as defined above,
(ii) C₁₋₄alkoxy,
(iii) hydroxy,
(iv) benzyloxy,
(v) guanidino,
(vi) -NR¹²R¹³ (wherein R¹² and R¹³ are each independently, hydrogen, C₁₋₄alkyl or COOR¹⁴ (wherein R¹⁴ is C₁₋₄alkyl or benzyl)),
(vii) a carbocyclic ring,
(viii) a heterocyclic ring,
(in which the carbocyclic ring or the heterocyclic ring defined above may be substituted by 1-3 groups selected from C₁₋₄alkyl, C₁₋₄alkoxy, halogen, hydroxy and trifluoromethyl);
R²⁰ is hydrogen, C₁₋₄alkyl, C₁₋₈alkoxycarbonyl, C₁₋₄alkoxycarbonyl substituted by phenyl, or C₁₋₈alkyl substituted by a group selected from hydroxy, C₁₋₄alkoxy, benzoyloxy, -COOR²¹ (wherein R²¹ is hydrogen, C₁₋₈alkyl or benzyl), -NR²²R²³ (wherein R²² and R²³ are each independently, hydrogen or C₁₋₄alkyl), a carbocyclic ring and a heterocyclic ring;
or R⁹ and R²⁰ together with the carbon atom and nitrogen atom to which they are attached represent a 5-7 membered heterocyclic ring containing one nitrogen)
or a non-toxic salt thereof.

2. A compound according to claim 1, wherein R² is -COOR³ (wherein R³ is as defined in claim 1).

3. A compound according to claim 1, wherein R² is -CONHOR⁴ (wherein R⁴ is as defined in claim 1).

4. A compound according to claim 1, wherein A is hydrogen or C₁₋₈alkyl.

5. A compound according to claim 1, wherein A is a carbocyclic ring or a heterocyclic ring.

6. A compound according to claim 1, which is:
(1) N-[4-(1-pentynyl)phenylsulfonyl]-D-alanine,
(2) N-[4-[4-(1-heptenyl)]phenylsulfonyl]-D-alanine,
(3) N-[4-[4-(1-hexynyl)]phenylsulfonyl]-D-alanine,
(4) N-[4-(1-octynyl)phenylsulfonyl]-D-alanine,
(5) N-[(4-ethynyl)phenylsulfonyl]-D-alanine,
(6) N-[4-(1-propynyl)phenylsulfonyl]-D-alanine,
(7) N-[4-(1-heptynyl)phenylsulfonyl]-L-alanine,
(8) N-[4-(1-pentynyl)phenylsulfonyl]-L-alanine,
(9) N-(4-ethynyl)phenylsulfonyl-N'-methyl-N'-benzyl-D-glutamine,
(10) N-(4-ethynyl)phenylsulfonyl-N'-(3-phenylpropyl)-D-glutamine,
(11) N-(4-ethynyl)phenylsulfonyl-N'-(2-phenylethyl)-D-glutamine,
(12) N-(4-ethynyl)phenylsulfonyl-N'-benzyl-D-glutamine,
(13) N-(4-ethynyl)phenylsulfonyl-N'-(1,2-diphenylethyl)-D-glutamine,
(14) N-(4-ethynyl)phenylsulfonyl-N'-diphenylmethyl-D-glutamine,
(15) N-(4-ethynyl)phenylsulfonyl-N'-phenyl-D-glutamine,
(16) N-(4-ethynyl)phenylsulfonyl-N'-(4-methoxyphenyl)-D-glutamine,
(17) N-methyl-N-[4-(1-pentynyl)phenylsulfonyl]-D-alanine,
(18) N-methyl-N-(4-ethynylphenylsulfonyl)-N'-methyl-N'-phenyl-D-glutamine,
(19) N-methyl-N-[4-(1-propynyl)phenylsulfonyl]-D-alanine,
(20) N-methyl-N-(4-ethynylphenylsulfonyl)-D-alanine,
(21) N-[4-(1-hexenyl)phenylsulfonyl]-D-alanine,
a methyl ester thereof, a t-butyl ester thereof, or a non-toxic salt thereof.

7. A compound according to claim 1, which is:
(1) N-[4-[2-(4-methylphenyl)ethynyl]phenylsulfonyl]-D-phenylalanine,
(2) N-[4-[2-(4-methylphenyl)ethynyl]phenylsulfonyl]-D-alanine,
(3) N-[4-(2-phenylethynyl)phenylsulfonyl]glycine,
(4) N-[4-[2-(4-methylphenyl)ethynyl]phenylsulfonyl]-D-tryptophan,
(5) N-[4-[2-(pyridin-2-yl)ethynyl]phenylsulfonyl]glycine,
(6) N-[4-[2-(4-methylphenyl)ethynyl]phenylsulfonyl]glycine,
(7) N-[4-[2-(4-methylphenyl)ethynyl]phenylsulfonyl]-D-valine,
(8) N-[4-[2-(2-methylphenyl)ethynyl]phenylsulfonyl]-D-alanine,
(9) N-[4-[2-(3-methylphenyl)ethynyl]phenylsulfonyl]-D-alanine,
(10) N-[4-[2-(2-naphthyl)ethynyl]phenylsulfonyl]-D-alanine,
(11) N-[4-[2-(4-isobutylphenyl)ethynyl]phenylsulfonyl]-D-tryptophan,
(12) N-[4-[2-(1,1'-biphenyl-4-yl)ethynyl]phenylsulfonyl]-D-tryptophan,
(13) N-[4-[2-(4-benzyloxyphenyl)ethynyl]phenylsulfonyl]-D-tryptophan,
(14) N-[4-[2-(4-hydroxymethylphenyl)ethynyl]phenylsulfonyl]-D-alanine,
(15) N-[4-[2-(4-imidazolylphenyl)ethynyl]phenylsulfonyl]-D-tryptophan,
(16) N-[4-[2-(4-methylphenyl)ethynyl]phenylsulfonyl]-L-tryptophan,
(17) N-[4-[2-(4-methylphenyl)ethynyl]phenylsulfonyl]-D-serine,
(18) N-[4-[2-(4-methylphenyl)ethynyl]phenylsulfonyl]-D-glutamic acid,
(19) N-[4-[2-(4-methylphenyl)ethynyl]phenylsulfonyl]-D-lysine,
(20) N-[4-(6-imidazolyl-1-hexynyl)phenylsulfonyl]-D-alanine,
(21) N-[4-(6-imidazolyl-1-hexynyl)phenylsulfonyl]-D-tryptophan,
(22) N-[4-[2-(4-dimethylaminomethylphenyl)ethynyl]phenylsulfonyl]-D-tryptophan,
(23) N-[4-[2-(4-imidazolylphenyl)ethynyl]phenylsulfonyl]-N'-methyl-N'-(2-phenylethyl) -D-glutamine,
(24) N-[4-[2-[4-(1-imidazolylmethyl)phenyl]ethynyl]phenylsulfonyl]-D-tryptophan,
(25) N-[4-[5-(1-imidazolyl)-1-pentynyl]phenylsulfonyl]-D-tryptophan,
(26) N-[4-[2-[4-(2-dimethylaminoethyl)phenyl]ethynyl]phenylsulfonyl]-D-tryptophan,
(27) N-[4-[2-[4-[2-(1-imidazolyl)ethyl]phenyl]ethynyl]phenylsulfonyl]-D-tryptophan,
(28) N-[4-[2-[4-(2-carboxyethyl)phenyl]ethynyl]phenylsulfonyl]-D-tryptophan,
(29) N-[4-[2-(4-methylphenyl)ethynyl]phenylsulfonyl]-L-alanine,
(30) N-[4-[2-(4-methylphenyl)ethynyl]phenylsulfonyl]-N'-methyl-N'-benzyl-D-glutamine,
(31) N-[4-[2-(4-methylphenyl)ethynyl]phenylsulfonyl]-N'-methyl-N'-(2-phenylethyl)-D-glutamine,
(32) N-[4-[2-(4-methylphenyl)ethynyl]phenylsulfonyl]-L-proline,
(33) N-methyl-N-[4-[2-(4-methylphenyl)ethynyl]phenylsulfonyl]-D-alanine,
(34) N-benzyl-N-[4-[2-(4-methylphenyl)ethynyl]phenylsulfonyl]-D-alanine,
(35) N-carboxymethyl-N-[4-[2-(4-methylphenyl)ethynyl]phenylsulfonyl]-D-alanine,
(36) N-methoxymethyl-N-[4-[2-(4-methylphenyl)ethynyl]phenylsulfonyl]-D-alanine,
(37) N-methyl-N-[4-[2-[4-(1-imidazolyl)phenyl]ethynyl]phenylsulfonyl]-D-alanine,
(38) N-(2-benzoyloxyethyl)-N-[4-[2-(4-methylphenyl)ethynyl]phenylsulfonyl]-D-alanine,
(39) N-2-(1-imidazolyl)ethyl-N-[4-[2-(4-methylphenyl)ethynyl]phenylsulfonyl]-D-alanine,
(40) N-2-morpholinoethyl-N-[4-[2-(4-methylphenyl)ethynyl]phenylsulfonyl]-D-alanine
(41) N-3-dimethylaminopropyl-N-[4-[2-(4-methylphenyl)ethynyl]phenylsulfonyl]-D-alanine,
(42) N-3-hydroxypropyl-N-[4-[2-(4-methylphenyl)ethynyl]phenylsulfonyl]-D-alanine,
(43) N-3-pyridylmethyl-N-[4-[2-(4-methylphenyl)ethynyl]phenylsulfonyl]-D-alanine,
(44) N-2-t-butoxyethyl-N-[4-[2-(4-methylphenyl)ethynyl]phenylsulfonyl]D-alanine
(45) N-[4-(2-phenylethenyl)phenylsulfonyl]glycine
(46) N-[4-[2-(4-methylphenyl)ethenyl]phenylsulfonyl]-D-alanine,
(47) N-[4-[2-(4-isopropylphenyl)ethenyl]phenylsulfonyl]-D-tryptophan,
a methyl ester thereof, a t-butyl ester thereof, or a non-toxic salt thereof.

8. A compound according to claim 1, which is:
(1) N-hydroxy-[N-[4-(1-pentynyl)phenylsulfonyl]-D-alanyl]amide,
(2) N-hydroxy-[N-[(4-ethynyl)phenylsulfonyl]-D-alanyl]amide,
(3) N-hydroxy-[N-[4-(1-octynyl)phenylsulfonyl]-D-alanyl]amide,
(4) N-hydroxy-[N-[4-[4-(1-hexynyl)]phenylsulfonyl]-D-alanyl]amide,
(5) N-hydroxy-[N-[4-[4-(1-heptynyl)]phenylsulfonyl]-D-alanyl]amide,
(6) N-hydroxy-[N-[4-(1-propynyl)phenylsulfonyl]-D-alanyl]amide,
(7) N-hydroxy-[N-[4-(1-heptynyl)phenylsulfonyl]-L-alanyl]amide,
(8) N-hydroxy-[N-[4-(1-pentynyl)phenylsulfonyl]-L-alanyl]amide,
(9) N-hydroxy-[N-(4-ethynyl)phenylsulfonyl-N'-methyl-N'-benzyl-D-glutaminyl]amide,
(10) N-hydroxy-[N-(4-ethynyl)phenylsulfonyl-N'-(3-phenylpropyl)-D-glutaminyl]amide,
(11) N-hydroxy-[N-(4-ethynyl)phenylsulfonyl-N'-(2-phenylethyl)-D-glutaminyl]amide,
(12) N-hydroxy-[N-(4-ethynyl)phenylsulfonyl-N'-benzyl-D-glutaminyl]amide,
(13) N-hydroxy-[N-(4-ethynyl)phenylsulfonyl-N'-(1,2-diphenylethyl)-D-glutaminyl] amide,
(14) N-hydroxy-[N-(4-ethynyl)phenylsulfonyl-N'-(diphenylmethyl)-D-glutaminyl]amide
(15) N-hydroxy-[N-(4-ethynyl)phenylsulfonyl-N'-phenyl-D-glutaminyl]amide
(16) N-hydroxy-[N-(4-ethynyl)phenylsulfonyl-N'-(4-methoxyphenyl)-D-glutaminyl] amide,
(17) N-hydroxy-[N-methyl-N-[4-(1-pentynyl)phenylsulfonyl]-D-alanyl]amide,
(18) N-hydroxy-[N-methyl-N-(4-ethynylphenylsulfonyl)-N'-methyl-N'-phenyl-D-glutaminyl]amide,
(19) N-hydroxy-[N-methyl-N-[4-(1-propynyl)phenylsulfonyl]-D-alanyl]amide,
(20) N-hydroxy-[N-methyl-N-(4-ethynylphenylsulfonyl)-D-alanyl]amide,
(21) N-hydroxy-[N-[4-(1-hexenyl)phenylsulfonyl]-D-alanyl]amide,
or a non-toxic salt thereof.

9. A compound according to claim 1, which is:
(1) N-hydroxy-[N-[4-[2-(4-methylphenyl)ethynyl]phenylsulfonyl]-D-tryptophyl]amide,
(2) N-hydroxy-[N-[4-[2-(4-methylphenyl)ethynyl]phenylsulfonyl]-D-alanyl]amide,
(3) N-hydroxy-[N-[4-[2-(4-methylphenyl)ethynyl]phenylsulfonyl]-N'-methyl-N'-(2-phenylethyl)-D-glutaminyl]amide,
(4) N-hydroxy-[N-[4-[2-(4-methylphenyl)ethynyl]phenylsulfonyl]-L-alanyl]amide,
(5) N-hydroxy-[N-[4-[2-(4-methylphenyl)ethynyl]phenylsulfonyl]-N'-methyl-N'-benzyl-D-glutaminyl]amide,
(6) N-hydroxy-[N-[4-[2-(4-methylphenyl)ethynyl]phenylsulfonyl]-D-prolyl]amide,
(7) N-hydroxy-[N-methyl-N-[4-[2-(4-methylphenyl)ethynyl]phenylsulfonyl]-D-alanyl] amide,
(8) N-hydroxy-[N-benzyl-N-[4-[2-(4-methylphenyl)ethynyl]phenylsulfonyl]-D-alanyl] amide,
(9) N-hydroxy-[N-methoxymethyl-N-[4-[2-(4-methylphenyl)ethynyl]phenylsulfonyl]-D-alanyl]amide,
(10) N-hydroxy-[N-methyl-N-[4-[2-[4-(1-imidazolyl)phenyl]ethynyl]phenylsulfonyl]-D-alanyl]amide,
(11) N-hydroxy-[N-(2-benzoyloxyethyl)-N-[4-[2-(4-methylphenyl)ethynyl]phenyl sulfonyl]-D-alanyl]amide,
(12) N-hydroxy-[N-2-(1-imidazolyl)ethyl-N-[4-[2-(4-methylphenyl)ethynyl] phenyl sulfonyl]-D-alanyl]amide,
(13) N-hydroxy-[N-2-morpholinoethyl-N-[4-[2-(4-methylphenyl]ethynyl] phenyl sulfonyl]-D-alanyl]amide,
(14) N-hydroxy-[N-3-dimethylaminopropyl-N-[4-[2-(4-methylphenyl)ethynyl]phenyl sulfonyl]-D-alanyl]amide,
(15) N-hydroxy-[N-3-hydroxypropyl-N-[4-[2-(4-methylphenyl)ethynyl]phenylsulfonyl]-D-alanyl]amide,
(16) N-hydroxy-[N-3-pyridylmethyl-N-[4-[2-(4-methylphenyl)ethynyl]phenylsulfonyl]-D-alanyl]amide,
(17) N-hydroxy-[N-2-t-butoxyethyl-N-[4-[2-(4-methylphenyl)ethynyl]phenylsulfonyl]-D-alanyl]amide,
(18) N-hydroxy-[N-2-hydroxyethyl-N-[4-[2-(4-methylphenyl)ethynyl]phenylsulfonyl]-D-alanyl]amide,
(19) N-hydroxy-[N-[4-[2-(4-methylphenyl)ethenyl]phenylsulfonyl]-D-alanyl]amide,
or a non-toxic salt thereof.

10. A process for the preparation of a compound of formula (IA-1) 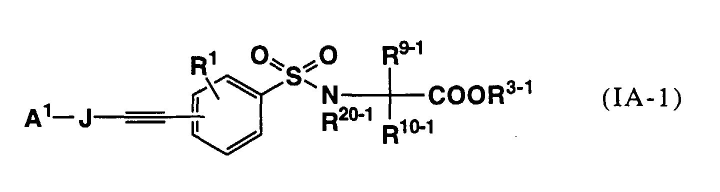
(wherein A¹ has the same meaning as A, with the proviso that -COOH, hydroxy or amino on a carbocyclic ring or a heterocyclic ring in A means a protected form thereof, R³⁻¹ is C₁₋₈alkyl, phenyl, or C₁₋₄alkyl substituted by phenyl, -OCOR¹⁵ (wherein R¹⁵ is as defined in claim 1) or -CONR¹⁶R¹⁷ (wherein R¹⁶ and R¹⁷ are as defined in claim 1), R⁹⁻¹ and R¹⁰⁻¹ each have the same meaning as R⁹ and R¹⁰ with the proviso that in R⁹ and R¹⁰, a group containing -COOH, hydroxy, or amino represents a protected form thereof, R²⁰⁻¹ has the same meaning as R²⁰, with the proviso that a group containing -COOH, hydroxy, or amino represents a protected form thereof, and the other symbols are as defined in claim 1) among the phenylsulfonamide derivatives of formula (I) defined in claim 1, or a non-toxic salt thereof, which is characterized by reacting a compound of formula (III-1-1) 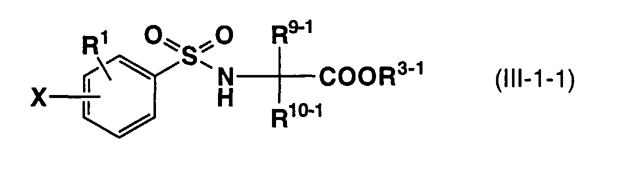
(wherein X is halogen or trifluoromethanesulfonyloxy and the other symbols are as defined in claim 1 or as defined above), and a compound of formula (III-1-2) 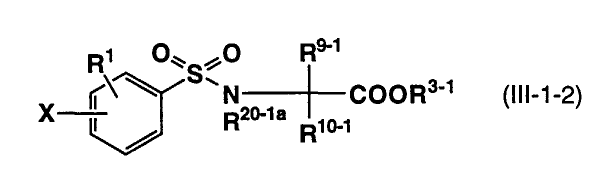
(wherein R^{20-1a} is C₁₋₄alkyl, C₁₋₈alkoxycarbonyl, C₁₋₄alkoxycarbonyl substituted by phenyl, or C₁₋₈alkyl substituted by a group selected from hydroxy, C₁₋₄alkoxy, benzoyloxy, -COOR²¹, -NR²²R²³, a carbocyclic ring, and a heterocyclic ring, with the proviso that any one of a group containing -COOH, hydroxy, or amino, is a protected form thereof, and all symbols are as defined in claim 1 or as defined above, with an acetylene derivative of formula (IV-1) 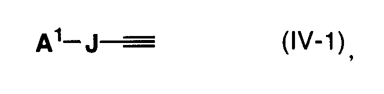
(wherein A¹ and J are as defined in claim 1 or as defined above), or an acetylene derivative of formula (IV-1) protected by silyl or reacting a compound of formula (III-1-1) with a compound of formula (IV-1), or a silyl-protected form thereof, followed by introducing R^{20-1a}.

11. A process for the preparation of formula (IA-2) 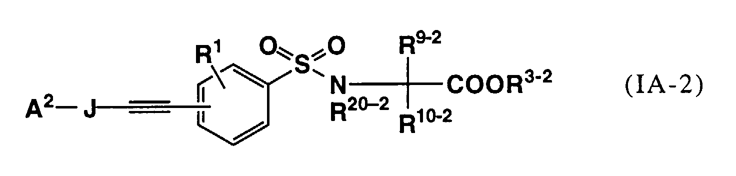
(wherein A², R⁹⁻², R¹⁰⁻², R²⁰⁻² and R³⁻² each have the same meaning as A, R⁹, R¹⁰, R²⁰, R³, with the proviso that at least one of them contains -COOH, hydroxy, or amino, and the other symbols are as defined in claim 1) among the phenylsulfonamide derivatives of formula (I) defined in claim 1, or a non-toxic salt thereof, characterized by subjecting to alkali hydrolysis or deprotection under acidic conditions a compound of the formula (IA-1) 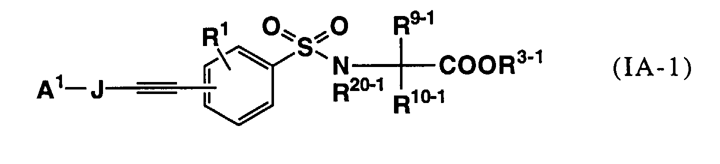
(wherein all symbols are as defined in claim 1 or claim 10), or by subjecting to decomposition with a base a compound of formula (II) 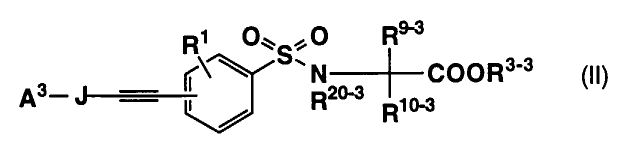
(wherein R³⁻³ is C₁₋₈alkyl, phenyl, or C₁₋₄alkyl substituted by phenyl, -OCOR¹⁵ (wherein R¹⁵ is as defined in claim 1) or -CONR¹⁶R¹⁷ (wherein R¹⁶ and R¹⁷ are as defined in claim 1), or a group removable by decomposition with abase; A³ has the same meaning as A, with the proviso that -COOR⁸ (which is a substituent of a carbocyclic ring or a heterocyclic ring in A) is -COOR⁸⁻³ (R⁸⁻³ is C₁₋₄alkyl, phenyl, C₁₋₄alkyl substituted by phenyl, or a group removable by decomposition with a base); hydroxy or amino is each protected by a group removable by decomposition with a base; R⁹⁻³ and R¹⁰⁻³ have the same meaning as R⁹ and R¹⁰, with the proviso that a group containing -COOH, hydroxy, amino represents a group protected by a group removable by decomposition with a base; R²⁰⁻³ has the same meaning as R²⁰, with the proviso that a group containing -COOH, hydroxy or amino, represented by R²⁰, represents a corresponding group protected by a group removable by decomposition with a base, and the other symbols are as defined in claim 1).

12. A process for the preparation of a compound of formula (IB) 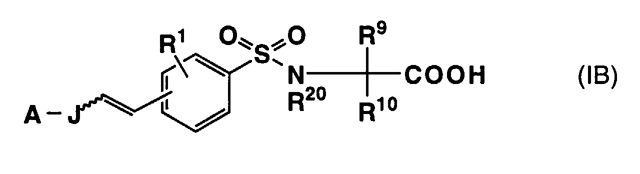
(wherein all symbols are as defined in claim 1) among the phenylsulfonamide derivatives of formula (I) defined in claim 1, or a non-toxic salt thereof, characterized by subjecting to dehydration reaction a compound of formula (VII) 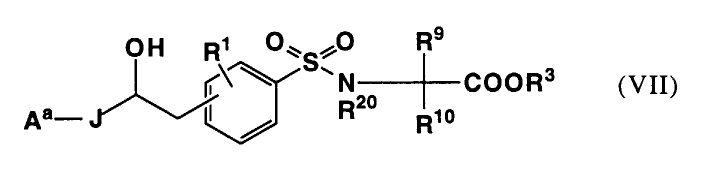
(wherein A^{a} is a carbocyclic ring or a heterocyclic ring, and the other symbols are as defined in claim 1) optionally followed by deprotection, or by reacting a compound of formula (III-1-1) 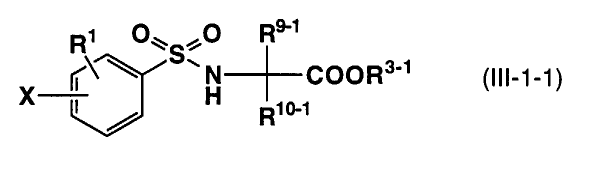
(wherein all symbols are as defined in claim 1 or claim 10), or a compound of (III-1-2) 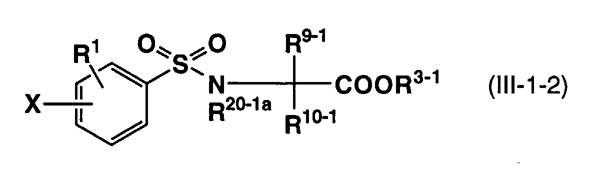
(wherein all symbols are as defined in claim 1 or claim 10) with a vinylzirconium compound of formula (XII) 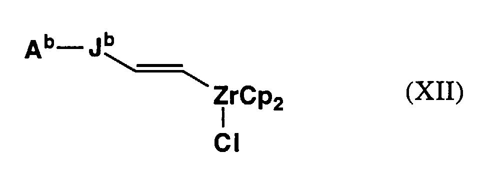
(wherein in A^{b}-J^{b}-, A^{b} is C₁₋₈alkyl when J^{b} is a single bond or C₁₋₈alkylene, and A is hydrogen when J^{b} is C₁₋₈alkylene, and Cp₂ represents bis(cyclopentadienyl)), followed by deprotection, or by reducing a compound of formula (IA-1) (wherein 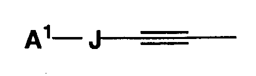 represents ethynyl protected by silyl 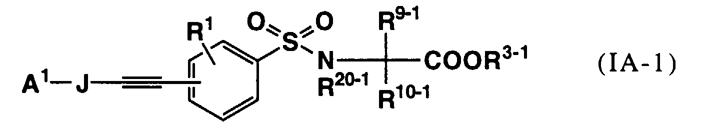
(wherein all symbols are as defined in claim 1 or claim 10)) followed by deprotection.

13. A process for the preparation of a compound of formula (IC) 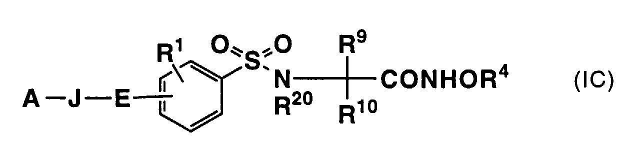
(wherein all symbols are as defined in claim 1) among the phenylsulfonamide derivatives of formula (I) defined in claim 1, or a non-toxic salt thereof, characterized by amidation of a compound of formula (IA-1a) 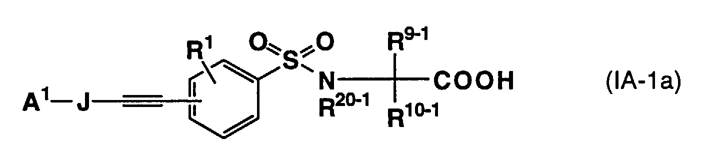
(wherein all symbols are as defined in claim 1 or claim 10), or a compound of formula (IB-4) 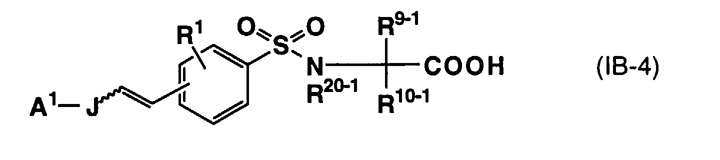
(wherein all symbols are as defined in claim 1 or claim 10) with a compound of formula (XIII)
H₂N-OR₄ (XIII)
(wherein R⁴ ia as defined in claim 1) or protected hydroxylamine, optionally followed by alkali hydrolysis and/or deprotection under acidic conditions.

14. A pharmaceutical composition which comprises a phenylsulfonamide derivative of the formula (I) as defined in claim 1 or a non-toxic salt thereof as an active ingredient.

15. A matrix metalloproteinase inhibitor which comprises a phenylsulfonamide derivative of formula (I) as defined in claim 1 or a non-toxic salt thereof as an active ingredient.

16. An agent for prevention and/or treatment of rheumatoid diseases, arthrosteitis, unusual bone resorption, osteoporosis, periodontitis, intestitial nephritis, arteriosclerosis, pulmonary emphysema, cirrhosis, cornea injury, metastasis or invasion of tumor cells, a disease induced by proliferation of tumor cells, an autoimmune disease (Crohn's disease or Sjogren's syndrome), a disease caused by vascular emigration and infiltration of leukocytes, or arterialization.
